# EUROPEAN PATENT APPLICATION

(11) **EP 4 617 274 A1**
(43) Date of publication of application: **17.09.2025**
(21) Application number: 23888119.7
(22) Date of filing: 10.11.2023
(51) Int. Cl.: C07D 487/22, A61K 31/407, A61K 31/4188, A61P 21/02, A61P 23/00, A61P 35/00

(54) **CUCURBITURIL COMPOUND AND MEDICAL USE THEREOF**

(30) Priority: 11.11.2022 CN 202211412397; 19.07.2023 CN 202310888540
(71) Applicant: Shanghai Senhui Medicine Co., Ltd., Shanghai 201203 (CN); Shanghai Shengdi Pharmaceutical Co., Ltd., Shanghai 201210 (CN); Jiangsu Hengrui Pharmaceuticals Co., Ltd., Lianyungang, Jiangsu 222047 (CN)
(72) Inventor: ZHU, Lingjian, Shanghai 201203 (CN); REN, Wenming, Shanghai 201210 (CN); ZOU, Yang, Shanghai 201203 (CN); LEI, Shenghui, Shanghai 201203 (CN); ZHU, Dan, Lianyungang, Jiangsu 222047 (CN); HUANG, Jian, Shanghai 201203 (CN)
(74) Representative: Potter Clarkson
(86) International application number: PCT/CN2023/131007
(87) International publication number: WO 2024/099434

(57) **Abstract**

The present disclosure relates to a cucurbituril compound and the medical use thereof. Specifically, the present disclosure relates to a compound as shown in formula (I), a pharmaceutically acceptable salt thereof, a preparation method therefor, a pharmaceutical composition comprising same and the use thereof as a therapeutic agent, in particular the use thereof in the treatment or prevention of proliferative diseases, cardiovascular-related diseases or blood cancer and the use thereof in the reversal of drug-induced neuromuscular blocking and/or anesthesia, wherein each substituent is as defined in the description. (I)

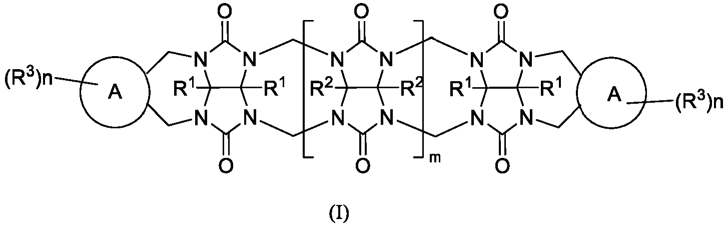

## Description

### TECHNICAL FIELD

The present disclosure pertains to the field of pharmaceutics and particularly relates to CUCURBITURIL-BASED COMPOUNDS AND PHARMACEUTICAL USE THEREOF.

### BACKGROUND

Muscle relaxation is one of the three fundamental elements in general anesthesia. While muscle relaxants satisfy the needs of tracheal intubation and surgery, they also pose a safety risk-residual muscle relaxation, which can cause patients subjective discomfort and a series of pulmonary complications such as hypoxemia, reflux, and aspiration. To reduce the incidence of residual muscle relaxation, strategies such as the use of intermediate- or short-acting muscle relaxants, optimizing intraoperative muscle relaxation management, antagonizing the effects of muscle relaxants after surgery, and perioperative objective muscle relaxation monitoring are often employed. There have constantly been advancements in resolving residual muscle relaxation, which is a challenging clinical issue.

Antagonizing muscle relaxation after surgery refers to the use of muscle relaxant antagonists to reverse the residual effects of non-depolarizing muscle relaxants. Currently, commonly used muscle relaxant antagonists can be broadly classified into two categories: competitive muscle relaxation antagonists, including neostigmine as an acetylcholine inhibitor, etc., and selective muscle relaxation antagonists, including sugammadex sodium as a steroidal muscle relaxant antagonist, cysteine as a benzylisoquinoline-based muscle relaxant antagonist, etc.

The prior art WO2012051407A discloses Calabadion2, which is an acyclic CB[n]-type molecular container with a cucurbituril structure. It can efficiently bind to benzylisoquinoline-based and steroidal muscle relaxants. By covering the quaternary ammonium sites of benzylisoquinoline-based and steroidal muscle relaxants, it prevents the muscle relaxants from binding to neuromuscular choline receptors, thereby achieving rapid reversal of muscle relaxation.

### SUMMARY

The present disclosure provides a compound represented by formula (I) or a pharmaceutically acceptable salt thereof: 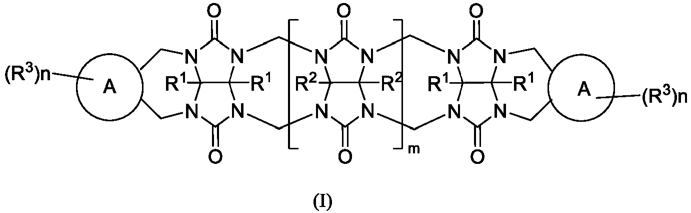 wherein:
each R¹ is independently selected from the group consisting of C₂₋₆ alkyl, C₁₋₆ haloalkyl, C₁₋₆ hydroxyalkyl, -C(O)₂R', R'-(O)-alkylene-, hydroxy, NR'(R"), 3- to 7-membered cycloalkyl, and 3- to 7-membered heterocyclyl; or two R¹ attached to adjacent carbon atoms form together 3- to 10-membered cycloalkyl or 3- to 10-membered heterocyclyl;
R¹ is optionally substituted with one or more R^{1A};
each R² is independently selected from the group consisting of hydrogen, C₁₋₆ alkyl, C₁₋₆ haloalkyl, C₁₋₆ hydroxyalkyl, -C(O)₂R', R'-(O)-alkylene-, hydroxy, NR'(R"), 3- to 7-membered cycloalkyl, and 3- to 7-membered heterocyclyl; or two R² attached to adjacent carbon atoms form together 3- to 10-membered cycloalkyl or 3- to 10-membered heterocyclyl; R² is optionally substituted with one or more R^{2A};
each ring A is independently selected from the group consisting of 5- to 12-membered aryl and 5- to 12-membered heteroaryl;
each R³ is independently selected from the group consisting of halogen, C₁₋₆ alkyl, hydroxy, nitro, cyano, -C(O)₂R', NR'(R"), R'-(O)-alkylene-, 3- to 7-membered cycloalkyl, 3- to 7-membered heterocyclyl, and 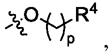 and at least one R³ is 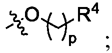 R³ is optionally substituted with one or more R^{3A};
R⁴ is 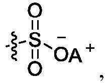 and A⁺ is a monovalent or divalent cation;
R^{1A}, R^{2A}, and R^{3A} are each independently selected from the group consisting of halogen, cyano, nitro, amino, C₁₋₆ alkyl, and C₁₋₆ alkoxy;
R' and R" are each independently selected from the group consisting of hydrogen, C₁₋₆ alkyl, C₁₋₆ hydroxyalkyl, C₁₋₆ haloalkyl, 3- to 7-membered cycloalkyl, and 3- to 7-membered heterocyclyl;
m and n are each independently selected from the group consisting of 1, 2, 3, 4, and 5; each p is independently selected from the group consisting of 1, 2, 3, 4, 5, and 6.

In an optional embodiment, provided in the present disclosure is the compound represented by formula (I) or the pharmaceutically acceptable salt thereof, wherein ring A is phenyl or naphthyl.

In an optional embodiment, provided in the present disclosure is the compound represented by formula (I) or the pharmaceutically acceptable salt thereof, wherein ring A is naphthyl.

In an optional embodiment, the compound represented by formula (I) or the pharmaceutically acceptable salt thereof provided in the present disclosure is a compound represented by formula (I-1) or a pharmaceutically acceptable salt thereof: 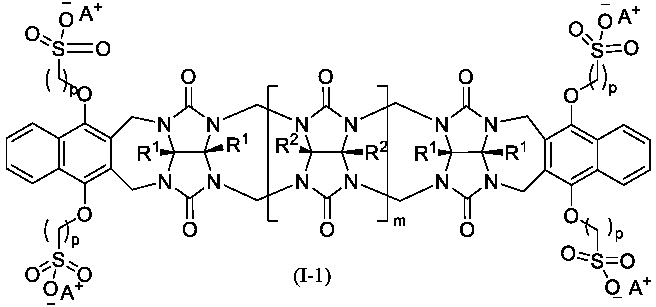 wherein R¹, R², p, m, and A⁺ are as defined in the compound represented by formula (I). In some embodiments, provided in the present disclosure is the compound represented by formula (I) or (I-1) or the pharmaceutically acceptable salt thereof, wherein two R¹ attached to adjacent carbon atoms form together 3- to 10-membered cycloalkyl or 3- to 10-membered heterocyclyl.

In some embodiments, provided in the present disclosure is the compound represented by formula (I) or (I-1) or the pharmaceutically acceptable salt thereof, wherein two R¹ attached to adjacent carbon atoms form together 4- to 8-membered cycloalkyl or 4- to 8-membered heterocyclyl.

In some embodiments, provided in the present disclosure is the compound represented by formula (I) or (I-1) or the pharmaceutically acceptable salt thereof, wherein two R¹ attached to adjacent carbon atoms form together 5- to 6-membered cycloalkyl or 5- to 6-membered heterocyclyl.

In some embodiments, provided in the present disclosure is the compound represented by formula (I) or (I-1) or the pharmaceutically acceptable salt thereof, wherein two R¹ attached to adjacent carbon atoms form together 6-membered cycloalkyl.

In some embodiments, provided in the present disclosure is the compound represented by formula (I) or (I-1) or the pharmaceutically acceptable salt thereof, wherein two R¹ attached to adjacent carbon atoms form together 5- to 6-membered heterocyclyl, wherein a heteroatom is nitrogen or oxygen.

In some embodiments, provided in the present disclosure is the compound represented by formula (I) or (I-1) or the pharmaceutically acceptable salt thereof, wherein two R¹ attached to adjacent carbon atoms form together 5- to 6-membered heterocyclyl, wherein a heteroatom is oxygen.

In some embodiments, provided in the present disclosure is the compound represented by formula (I) or (I-1) or the pharmaceutically acceptable salt thereof, wherein each R¹ is independently C₂₋₆ alkyl.

In some embodiments, provided in the present disclosure is the compound represented by formula (I) or (I-1) or the pharmaceutically acceptable salt thereof, wherein each R¹ is independently ethyl.

In some embodiments, provided in the present disclosure is the compound represented by formula (I) or (I-1) or the pharmaceutically acceptable salt thereof, wherein each R¹ is independently C₁₋₆ haloalkyl.

In some embodiments, provided in the present disclosure is the compound represented by formula (I) or (I-1) or the pharmaceutically acceptable salt thereof, wherein each R¹ is independently C₁₋₃ haloalkyl.

In some embodiments, provided in the present disclosure is the compound represented by formula (I) or (I-1) or the pharmaceutically acceptable salt thereof, wherein each R¹ is independently methyl substituted with one or two or three fluorine atoms.

In some embodiments, provided in the present disclosure is the compound represented by formula (I) or (I-1) or the pharmaceutically acceptable salt thereof, wherein each R¹ is independently -C(O)₂R', and R' and R" are each independently selected from the group consisting of hydrogen, C₁₋₆ alkyl, and C₁₋₆ haloalkyl.

In some embodiments, provided in the present disclosure is the compound represented by formula (I) or (I-1) or the pharmaceutically acceptable salt thereof, wherein each R¹ is independently -C(O)₂R', and R' is selected from the group consisting of H and C₁₋₆ alkyl.

In some embodiments, provided in the present disclosure is the compound represented by formula (I) or (I-1) or the pharmaceutically acceptable salt thereof, wherein each R¹ is independently -C(O)₂R', and R' is selected from the group consisting of methyl and ethyl.

In some embodiments, provided in the present disclosure is the compound represented by formula (I) or (I-1) or the pharmaceutically acceptable salt thereof, wherein each R¹ is independently C₁₋₆ hydroxyalkyl or C₁₋₆ alkyl-O-C₁₋₆ alkylene-.

In some embodiments, provided in the present disclosure is the compound represented by formula (I) or (I-1) or the pharmaceutically acceptable salt thereof, wherein each R¹ is independently C₁₋₃ hydroxyalkyl or C₁₋₃ alkyl-O-C₁₋₃ alkylene-.

In some embodiments, provided in the present disclosure is the compound represented by formula (I) or (I-1) or the pharmaceutically acceptable salt thereof, wherein each R¹ is independently hydroxymethyl or methyl-O-methylene-.

In some embodiments, provided in the present disclosure is the compound represented by formula (I) or (I-1) or the pharmaceutically acceptable salt thereof, wherein each R¹ is independently R'-(O)-alkylene-, and R' is selected from the group consisting of C₁₋₆ alkyl, C₁₋₆ hydroxyalkyl, C₁₋₆ haloalkyl, 3- to 7-membered cycloalkyl, and 3- to 7-membered heterocyclyl.

In some embodiments, provided in the present disclosure is the compound represented by formula (I) or (I-1) or the pharmaceutically acceptable salt thereof, wherein each R¹ is independently R'-(O)-alkylene-, and R' is C₁₋₆ alkyl or C₁₋₆ haloalkyl.

In some embodiments, provided in the present disclosure is the compound represented by formula (I) or (I-1) or the pharmaceutically acceptable salt thereof, wherein each R¹ is independently R'-(O)-alkylene-, and R' is C₁₋₃ alkyl.

In some embodiments, provided in the present disclosure is the compound represented by formula (I) or (I-1) or the pharmaceutically acceptable salt thereof, wherein each R² is independently hydrogen.

In some embodiments, provided in the present disclosure is the compound represented by formula (I) or (I-1) or the pharmaceutically acceptable salt thereof, wherein A⁺ is a monovalent cation selected from the group consisting of H⁺, Na⁺, K⁺, H₄N⁺, Et₃NH⁺, (HOCH₂CH₂)₃NH⁺, and cationic forms of ethylenediamine, piperazine, and triphenylmethylaminomethane.

In some embodiments, provided in the present disclosure is the compound represented by formula (I) or (I-1) or the pharmaceutically acceptable salt thereof, wherein A⁺ is a monovalent cation selected from the group consisting of H⁺, Na⁺, and K⁺.

In some embodiments, provided in the present disclosure is the compound represented by formula (I) or (I-1) or the pharmaceutically acceptable salt thereof, wherein A⁺ is a monovalent cation, and the monovalent cation is Na⁺.

In some embodiments, provided in the present disclosure is the compound represented by formula (I) or (I-1) or the pharmaceutically acceptable salt thereof, wherein A⁺ is a divalent cation selected from the group consisting of Ca²⁺, Mg²⁺, and Zn²⁺.

In some embodiments, provided in the present disclosure is the compound represented by formula (I) or (I-1) or the pharmaceutically acceptable salt thereof, wherein each p is independently selected from the group consisting of 2, 3, and 4.

In some embodiments, provided in the present disclosure is the compound represented by formula (I) or (I-1) or the pharmaceutically acceptable salt thereof, wherein p is 3.

In some embodiments, provided in the present disclosure is the compound represented by formula (I) or (I-1) or the pharmaceutically acceptable salt thereof, wherein each m is independently selected from the group consisting of 2, 3, and 4.

In some embodiments, provided in the present disclosure is the compound represented by formula (I) or (I-1) or the pharmaceutically acceptable salt thereof, wherein m is 2.

In some embodiments, provided in the present disclosure is the compound represented by formula (I) or (I-1) or the pharmaceutically acceptable salt thereof, wherein two R¹ attached to adjacent carbon atoms form together 5-membered cycloalkyl, 6-membered cycloalkyl, or 7-membered cycloalkyl, for example,

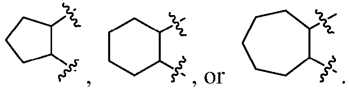

In some embodiments, provided in the present disclosure is the compound represented by formula (I) or (I-1) or the pharmaceutically acceptable salt thereof, wherein two R¹ attached to adjacent carbon atoms form together 6-membered heterocycloalkyl, for example, 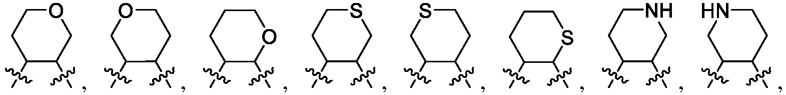 or

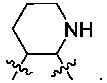

In some embodiments, provided in the present disclosure is the compound represented by formula (I) or (I-1) or the pharmaceutically acceptable salt thereof, wherein two R¹ attached to adjacent carbon atoms form together 5-membered heterocycloalkyl, for example,

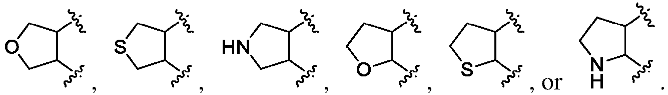

In an optional embodiment, the compound represented by formula (I) or the pharmaceutically acceptable salt thereof provided in the present disclosure is a compound represented by formula (I-1-A), formula (I-1-B), formula (I-1-C), formula (I-1-D), or formula (I-1-E) or a pharmaceutically acceptable salt thereof: 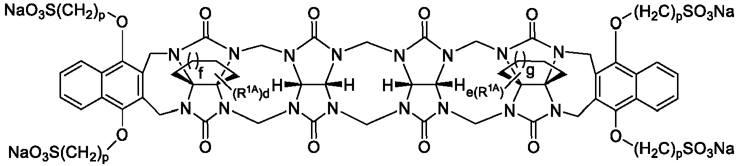 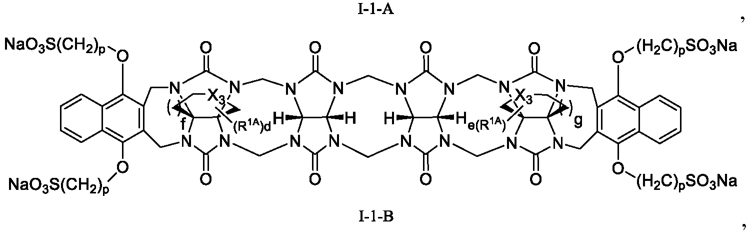 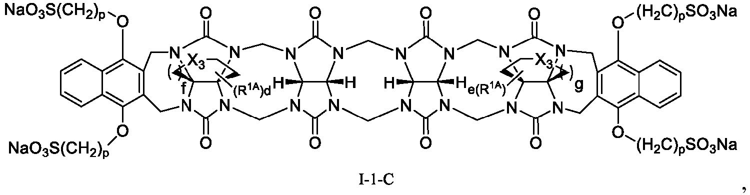 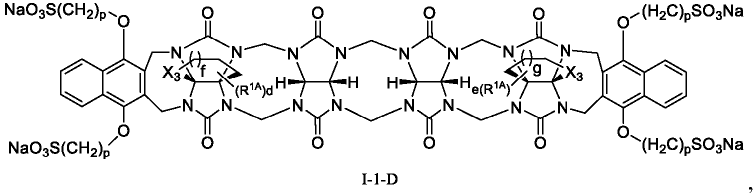 or 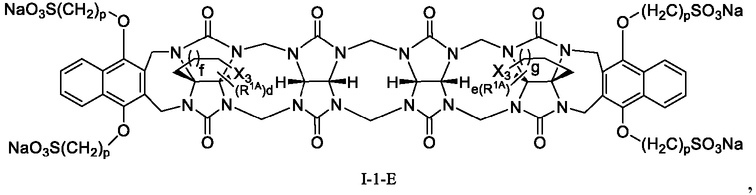
wherein each X₃ is independently selected from the group consisting of O, S, and NH;
each d is independently selected from the group consisting of 0, 1, 2, 3, 4, 5, 6, 7, and 8;
each e is independently selected from the group consisting of 0, 1, 2, 3, 4, 5, 6, 7, and 8;
each f is independently selected from the group consisting of 0, 1, and 2;
each g is independently selected from the group consisting of 0, 1, and 2;
R^{1A} and p are as defined in the foregoing.

In some embodiments, provided in the present disclosure is the compound represented by formula (I), formula (I-1), formula (I-1-A), formula (I-1-B), formula (I-1-C), formula (I-1-D), or formula (I-1-E) or the pharmaceutically acceptable salt thereof, wherein each R^{1A} is independently selected from the group consisting of halogen, cyano, amino, C₁₋₆ alkyl, and C₁₋₆ alkoxy.

In some embodiments, provided in the present disclosure is the compound represented by formula (I), formula (I-1), formula (I-1-A), formula (I-1-B), formula (I-1-C), formula (I-1-D), or formula (I-1-E) or the pharmaceutically acceptable salt thereof, wherein each R^{1A} is independently selected from the group consisting of halogen, C₁₋₆ alkyl, and C₁₋₆ alkoxy.

In some embodiments, provided in the present disclosure is the compound represented by formula (I), formula (I-1), formula (I-1-A), formula (I-1-B), formula (I-1-C), formula (I-1-D), or formula (I-1-E) or the pharmaceutically acceptable salt thereof, wherein each R^{1A} is independently selected from the group consisting of fluorine, chlorine, methyl, ethyl, methoxy, and ethoxy.

The compound represented by formula (I), formula (1-1), formula (I-1-A), formula (I-1-B), formula (I-1-C), formula (I-1-D), or formula (I-1-E) or the pharmaceutically acceptable salt thereof provided in the present disclosure is selected from the group consisting of: 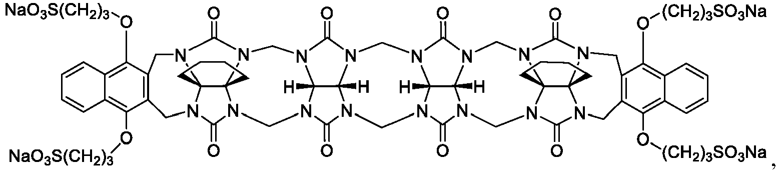 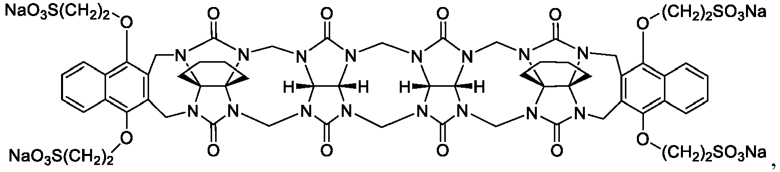 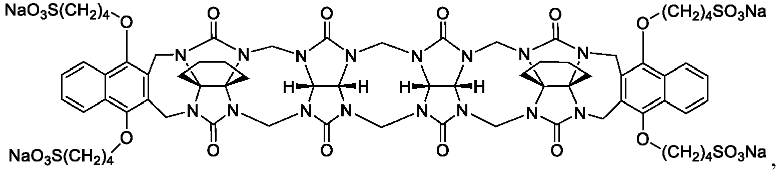 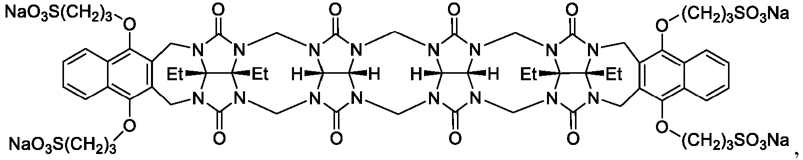 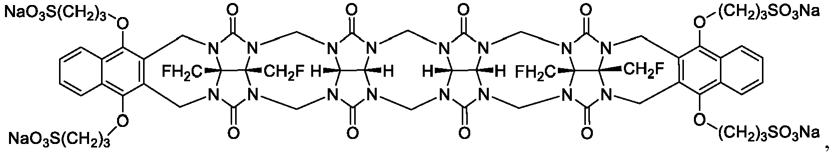 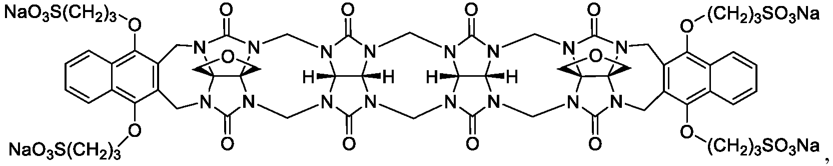 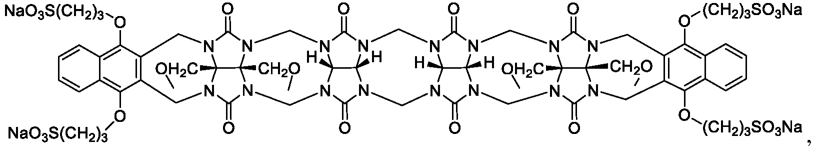 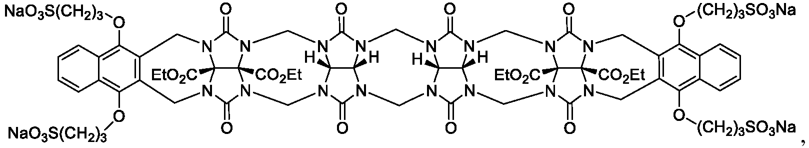 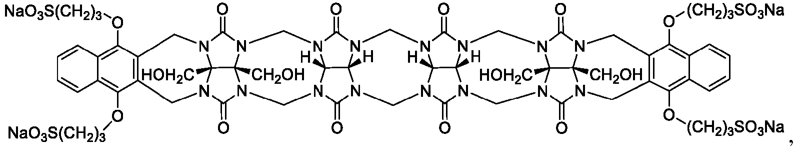 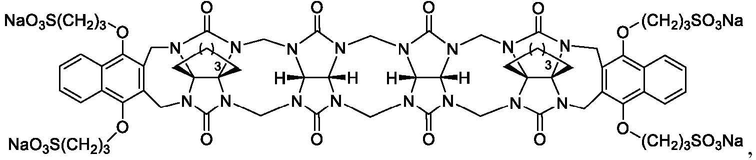 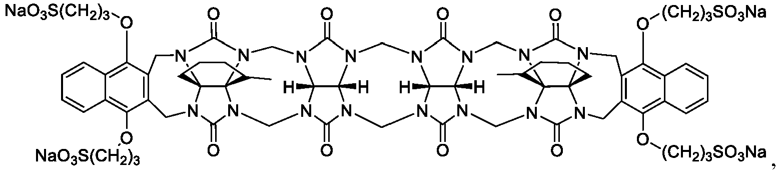 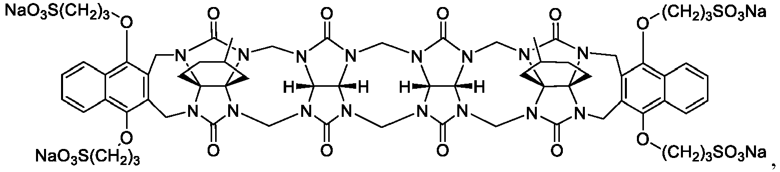 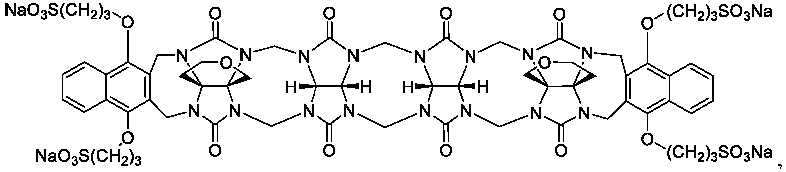 and

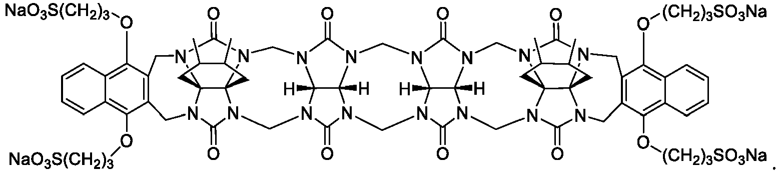

In another aspect, the present disclosure provides a compound represented by formula (II) or a pharmaceutically acceptable salt thereof: 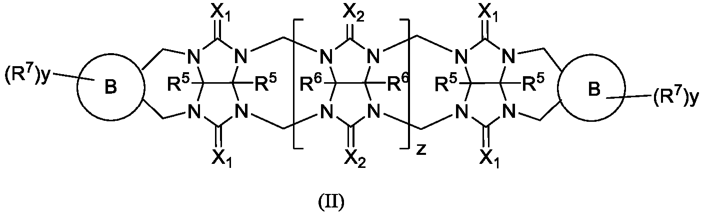
wherein X₁ and X₂ are each independently selected from the group consisting of O, S, and -NH-, provided that X₁ and X₂ are not simultaneously O;
each R⁵ is independently selected from the group consisting of C₁₋₆ alkyl, C₁₋₆ haloalkyl, C₁₋₆ hydroxyalkyl, -C(O)₂R', R'-(O)-alkylene-, hydroxy, NR'(R"), 3- to 7-membered cycloalkyl, and 3- to 7-membered heterocyclyl; or two R⁵ attached to adjacent carbon atoms form together 3- to 10-membered cycloalkyl or 3- to 10-membered heterocyclyl; R⁵ is optionally substituted with one or more R^{3A};
each R⁶ is independently selected from the group consisting of hydrogen, C₁₋₆ alkyl, C₁₋₆ haloalkyl, C₁₋₆ hydroxyalkyl, -C(O)₂R', R'-(O)-alkylene-, hydroxy, NR'(R"), 3- to 7-membered cycloalkyl, and 3- to 7-membered heterocyclyl; or two R⁶ attached to adjacent carbon atoms form together 3- to 10-membered cycloalkyl or 3- to 10-membered heterocyclyl; R⁶ is optionally substituted with one or more R^{6A};
ring B is selected from the group consisting of 5- to 12-membered aryl and 5- to 12-membered heteroaryl;
each R⁷ is independently selected from the group consisting of halogen, C₁₋₆ alkyl, hydroxy, nitro, cyano, -C(O)₂R', NR'(R"), R'-(O)-alkylene-, 3- to 7-membered cycloalkyl, 3- to 7-membered heterocyclyl, and 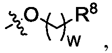 and at least one R⁷ is 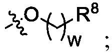 R⁷ is optionally substituted with one or more R^{7A};
R⁸ is 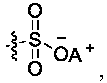 and A⁺ is a monovalent or divalent cation;
R^{5A}, R^{6A}, and R^{7A} are each independently selected from the group consisting of halogen, cyano, nitro, amino, C₁₋₆ alkyl, and C₁₋₆ alkoxy;
R' and R" are each independently selected from the group consisting of hydrogen, C₁₋₆ alkyl, C₁₋₆ hydroxyalkyl, C₁₋₆ haloalkyl, 3- to 7-membered cycloalkyl, and 3- to 7-membered heterocyclyl;
wherein y and z are each independently selected from the group consisting of 1, 2, 3, 4, and 5;
w is selected from the group consisting of 1, 2, 3, 4, 5, and 6.

In some embodiments, provided in the present disclosure is the compound represented by formula (II) or the pharmaceutically acceptable salt thereof, wherein ring B is selected from the group consisting of phenyl and naphthyl.

In some embodiments, provided in the present disclosure is the compound represented by formula (II) or the pharmaceutically acceptable salt thereof, wherein ring B is naphthyl.

In some embodiments, the compound represented by formula (II) or the pharmaceutically acceptable salt thereof provided in the present disclosure is a compound represented by formula (II-1) or a pharmaceutically acceptable salt thereof: 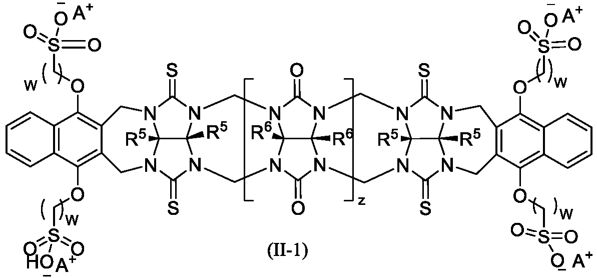 wherein R⁵, R⁶, w, z, and A⁺ are as defined in the compound represented by formula (II).

In some embodiments, the compound represented by formula (II) or the pharmaceutically acceptable salt thereof provided in the present disclosure is a compound represented by formula (II-2) or a pharmaceutically acceptable salt thereof: 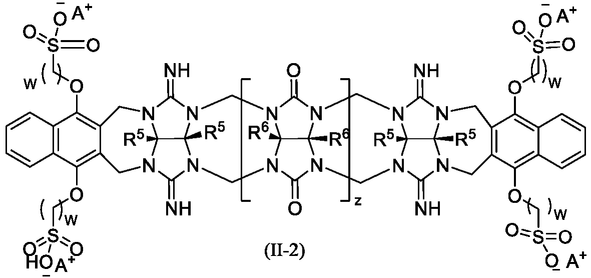 wherein R⁵, R⁶, w, z, and A⁺ are as defined in the compound represented by formula (II).

In some embodiments, the compound represented by formula (II) or the pharmaceutically acceptable salt thereof provided in the present disclosure is a compound represented by formula (II-3) or a pharmaceutically acceptable salt thereof: 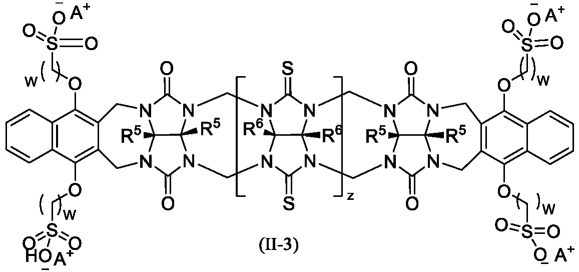 wherein R⁵, R⁶, w, z, and A⁺ are as defined in the compound represented by formula (II).

In some embodiments, provided in the present disclosure is the compound represented by formula (II), (II-1), (II-2), or (II-3) or the pharmaceutically acceptable salt thereof, wherein each R⁵ is independently C₁₋₆ alkyl or C₁₋₆ haloalkyl.

In some embodiments, provided in the present disclosure is the compound represented by formula (II), (II-1), (II-2), or (II-3) or the pharmaceutically acceptable salt thereof, wherein each R⁵ is independently C₁₋₃ alkyl.

In some embodiments, provided in the present disclosure is the compound represented by formula (II), (II-1), (II-2), or (II-3) or the pharmaceutically acceptable salt thereof, wherein each R⁵ is independently methyl.

In some embodiments, provided in the present disclosure is the compound represented by formula (II), (II-1), (II-2), or (II-3) or the pharmaceutically acceptable salt thereof, wherein each R⁶ is independently hydrogen.

In some embodiments, provided in the present disclosure is the compound represented by formula (II), (II-1), (II-2), or (II-3) or the pharmaceutically acceptable salt thereof, wherein A⁺ is selected from the group consisting of a monovalent cation selected from the group consisting of H⁺, Na⁺, K⁺, H₄N⁺, Et₃NH⁺, (HOCH₂CH₂)₃NH⁺, and cationic forms of ethylenediamine, piperazine, and triphenylmethylaminomethane.

In some embodiments, provided in the present disclosure is the compound represented by formula (II), (II-1), (II-2), or (II-3) or the pharmaceutically acceptable salt thereof, wherein A⁺ is a monovalent cation selected from the group consisting of H⁺, Na⁺, and K⁺.

In some embodiments, provided in the present disclosure is the compound represented by formula (II), (II-1), (II-2), or (II-3) or the pharmaceutically acceptable salt thereof, wherein A⁺ is a divalent cation selected from the group consisting of Ca²⁺, Mg²⁺, and Zn²⁺.

In some embodiments, provided in the present disclosure is the compound represented by formula (II), (II-1), (II-2), or (II-3) or the pharmaceutically acceptable salt thereof, wherein each w is independently selected from the group consisting of 2, 3, and 4.

In some embodiments, provided in the present disclosure is the compound represented by formula (II), (II-1), (II-2), or (II-3) or the pharmaceutically acceptable salt thereof, wherein each w is independently 3.

In some embodiments, provided in the present disclosure is the compound represented by formula (II), (II-1), (II-2), or (II-3) or the pharmaceutically acceptable salt thereof, wherein each z is independently selected from the group consisting of 2, 3, and 4.

In some embodiments, provided in the present disclosure is the compound represented by formula (II), (II-1), (II-2), or (II-3) or the pharmaceutically acceptable salt thereof, wherein each z is independently 2.

In some embodiments, the compound represented by formula (II), (II-1), (II-2), or (II-3) or the pharmaceutically acceptable salt thereof provided in the present disclosure is selected from the group consisting of: 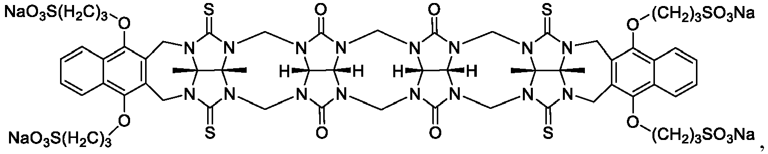 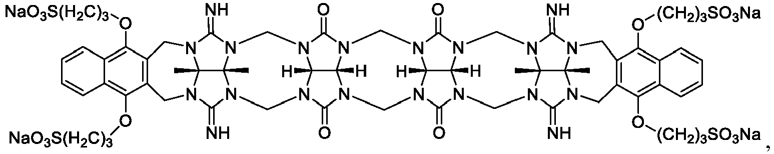 and

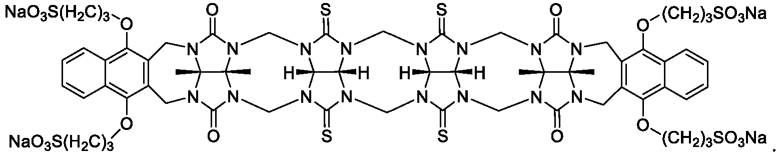

In another aspect, the present disclosure provides a compound represented by formula (III) or a pharmaceutically acceptable salt thereof: 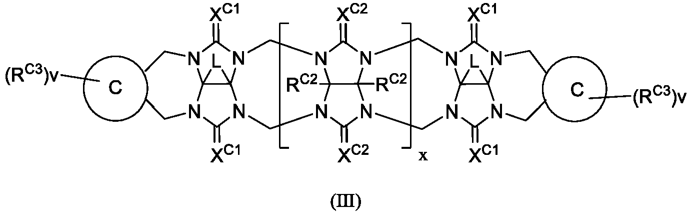 wherein:
X^{C1} and X^{C2} are each independently selected from the group consisting of O, S, and -NH-;
R^{C2} is selected from the group consisting of hydrogen, C₁₋₆ alkyl, C₁₋₆ haloalkyl, C₁₋₆ hydroxyalkyl, -C(O)₂R', R'-(O)-alkylene-, hydroxy, NR'(R"), 3- to 7-membered cycloalkyl, and 3- to 7-membered heterocyclyl; or two R^{C2} attached to adjacent carbon atoms form together 3- to 10-membered cycloalkyl or 3- to 10-membered heterocyclyl;
R^{C2} is optionally substituted with one or more halogen, cyano, nitro, amino, C₁₋₆ alkyl, or C₁₋₆ alkoxy groups;
ring C is selected from the group consisting of 6- to 18-membered aryl and 5- to 18-membered heteroaryl;
each R^{C3} is independently 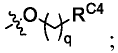
each R^{C4} is independently 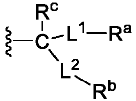 , a carboxylic acid group, a -carboxylate-cation, a phosphoric acid group, a -phosphate-cation, a sulfonic acid group, or a -sulfonate-cation;
L, L¹, and L² are identical or different and are each independently alkylene or heteroalkylene, and the alkylene or heteroalkylene is optionally substituted with one or more halogen, cyano, nitro, amino, C₁₋₆ alkyl, or C₁₋₆ alkoxy groups;
each R^{c} is independently selected from the group consisting of hydrogen, halogen, cyano, nitro, amino, C₁₋₆ alkyl, and C₁₋₆ alkoxy;
R^{a} and R^{b} are identical or different and are each independently selected from the group consisting of a carboxylic acid group, a -carboxylate-cation, a phosphoric acid group, a -phosphate-cation, a sulfonic acid group, and a -sulfonate-cation;
R' and R" are each independently selected from the group consisting of hydrogen, C₁₋₆ alkyl, C₁₋₆ hydroxyalkyl, C₁₋₆ haloalkyl, 3- to 7-membered cycloalkyl, and 3- to 7-membered heterocyclyl;
x is selected from the group consisting of 1, 2, 3, 4, and 5;
each v is independently selected from the group consisting of 1, 2, 3, 4, and 5;
each q is independently selected from the group consisting of 1, 2, 3, 4, 5, and 6.

In some embodiments, provided in the present disclosure is the compound represented by formula (III) or the pharmaceutically acceptable salt thereof, wherein X^{C1} and X^{C2} are identical and are selected from the group consisting of O, S, and -NH-. In certain embodiments, X^{C1} and X^{C2} are both O.

In some embodiments, provided in the present disclosure is the compound represented by formula (III) or the pharmaceutically acceptable salt thereof, wherein each L is independently C₁₋₆ alkylene.

In some embodiments, provided in the present disclosure is the compound represented by formula (III) or the pharmaceutically acceptable salt thereof, wherein ring C is selected from the group consisting of a benzene ring, a naphthalene ring, and an anthracene ring, preferably from the group consisting of a naphthalene ring and an anthracene ring.

In some embodiments, the compound represented by formula (III) or the pharmaceutically acceptable salt thereof provided in the present disclosure is selected from the group consisting of compounds represented by formula (III-A), formula (III-B), formula (III-C), and formula (III-D) or pharmaceutically acceptable salts thereof: 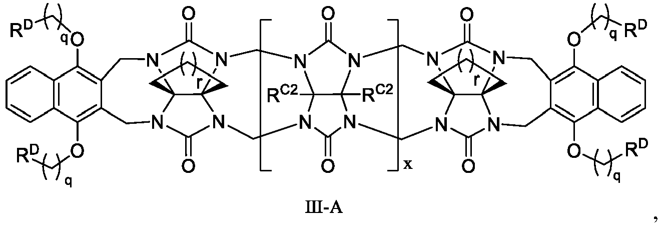 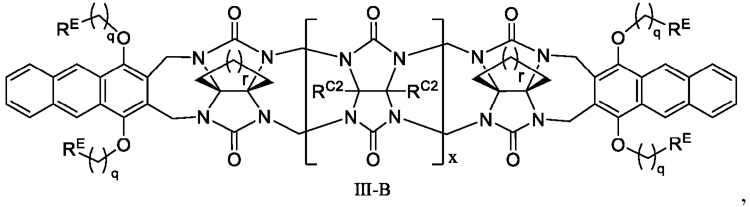 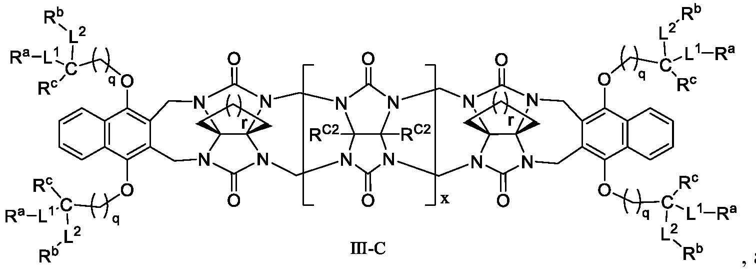 and 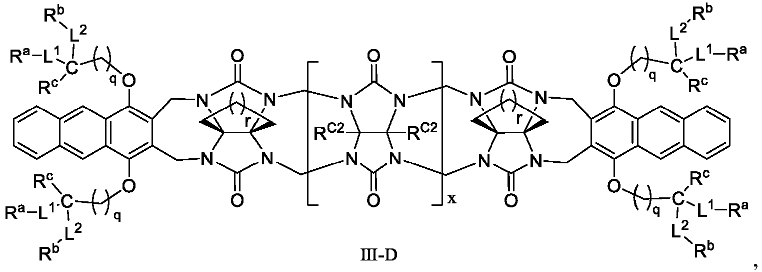 wherein:
each R^{D} is independently selected from the group consisting of a carboxylic acid group, a -carboxylate-cation, a phosphoric acid group, and a -phosphate-cation;
each R^{E} is independently selected from the group consisting of a carboxylic acid group, a -carboxylate-cation, a phosphoric acid group, a -phosphate-cation, a sulfonic acid group, and a -sulfonate-cation;
r is selected from the group consisting of 1, 2, and 3;
R^{C2} q, x, L¹, L², R^{a}, R^{b}, and R^{c} are as defined in the foregoing.

In some embodiments, provided in the present disclosure is the compound represented by formula (III), formula (III-A), formula (III-B), formula (III-C), or formula (III-D) or the pharmaceutically acceptable salt thereof, wherein L₁ and L₂ are identical or different and are each independently C₁₋₆ alkylene.

In some embodiments, provided in the present disclosure is the compound represented by formula (III), formula (III-A), formula (III-B), formula (III-C), or formula (III-D) or the pharmaceutically acceptable salt thereof, wherein each R^{D} is independently selected from the group consisting of a carboxylic acid group and a -carboxylate-cation.

In some embodiments, provided in the present disclosure is the compound represented by formula (III), formula (III-A), formula (III-B), formula (III-C), or formula (III-D) or the pharmaceutically acceptable salt thereof, wherein each R^{E} is independently selected from the group consisting of a carboxylic acid group, a -carboxylate-cation, a sulfonic acid group, and a -sulfonate-cation.

In some embodiments, provided in the present disclosure is the compound represented by formula (III), formula (III-A), formula (III-B), formula (III-C), or formula (III-D) or the pharmaceutically acceptable salt thereof, wherein R^{a} and R^{b} are identical or different and are each independently selected from the group consisting of a carboxylic acid group, a -carboxylate-cation, a sulfonic acid group, and a -sulfonate-cation.

In some embodiments, the compound represented by formula (III) or the pharmaceutically acceptable salt thereof provided in the present disclosure is selected from the group consisting of compounds represented by formula (III-A-1), formula (III-B-1), formula (III-B-2), formula (III-C-1), formula (III-C-2), formula (III-C-3), formula (III-D-1), formula (III-D-2), and formula (III-D-3) or pharmaceutically acceptable salts thereof: 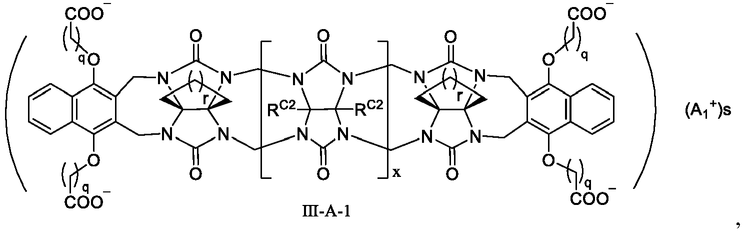 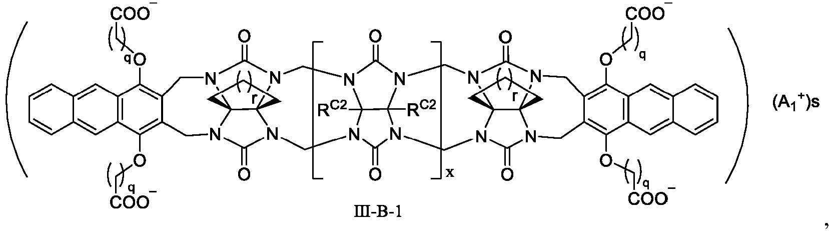 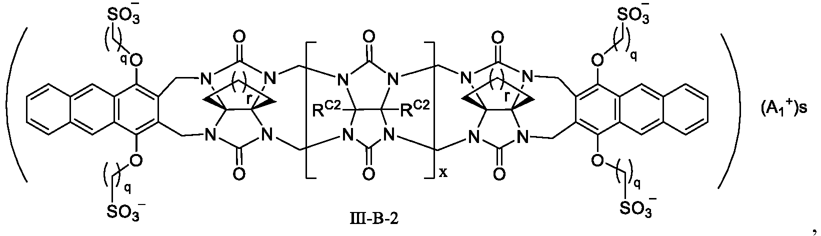 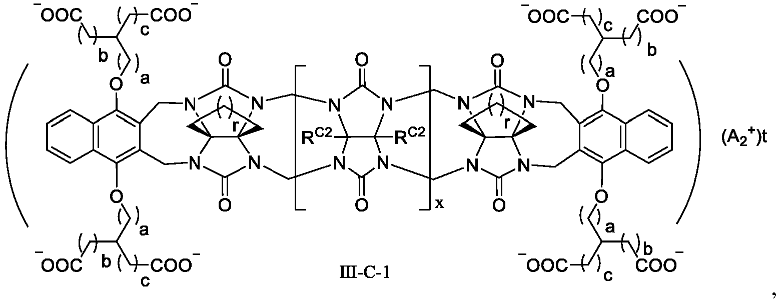 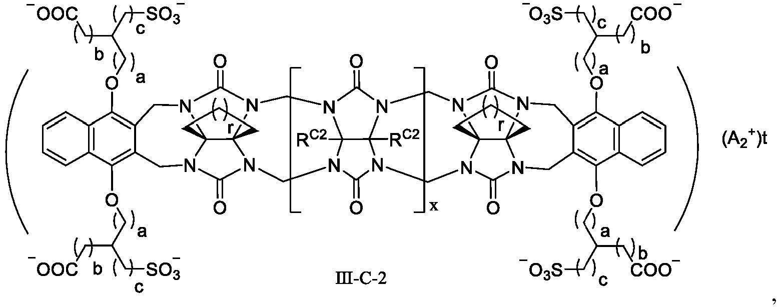 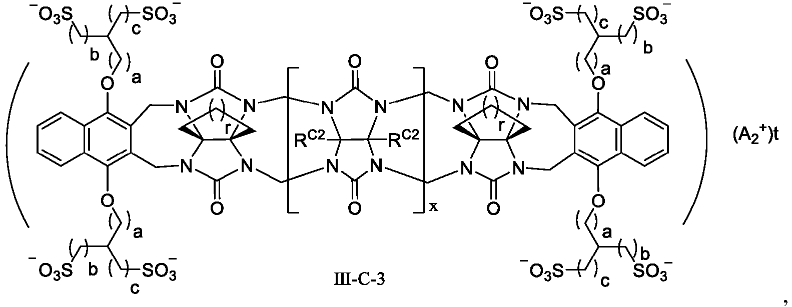 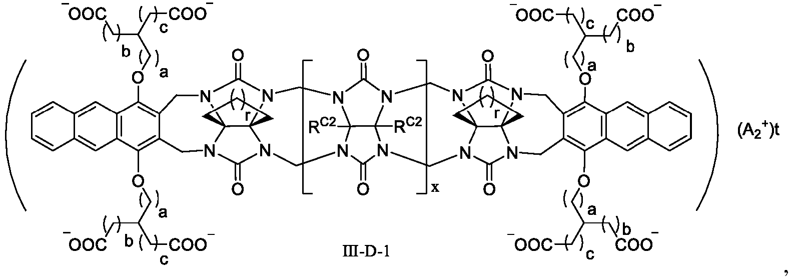 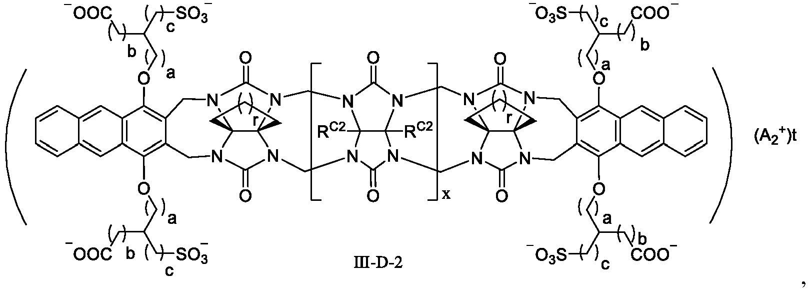 and 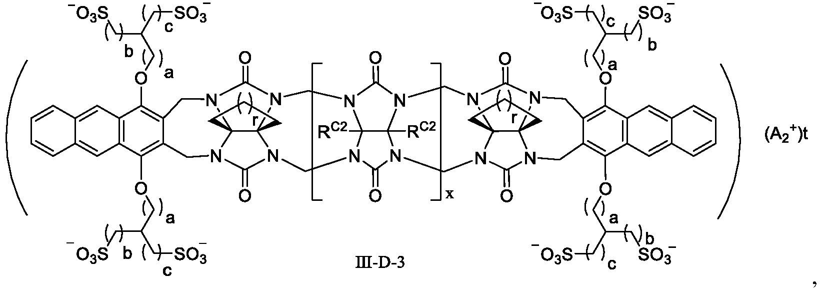 wherein:
each a is independently selected from the group consisting of 1, 2, 3, and 4;
b is selected from the group consisting of 1, 2, 3, and 4;
c is selected from the group consisting of 1, 2, 3, and 4;
A₁⁺ or A₂⁺ is identical or different and is each independently selected from the group consisting of a monovalent cation and a divalent cation;
when A₁⁺ is a monovalent cation, s is 4;
when A₁⁺ is a divalent cation, s is 2;
when A₂⁺ is a monovalent cation, t is 8;
when A₂⁺ is a divalent cation, t is 4;
R^{C2}, q, x, and r are as defined in the foregoing.

In some embodiments, provided in the present disclosure is the compound represented by formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof, wherein each R^{C2} is independently hydrogen.

In some embodiments, provided in the present disclosure is the compound represented by formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof, wherein x is selected from the group consisting of 2, 3, and 4. In certain embodiments, x is selected from the group consisting of 2 and 3. In certain embodiments, x is 2.

In some embodiments, provided in the present disclosure is the compound represented by formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof, wherein each q is independently selected from the group consisting of 2, 3, and 4. In certain embodiments, q is selected from the group consisting of 3 and 4. In certain embodiments, q is 3.

In some embodiments, provided in the present disclosure is the compound represented by formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof, wherein r is selected from the group consisting of 2 and 3. In certain embodiments, r is 2.

In some embodiments, provided in the present disclosure is the compound represented by formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof, wherein a, b, and c are identical or different and are each independently selected from the group consisting of 1, 2, and 3; preferably, a, b, and c are all 1.

In some embodiments, provided in the present disclosure is the compound represented by formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof, wherein A₁⁺ and A₂⁺ are each independently a monovalent cation selected from the group consisting of H⁺, Na⁺, K⁺, H₄N⁺, Et₃NH⁺, (HOCH₂CH₂)₃NH⁺, and cationic forms of ethylenediamine, piperazine, and triphenylmethylaminomethane. In certain embodiments, the monovalent cation is selected from the group consisting of H⁺, Na⁺, and K⁺. In certain embodiments, the monovalent cation is Na⁺.

In some embodiments, provided in the present disclosure is the compound represented by formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof, wherein A₁⁺ and A₂⁺ are each independently a divalent cation selected from the group consisting of Ca²⁺, Mg²⁺, and Zn²⁺.

In some embodiments, the compound represented by formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof provided in the present disclosure is selected from the group consisting of: 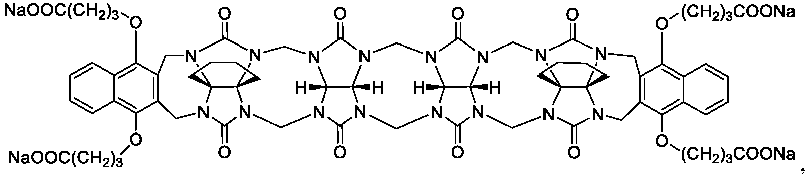 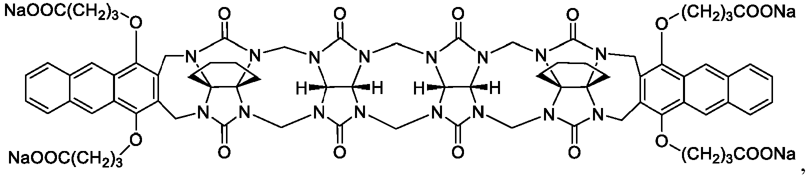 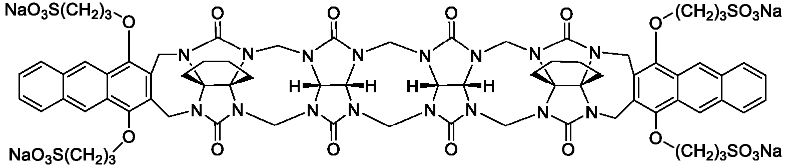 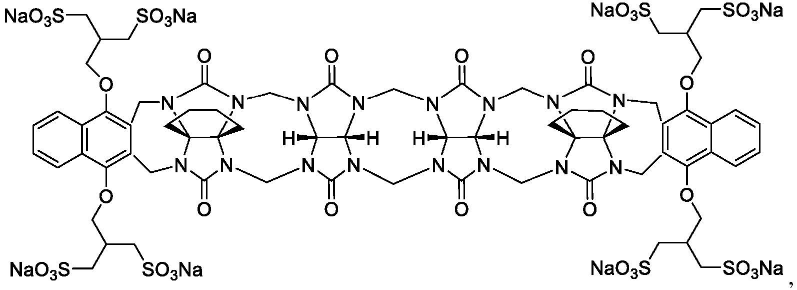 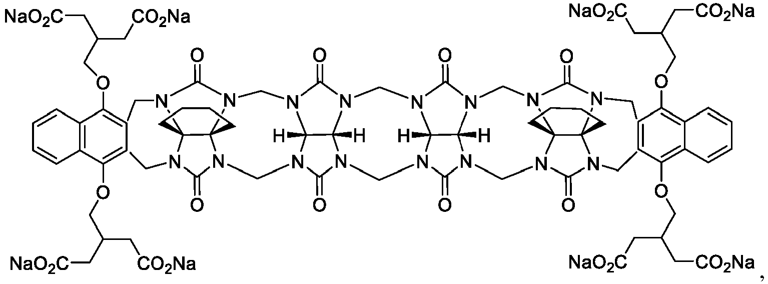 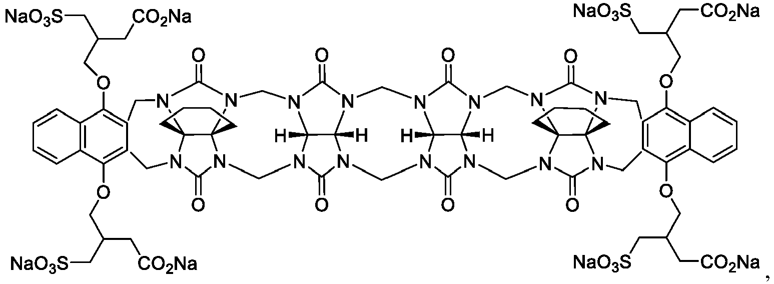 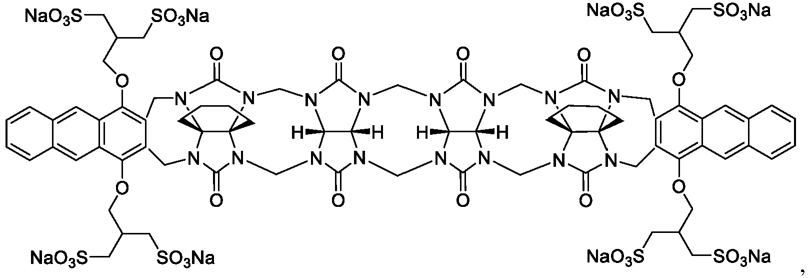 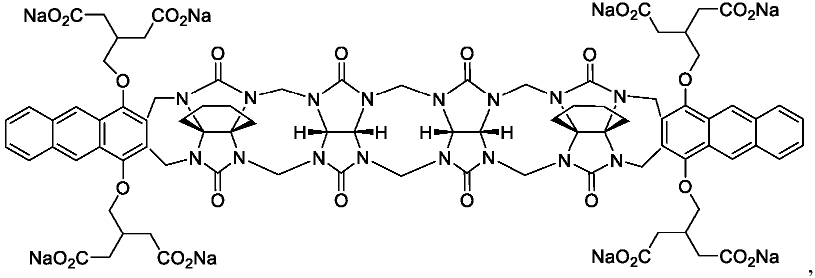 and

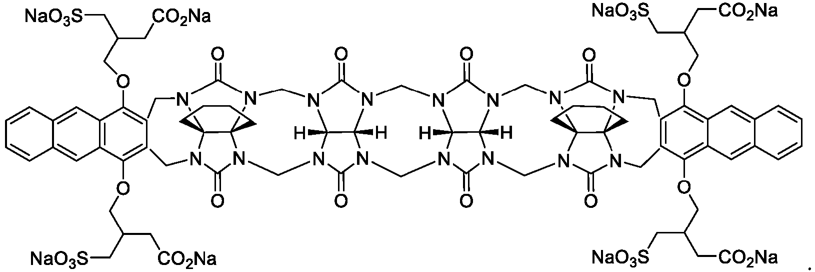

In another aspect, the present disclosure provides a compound represented by formula (IV) or a pharmaceutically acceptable salt thereof: 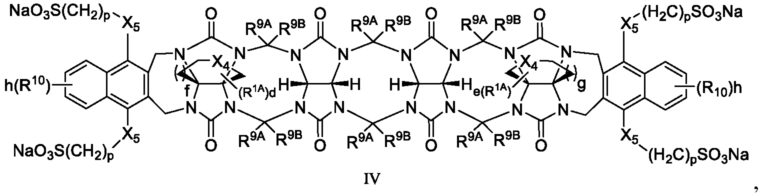
wherein each X₄ is independently selected from the group consisting of O, S, and NH; each X₅ is independently selected from the group consisting of O, S, and NH;
each R^{9A} is independently selected from the group consisting of hydrogen, halogen, cyano, nitro, amino, carboxyl, sulfhydryl, C₁₋₆ alkyl, and C₁₋₆ alkoxy, and the alkyl or alkoxy is optionally substituted with one or more halogen, cyano, nitro, amino, carboxyl, or sulfhydryl groups;
each R^{9B} is independently selected from the group consisting of hydrogen, halogen, cyano, nitro, amino, carboxyl, sulfhydryl, C₁₋₆ alkyl, and C₁₋₆ alkoxy, and the alkyl or alkoxy is optionally substituted with one or more halogen, cyano, nitro, amino, carboxyl, or sulfhydryl groups;
each R¹⁰ is independently selected from the group consisting of hydrogen, halogen, cyano, nitro, amino, carboxyl, sulfhydryl, C₁₋₆ alkyl, and C₁₋₆ alkoxy, and the alkyl or alkoxy is optionally substituted with one or more halogen, cyano, nitro, amino, carboxyl, or sulfhydryl groups;
each h is independently selected from the group consisting of 1, 2, 3, and 4;
R^{1A}, d, e, f, g, and p are as defined in the foregoing.

In some embodiments, X₄ is simultaneously O. Alternatively, in some embodiments, X₄ is simultaneously S. Alternatively, in some embodiments, X₄ is simultaneously NH.

In some embodiments, X₅ is simultaneously O. Alternatively, in some embodiments, X₅ is simultaneously S. Alternatively, in some embodiments, X₅ is simultaneously NH.

In some embodiments, the compound represented by formula (IV) or the pharmaceutically acceptable salt thereof provided in the present disclosure is selected from the group consisting of compounds represented by formula (IV-A), formula (IV-B), and formula (IV-C) or pharmaceutically acceptable salts thereof: 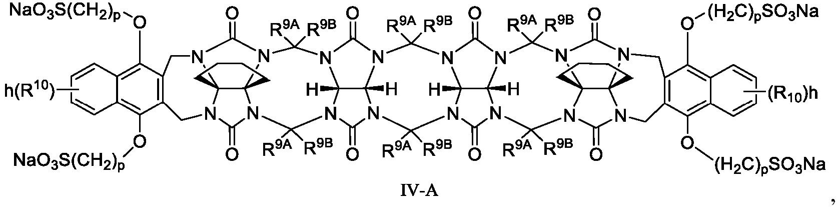 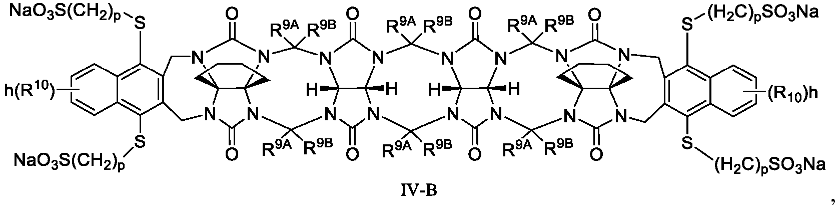 and 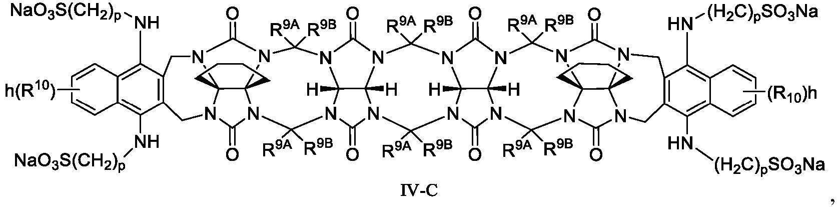 wherein R^{9A}, R^{9B}, R¹⁰, h, and p are as defined in the foregoing.

In some embodiments, each R^{9A} is independently selected from the group consisting of hydrogen, C₁₋₆ alkyl, and C₁₋₆ alkoxy. In certain specific embodiments, each R^{9A} is independently selected from the group consisting of hydrogen, methyl, ethyl, methoxy, and ethoxy.

In some embodiments, each R^{9B} is independently selected from the group consisting of hydrogen, C₁₋₆ alkyl, and C₁₋₆ alkoxy. In certain specific embodiments, each R^{9B} is independently selected from the group consisting of hydrogen, methyl, ethyl, methoxy, and ethoxy.

In some embodiments, each R¹⁰ is independently selected from the group consisting of hydrogen, C₁₋₆ alkyl, and C₁₋₆ alkoxy. In certain specific embodiments, each R¹⁰ is independently selected from the group consisting of hydrogen, methyl, ethyl, methoxy, and ethoxy.

In some embodiments, the compound represented by formula (IV), formula (IV-A), formula (IV-B), or formula (IV-C) or the pharmaceutically acceptable salt thereof provided in the present disclosure is selected from the group consisting of: 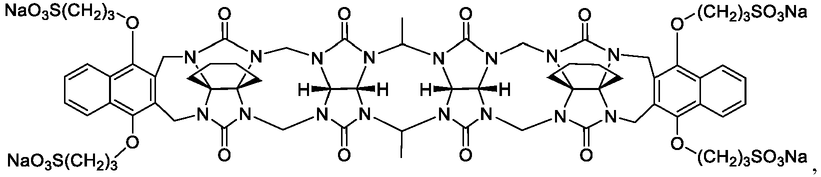 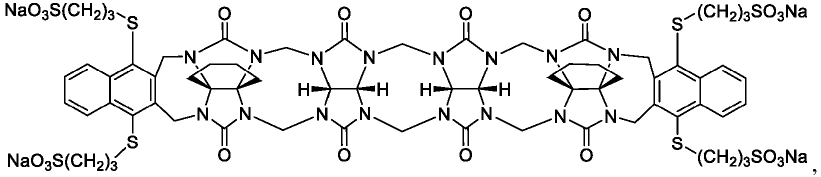 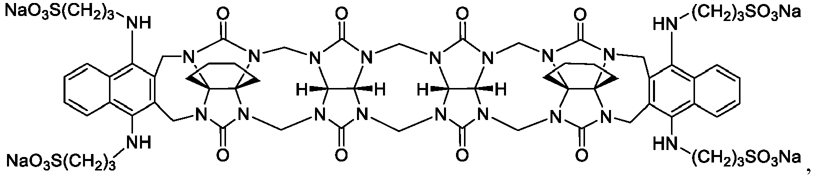 and

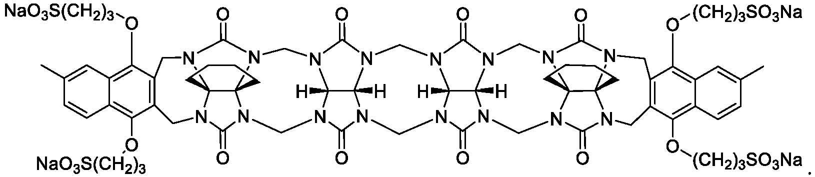

In another aspect, the present disclosure provides a preparation method for the compound represented by formula (I-1) or the pharmaceutically acceptable salt thereof, the preparation method comprising a step of reacting a compound represented by formula (C) with a compound represented by formula (B) in an acidic environment:

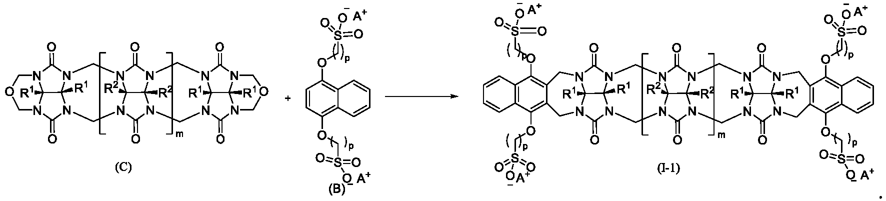

In another aspect, the present disclosure provides a preparation method for the compound represented by formula (II-1) or the pharmaceutically acceptable salt thereof, the preparation method comprising a step of reacting a compound represented by formula (D) with a compound represented by formula (B) in an acidic environment:

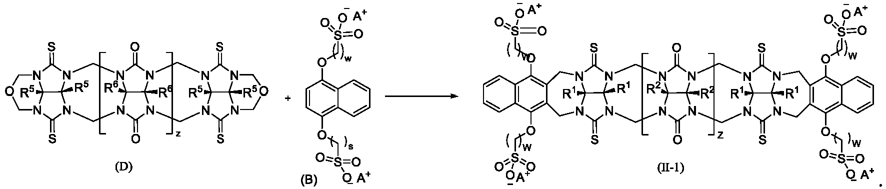

The reagent providing the acidic environment in the present disclosure may be selected from the group consisting of an organic or inorganic acid, for example, trifluoroacetic acid.

In another aspect, the present disclosure provides a composition comprising the compound represented by formula (I), (1-1), (II), (II-1), (II-2), (II-3), formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), or formula (III-D-3) or the pharmaceutically acceptable salt thereof or an isotopically substituted form thereof and a pharmaceutically acceptable excipient thereof.

In some embodiments, a unit dose of the pharmaceutical composition is 0.001 mg-1000 mg.

In certain embodiments, the pharmaceutical composition comprises 0.01-99.99% of the aforementioned compound or the pharmaceutically acceptable salt thereof or the isotopically substituted form thereof based on the total weight of the composition. In certain embodiments, the pharmaceutical composition comprises 0.1-99.9% of the aforementioned compound or the pharmaceutically acceptable salt thereof or the isotopically substituted form thereof. In certain embodiments, the pharmaceutical composition comprises 0.5%-99.5% of the aforementioned compound or the pharmaceutically acceptable salt thereof or the isotopically substituted form thereof. In certain embodiments, the pharmaceutical composition comprises 1%-99% of the aforementioned compound or the pharmaceutically acceptable salt thereof or the isotopically substituted form thereof. In certain embodiments, the pharmaceutical composition comprises 2%-98% of the aforementioned compound or the pharmaceutically acceptable salt thereof or the isotopically substituted form thereof.

In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In another aspect, the present disclosure provides use of the compound represented by formula (I), formula (1-1), formula (I-1-A), formula (I-1-B), formula (I-1-C), formula (I-1-D), formula (I-1-E), formula (II), formula (II-1), formula (II-2), formula (II-3), formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), formula (III-D-3), formula (IV), formula (IV-A), formula (IV-B), or formula (IV-C) or the pharmaceutically acceptable salt thereof, the isotopically substituted form, or the composition described above in the preparation of a medicament for treating or preventing a disease or disorder selected from the group consisting of a proliferative disease, a hematological cancer, a cardiovascular related disease, and an infectious disease.

In another aspect, the present disclosure provides a method for treating or preventing a disease or disorder selected from the group consisting of a proliferative disease, a hematological cancer, a cardiovascular related disease, and an infectious disease, the method comprising administering to a patient the compound represented by formula (I), formula (1-1), formula (I-1-A), formula (I-1-B), formula (I-1-C), formula (I-1-D), formula (I-1-E), formula (II), formula (II-1), formula (II-2), formula (II-3), formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), formula (III-D-3), formula (IV), formula (IV-A), formula (IV-B), or formula (IV-C) or the pharmaceutically acceptable salt thereof, the isotopically substituted form, or the composition described above.

In another aspect, the present disclosure provides use of the compound represented by formula (I), formula (1-1), formula (I-1-A), formula (I-1-B), formula (I-1-C), formula (I-1-D), formula (I-1-E), formula (II), formula (II-1), formula (II-2), formula (II-3), formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), formula (III-D-3), formula (IV), formula (IV-A), formula (IV-B), or formula (IV-C) or the pharmaceutically acceptable salt thereof, the isotopically substituted form, or the composition described above in the preparation of a medicament for reversing drug-induced neuromuscular blockade and/or anesthesia.

In another aspect, the present disclosure provides a method of reversing drug-induced neuromuscular blockade and/or anesthesia, the method comprising administering to a patient the compound represented by formula (I), formula (1-1), formula (I-1-A), formula (I-1-B), formula (I-1-C), formula (I-1-D), formula (I-1-E), formula (II), formula (II-1), formula (II-2), formula (II-3), formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), formula (III-D-3), formula (IV), formula (IV-A), formula (IV-B), or formula (IV-C) or the pharmaceutically acceptable salt thereof, the isotopically substituted form, or the composition described above.

The proliferative disease described in the present disclosure is selected from the group consisting of fibrosarcoma, myxosarcoma, liposarcoma, chondrosarcoma, osteosarcoma, chordoma, angiosarcoma, endotheliosarcoma, lymphangiosarcoma, pseudomyxoma peritonei, lymphangiosarcoma, synovioma, synovial sarcoma, colonal sarcoma, mesothelioma, mesothelioma, breast cancer, ovarian cancer, prostate cancer, squamous cell carcinoma, basal cell carcinoma, adenocarcinoma, head and neck cancer, sweat gland carcinoma, sebaceous carcinoma, papillary carcinoma, papillary adenocarcinoma, cystadenocarcinoma, medullary cancer, bronchogenic carcinoma, renal cell carcinoma, liver cancer, cholangiocarcinoma, choriocarcinoma, seminoma, embryonal carcinoma, Wilm's tumor, cervical cancer, testicular tumor, lung cancer, small cell lung cancer, bladder cancer, epithelial carcinoma, neuroglioma, astrocytoma, medulloblastoma, craniopharyngioma, ependymoma, pinealoma, hemangioblastoma, urinoma, oligodendroglioma, meningioma, melanoma, neuroblastoma, retinoblastoma, leukemia, lymphoma, multiple myeloma, thymoma, Waldenström macroglobulinemia, and heavy chain disease.

The hematological cancer described in the present disclosure is selected from the group consisting of leukemia, lymphoma, and myeloma.

In another aspect, the present disclosure provides use of the compound represented by formula (I), formula (1-1), formula (I-1-A), formula (I-1-B), formula (I-1-C), formula (I-1-D), formula (I-1-E), formula (II), formula (II-1), formula (II-2), formula (II-3), formula (III), formula (III-A), formula (III-B), formula (III-C), formula (III-D), formula (III-A-1), formula (III-B-1), formula (III-B-2), formula (III-C-1), formula (III-C-2), formula (III-C-3), formula (III-D-1), formula (III-D-2), formula (III-D-3), formula (IV), formula (IV-A), formula (IV-B), or formula (IV-C) or the pharmaceutically acceptable salt thereof, an isotopically substituted form thereof, or a compound prepared using an aforementioned method as a medicament.

The pharmaceutically acceptable salts of the compounds described in the present disclosure may be selected from the group consisting of inorganic salts and organic salts. The inorganic salts include, but are not limited to, Na⁺, K⁺, Ca²⁺, Mg²⁺, and Zn²⁺. The organic salts include, but are not limited to, H₄N⁺, Et₃NH⁺, (HOCH₂CH₂)₃NH⁺, or cationic forms of ethylenediamine, piperazine, and triphenylmethylaminomethane.

The compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure. The compounds of the present disclosure containing asymmetric carbon atoms may be separated in optically active pure form or racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

In the chemical structures of the compounds described in the present disclosure, a bond " 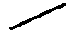 " represents an unspecified configuration; that is, if chiral isomers exist in the chemical structures, the bond " 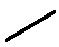 '' may be " 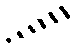 " or " 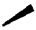 ", or both the configurations of " 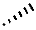 " and " 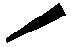 " are included simultaneously.

The compounds and intermediates of the present disclosure may also exist in different tautomeric forms, and all such forms are included within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown below.

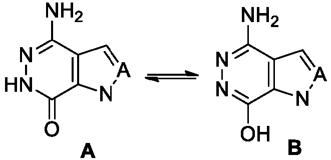

All compounds in the present disclosure can be drawn as form A or form B. All tautomeric forms fall within the scope of the present disclosure. The names of the compounds do not exclude any tautomers.

The present disclosure also includes isotopically labeled compounds that are identical to those recited herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compound of formula (I). Each available hydrogen atom linked to a carbon atom may be independently replaced by a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

"Optionally" or "optional" means that the event or circumstance subsequently described may, but does not necessarily, occur, and this description includes instances where the event or circumstance occurs or does not occur. For example, "C₁₋₆ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

Terms and definitions:
"Pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. "Pharmaceutically acceptable excipient" includes, but is not limited to, any adjuvant, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration (FDA) as acceptable for use in humans or livestock animals.

As used herein, "effective amount" or "therapeutically effective amount" includes an amount sufficient to ameliorate or prevent a symptom or disorder of a medical disorder. An effective amount also means an amount sufficient to allow or facilitate diagnosis. The effective amount for a particular patient or veterinary subject may vary depending on factors such as the disorder to be treated, the general health of the patient, the method and route and dosage of administration, and the severity of side effects. An effective amount may be the maximum dose or administration regimen to avoid significant side effects or toxic effects.

The prefix "Cᵤ₋ᵥ" indicates that the following group has from u to v carbon atoms. For example, "C₁₋₆ alkyl" indicates that the alkyl group has 1 to 6 carbon atoms. Specifically, it may be an alkyl group having 1, 2, 3, 4, 5, or 6 carbon atoms.

The term "alkyl" refers to an unbranched or branched saturated hydrocarbon chain. As used herein, alkyl has 1 to 20 carbon atoms (i.e., C₁₋₂₀ alkyl), 1 to 8 carbon atoms (i.e., C₁₋₈ alkyl), 1 to 6 carbon atoms (i.e., C₁₋₆ alkyl), or 1 to 4 carbon atoms (i.e., C₁₋₄ alkyl). Examples of alkyl groups include methyl, ethyl, propyl, isopropyl, *n*-butyl, *sec*-butyl, isobutyl, *tert*-butyl, pentyl, 2-pentyl, isopentyl, neopentyl, hexyl, 2-hexyl, 3-hexyl, and 3-methylpentyl. When an alkyl residue having a specific number of carbon atoms is named by a chemical name or identified by a molecular formula, all positional isomers having that number of carbon atoms may be included; thus, for example, "butyl" includes *n*-butyl (i.e., -(CH₂)₃CH₃), *sec-*butyl (i.e., -CH(CH₃)CH₂CH₃), isobutyl (i.e., -CH₂CH(CH₃)₂), and *tert*-butyl (i.e., -C(CH₃)₃); and "propyl" includes *n*-propyl (i.e., -(CH₂)₂CH₃) and isopropyl (i.e., -CH(CH₃)₂).

The term "cycloalkyl" or "carbocycle" refers to a saturated or partially unsaturated cyclic alkyl group having a single ring or multiple rings (including fused, bridged, and spiro ring systems). The term "cycloalkyl" includes cycloalkenyl (that is, the cyclic group has at least one double bond). As used herein, cyclic alkyl has 3 to 20 ring carbon atoms (i.e., C₃₋₂₀ cycloalkyl), 3 to 12 ring carbon atoms (i.e., C₃₋₁₂ cycloalkyl), 3 to 10 ring carbon atoms (i.e., C₃₋₁₀ cycloalkyl), 3 to 8 ring carbon atoms (i.e., C₃₋₈ cycloalkyl), or 3 to 7 ring carbon atoms (i.e., C₃₋₇ cycloalkyl), or 3 to 6 ring carbon atoms (i.e., C₃₋₆ cycloalkyl). Examples of cycloalkyl groups include cyclopropyl, cyclobutyl, cyclopentyl, and cyclohexyl, cyclohexenyl, and cyclohexadienyl. The cycloalkyl ring may be fused to an aryl or heteroaryl ring, wherein the ring attached to the parent structure is a cycloalkyl group; non-limiting examples include indanyl, tetrahydronaphthyl, benzocycloheptyl, etc.

The term "heterocyclyl" or "heterocycloalkyl" refers to a saturated or unsaturated cycloalkyl group having one or more ring heteroatoms independently selected from the group consisting of nitrogen, oxygen, sulfur, and phosphorus. The term "heterocycloalkyl" includes heterocycloalkenyl groups (i.e., heterocyclyl groups having at least one double bond), bridged-heterocyclyl groups, fused-heterocyclyl groups, and spiro-heterocyclyl groups. A heterocyclyl group may be a single ring or multiple rings, wherein the multiple rings may be fused, bridged, or spiro. Any non-aromatic ring containing at least one heteroatom is considered a heterocyclyl group, regardless of the attachment (i.e., can be bound through a carbon atom or a heteroatom). Further, the term heterocyclyl is intended to include any non-aromatic ring containing at least one heteroatom, and the ring may be fused to an aryl or heteroaryl ring, regardless of the attachment to the remainder of the molecule. As used herein, heterocyclyl has 3 to 20 ring atoms (i.e., 3- to 20-membered heterocyclyl), 3 to 12 ring atoms (i.e., 3- to 12-membered heterocyclyl), 3 to 10 ring atoms (i.e., 3- to 10-membered heterocyclyl), 3 to 8 ring atoms (i.e., 3- to 8-membered heterocyclyl), 3 to 7 ring atoms (i.e., 3- to 7-membered heterocyclyl), or 3 to 6 ring atoms (i.e., 3- to 6-membered heterocyclyl) and has 1 to 5 ring heteroatoms, 1 to 4 ring heteroatoms, 1 to 3 ring heteroatoms, 1 to 2 ring heteroatoms, or 1 ring heteroatom, and the ring heteroatoms are independently selected from the group consisting of nitrogen, sulfur, phosphorus, and oxygen. Examples of heterocyclyl groups include pyrrolidinyl, imidazolidinyl, oxetanyl, dioxolanyl, azetidinyl, tetrahydrofuranyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, and homopiperazinyl.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Preferably, heteroaryl is 6- to 12-membered. More preferably, heteroaryl is 5- or 6-membered. For example, its non-limiting examples include: imidazolyl, furyl, thienyl, thiazolyl, pyrazolyl, oxazolyl, pyrrolyl, tetrazolyl, pyridyl, pyrimidinyl, thiadiazole, pyrazine, 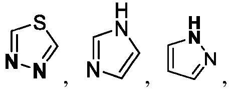 etc.

The heteroaryl ring may be fused to an aryl, heterocyclyl, or cycloalkyl ring, wherein the ring attached to the parent structure is the heteroaryl ring; its non-limiting examples include:

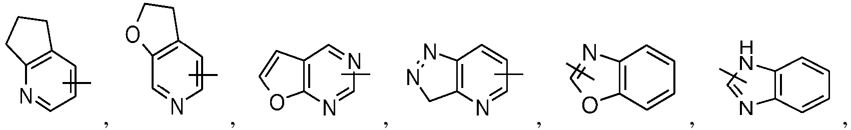

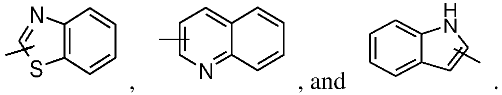

The term "alkoxy" refers to the group "alkyl-O-", wherein the alkyl is as defined above. Examples of alkoxy groups include methoxy, ethoxy, *n*-propoxy, isopropoxy, *n*-butoxy, *tert*-butoxy, *sec*-butoxy, *n*-pentoxy, *n*-hexoxy, and 1,2-dimethylbutoxy.

The term "haloalkyl" refers to an unbranched or branched alkyl group as defined above, wherein one or more hydrogen atoms are replaced by halogen. For example, when a residue is substituted with more than one halogen, it may be referred to by using a prefix corresponding to the number of halogen moieties attached. Dihaloalkyl and trihaloalkyl refer to alkyl groups substituted with two or three halogen groups, respectively, which may be, but are not necessarily, the same halogen. Examples of haloalkyl groups include difluoromethyl (-CHF₂) and trifluoromethyl (-CF₃).

The term "haloalkoxy" refers to an alkoxy group as defined above, wherein one or more hydrogen atoms are replaced by halogen.

The term "hydroxyalkyl" refers to an alkyl group substituted with one or more hydroxy groups, wherein the alkyl is as defined above.

"Monovalent group" refers to a group formed by "formally" eliminating a monovalent atom or group from a compound. "-ylene" refers to a group formed by "formally" eliminating two monovalent atoms or atomic groups or one divalent atom or atomic group from a compound.

The term "alkylene" refers to the remainder of an alkane molecule after 2 hydrogen atoms are removed from the alkane molecule, including linear and branched -ylene groups of 1 to 20 carbon atoms. Non-limiting examples of alkylene groups containing 1 to 6 carbon atoms include methylene (-CH₂-) and ethylene (e.g., -CH₂CH₂- or -CH(CH₃)-). Unless otherwise specified, alkylene may be substituted or unsubstituted. As used in any context herein, alkenyl is optionally substituted in the same manner as alkyl.

The term "heteroalkylene" means that one or more -CH₂- in alkylene are replaced by a heteroatom selected from the group consisting of N, O, and S, wherein the alkylene is as defined above; heteroalkylene may be substituted or unsubstituted. Unless otherwise specified, heteroalkylene may be substituted or unsubstituted. As used in any context herein, heteroalkylene is optionally substituted in the same manner as alkyl.

The term "hydroxy" refers to the -OH group.

The term "halogen" refers to fluorine, chlorine, bromine, or iodine.

The term "cyano" refers to -CN.

The term "nitro" refers to -NO₂.

The term "oxo" refers to the =O substituent.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitutions without undue effort.

### BRIEF DESCRIPTION OF THE DRAWINGS

FIG. 1 shows a comparison of the TOF0.9 values of the antagonists administered after cisatracurium antagonism.
FIG. 2 shows a comparison of the TOF0.9 values of the compounds at 20 mpk, wherein * represents p = 0.05.
FIG. 3 shows the TOF0.9 times of the muscle relaxant antagonists after cisatracurium administration, wherein * p < 0.05, ** p < 0.01, and *** p < 0.001.

### DETAILED DESCRIPTION

The present disclosure is further described below with reference to examples, but these examples are not intended to limit the scope of the present disclosure.

Experimental procedures without conditions specified in the examples of the present disclosure were generally conducted according to conventional conditions or according to conditions recommended by the manufacturers of the raw material or the goods. Reagents without specific origins indicated were commercially available conventional reagents.

The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE-400 nuclear magnetic resonance instrument, with dimethyl sulfoxide-D6 (DMSO-d₆), chloroform-D (CDCl₃), and methanol-D4 (CD₃OD) as solvents and tetramethylsilane (TMS) as an internal standard.

The MS analyses were performed using a Shimadzu 2010 Mass Spectrometer or Agilent 6110A MSD Mass Spectrometer.

The HPLC analyses were performed using Shimadzu LC-20A systems, Shimadzu LC-2010HT series, or an Agilent 1200 LC high-performance liquid chromatograph (Ultimate XB-C18 3.0 × 150 mm column or Xtimate C18 2.1 × 30 mm column).

The chiral HPLC analyses were performed using Chiralpak IC-3 100 × 4.6 mm I.D., 3 µm, Chiralpak AD-3 150 × 4.6 mm I.D., 3 µm, Chiralpak AD-3 50 × 4.6 mm I.D., 3 µm, Chiralpak AS-3 150 × 4.6 mm I.D., 3 µm, Chiralpak AS-3 100 × 4.6 mm I.D., 3 µm, ChiralCel OD-3 150 × 4.6 mm I.D., 3 µm, Chiralcel OD-3 100 × 4.6 mm I.D., 3 µm, ChiralCel OJ-H 150 × 4.6 mm I.D., 5 µm, and Chiralcel OJ-3 150 × 4.6 mm I.D., 3 µm columns.

The thin-layer chromatography silica gel plates used were Yantai Huanghai HSGF254 or Qingdao GF254 silica gel plates. The silica gel plates used in the thin-layer chromatography (TLC) had a layer thickness of 0.15 mm-0.2 mm, and those used in the thin-layer chromatography separation and purification had a layer thickness of 0.4 mm-0.5 mm.

The column chromatography generally used 100-200 mesh, 200-300 mesh, or 300-400 mesh Yantai Huanghai silica gel as the carrier.

The preparative chiral columns used were DAICEL CHIRALPAK IC (250 mm × 30 mm, 10 µm) or Phenomenex-Amylose-1 (150 mm × 30 mm, 5 µm) columns.

The CombiFlash preparative flash chromatograph used was Combiflash Rf150 (TELEDYNE ISCO).

The known starting materials in the present disclosure may be synthesized by using or following methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of the reaction progress in the examples used thin-layer chromatography (TLC). The developing solvent used in the reactions, the eluent systems used in the column chromatography purification, the developing solvent systems used in the thin-layer chromatography, and the volume ratio of the solvents were adjusted depending on the polarity of the compound, or a small amount of basic or acidic reagents such as triethylamine and acetic acid could be added for adjustment.

### Example 1. Preparation of Compound 2

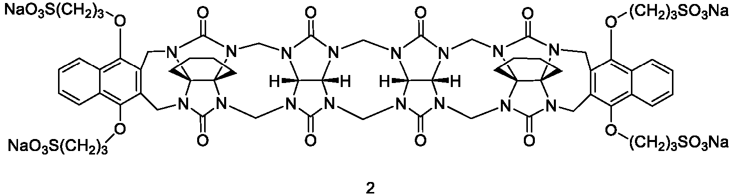

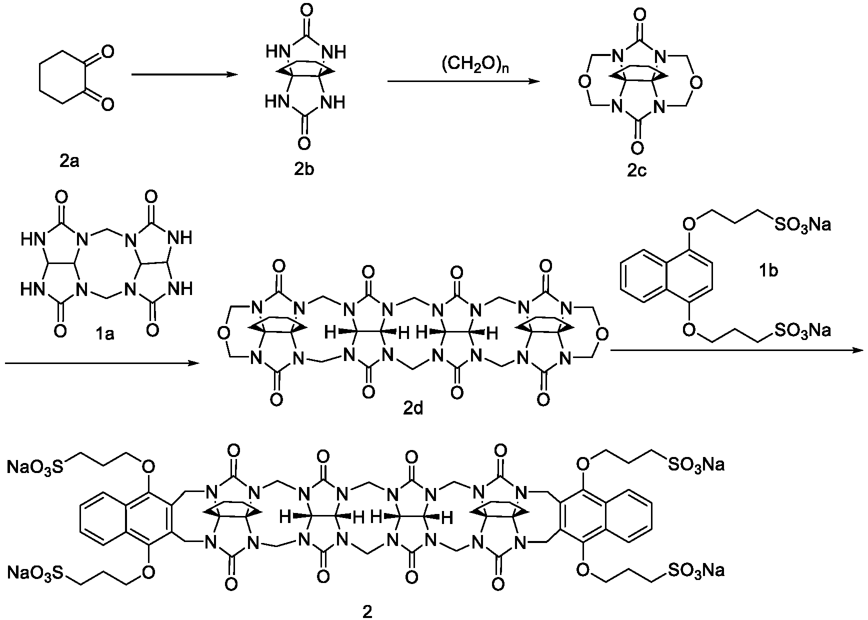

Step 1: Urea (38.89 g, 647.46 mmol), 0.3 M dilute hydrochloric acid (80 mL), and 1,2-cyclohexanedione 2a (22.0 g, 196.20 mmol) were added to a 500-mL three-necked flask, heated to 50 °C, and stirred for 16 h. The reaction mixture was cooled to room temperature and filtered, and the filter cake was rinsed with 100 mL of water, rinsed with 100 mL of absolute ethanol, and dried to give compound 2b (a pale yellow solid, 27.4 g, yield: 71%).

MS m/z (ESI): 197.1[M+1]⁺.

¹H NMR (400 MHz, DMSO-d6): δ 7.02 (s, 4H), 1.72-1.68 (m, 4H), 1.42-1.35 (m, 4H).

Step 2: Compound 2b (27.4 g, 139.65 mmol), 140 mL of 9 M hydrochloric acid, and paraformaldehyde (20.9 g, 698.23 mmol) were added to a 1-L three-necked flask, and the reaction mixture was stirred at room temperature for 24 h. Water (500 mL) was added to the reaction system, and the mixture was stirred at room temperature for another 16 h. The reaction mixture was filtered, washed, and dried to give compound 2c (a white solid, 20.2 g, yield: 52%).

MS (ESI): 181.1[M+1]⁺.

¹H NMR (400 MHz, DMSO-d6): δ 5.20 (d, 4 H, J = 11.6), 4.91 (d, 4 H, J = 11.2), 2.25-2.18 (m, 4H), 1.56-1.50 (m, 4H).

Step 3: Compound 2c (2.73 g, 9.73 mmol) was weighed into a dry three-necked flask, and the system was purged with argon. Methanesulfonic acid (10 mL) was added to dissolve the compound, and after compound 1a (1 g, 3.24 mmol, prepared using the well-known method "WO2012/051407A") was added, the mixture was stirred at room temperature for 24 h. The reaction mixture was slowly added to 100 mL of water (cooled in an ice-water bath), and after the addition, the mixture was warmed to room temperature, filtered, and dried to give a crude product (1.77 g). The crude product was dissolved by heat in TFA (4 mL), and 16 mL of water was then added. The mixture was stirred, filtered, and dried *in vacuo* to give compound 2d (1.21 g, yield: 44.9%).

MS (ESI): 837.3[M+1]⁺.

¹H NMR (400 MHz, CDCl₃): δ 5.72-5.37 (m, 10 H), 5.15 (d, 4 H), 4.75 (d, 4 H), 4.15-4.11 (m, 6 H), 2.28 (br, 4 H), 2.05 (br, 4 H), 1.45 (br, 8 H).

Step 4: Compound 2d (1.06 g, 1.27 mmol) was weighed into a dry three-necked flask, and the system was purged with argon. TFA (10 mL) was added to dissolve the compound, and compound 1b (1.42 g, 3.17 mmol, prepared using the well-known method "WO2012/051407A") was then added. After the addition, the reaction mixture was heated to 60 °C and stirred for 3 h. TFA was removed by evaporation under reduced pressure, and 20 mL of ethanol was added to the resulting solid. The mixture was heated at reflux for 2 h, cooled to room temperature, and filtered. The filter cake was washed with ethanol and dried. The resulting solid was dissolved by heat in 10 mL of water, and 30 mL of ethanol was then added. The mixture was filtered, and the filter cake was then purified by high performance liquid chromatography (column: SharpSil-T, 30 × 150 mm, 5 µm; mobile phase: aqueous phase and acetonitrile; gradient ratio: aqueous phase 25%-42%) and finally salified with sodium hydroxide to give compound 2 (0.27 g, yield: 12.6%).

MS m/z (ESI): 1605.2[M-4Na+5H]⁺.

¹H NMR (400 MHz, D₂O): δ 7.68-7.65 (m, 4 H), 7.07-7.05 (m, 4 H), 5.50-5.45 (m, 6 H), 5.26-5.16 (m, 8 H), 4.39 (d, 4 H), 4.17-4.05 (m, 8H), 3.94-3.82 (m, 6H), 3.20-3.04 (m, 8 H), 2.26-2.07 (m, 16 H), 1.46 (br s, 8 H).

### Example 2. Preparation of Compound 3

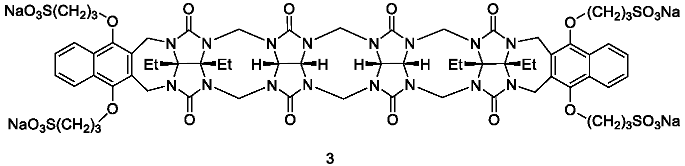

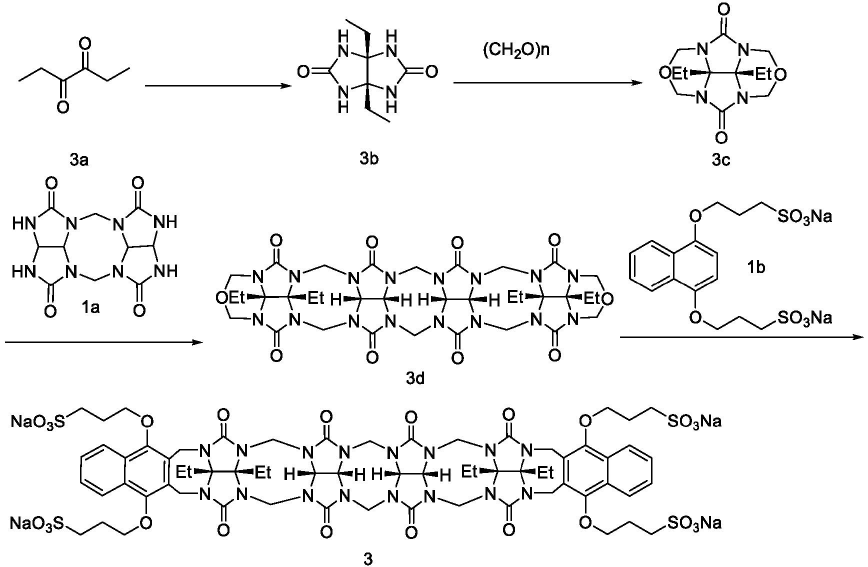

Step 1: Urea (12 g, 0.2 mol) was dissolved in 0.3 M HCl (30 mL), and compound 3a (6.97 g, 0.061 mol) was added at room temperature. Subsequently, the mixture was stirred at room temperature for 24 h, filtered, washed, and dried to give the title product 3b (6.8 g, yield: 56.2%).

Step 2: Substrate 3b (3.4 g, 17.2 mmol) was weighed into a dry three-necked flask, and 2.54 g of paraformaldehyde and 9 M HCl (15 mL) were added. The mixture was stirred at room temperature for 24 h. The reaction mixture was stirred at room temperature for another 24 h, filtered, washed, and dried to give the title product 3c (2.1 g, yield: 43.2%).

MS m/z (ESI): 283.1[M+1]+.

¹H NMR (400 MHz, DMSO-d6): δ 5.21 (d, 4 H), 4.93 (d, 4 H), 2.33 (q, 4 H), 0.91 (d, 6 H).

Step 3: Compound 3c (2.75 g, 9.73 mmol) was dissolved in methanesulfonic acid (10 mL), and after compound 1a (1 g, 3.24 mmol) was added, the mixture was stirred at room temperature for 18 h. The reaction mixture was slowly added to 100 mL of water (cooled in an ice-water bath), and after the addition, the mixture was warmed to room temperature, filtered, and dried to give a crude product (1.67 g). The crude product was dissolved by heat in TFA (4 mL), and 16 mL of water was then added. The mixture was stirred, filtered, and dried *in vacuo* to give the title product 3d (an off-white solid, 1.56 g, yield: 57.3%).

MS m/z (ESI): 833.2[M+1]+.

¹H NMR (400 MHz, DMSO-d6): δ 5.68 (d, 2 H), 5.53 (t, 6 H), 5.38 (d, 2 H), 5.15 (d, 4 H), 4.77 (d, 4 H), 4.20-4.15 (m, 6 H), 2.35-2.32 (m, 4 H), 2.20-2.15 (m, 4 H), 0.88-0.80 (m, 12 H).

Step 4: 3d (0.89 g, 1.06 mmol) was weighed into a dry three-necked flask, and the system was purged with argon. TFA (10 mL) was added to dissolve the compound, and compound 1b (1.19 g, 2.66 mmol) was then added. After the addition, the reaction mixture was heated to 70 °C and stirred for 3 h. TFA was removed by evaporation under reduced pressure, and 20 mL of ethanol was added to the resulting solid. The mixture was heated at reflux for 2 h, cooled to room temperature, filtered, and dried. The resulting solid was dissolved by heat in 6.6 mL of water, and 19.8 mL of ethanol was then added. The mixture was filtered, and the filter cake was washed, then purified by high performance liquid chromatography (column: SharpSil-T, 30 × 150 mm, 5 µm; mobile phase: aqueous phase and acetonitrile; gradient ratio: aqueous phase 25%-42%), and finally salified with sodium hydroxide to give the title product 3 (0.28 g, yield: 15.5%).

MS (ESI): 1607.4[M-4Na+3H]⁻.

¹H NMR (400 MHz, D₂O): δ 7.41-7.39 (m, 4 H), 6.81-6.80 (m, 4 H), 5.46 (d, 4 H), 5.38 (d, 2 H), 5.30 (d, 4 H), 5.20 (d, 2 H), 5.08 (d, 2 H), 4.38 (d, 4 H), 4.09-4.01 (m, 8 H), 3.84-3.76 (m, 6 H), 3.19-3.02 (m, 8 H), 2.36-2.18 (m, 16 H), 0.88 (t, 6 H), 0.82 (t, 6 H).

### Example 3. Preparation of Compound 1

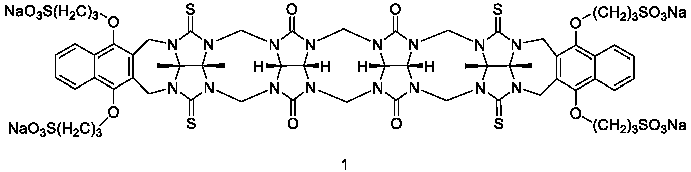

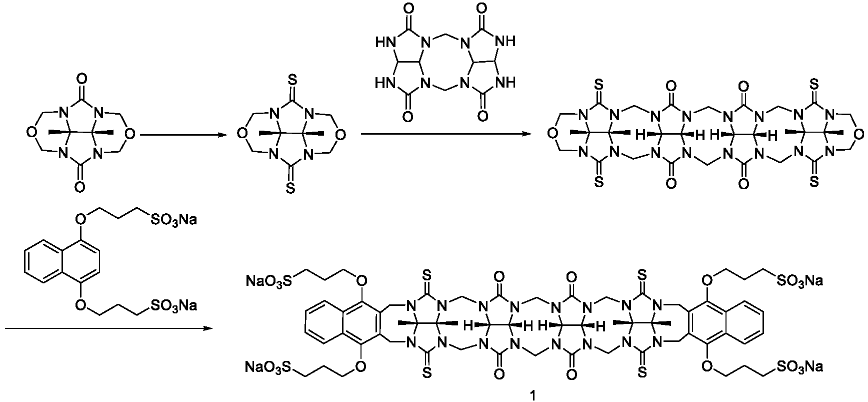

### Example 4. Preparation of Compound 4

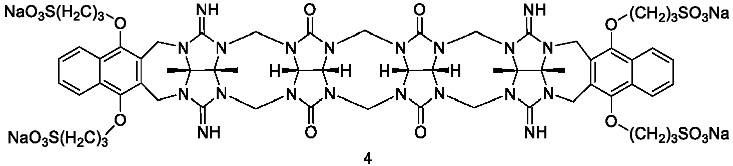

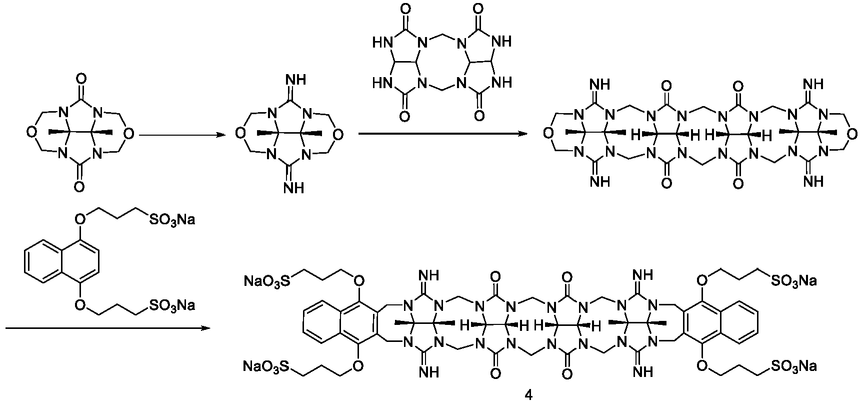

### Example 5. Preparation of Compound 5

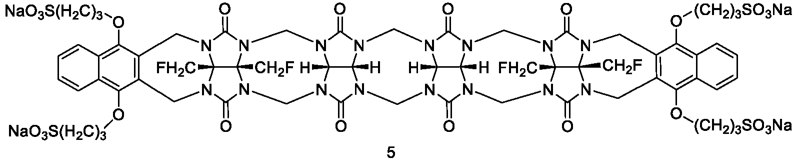

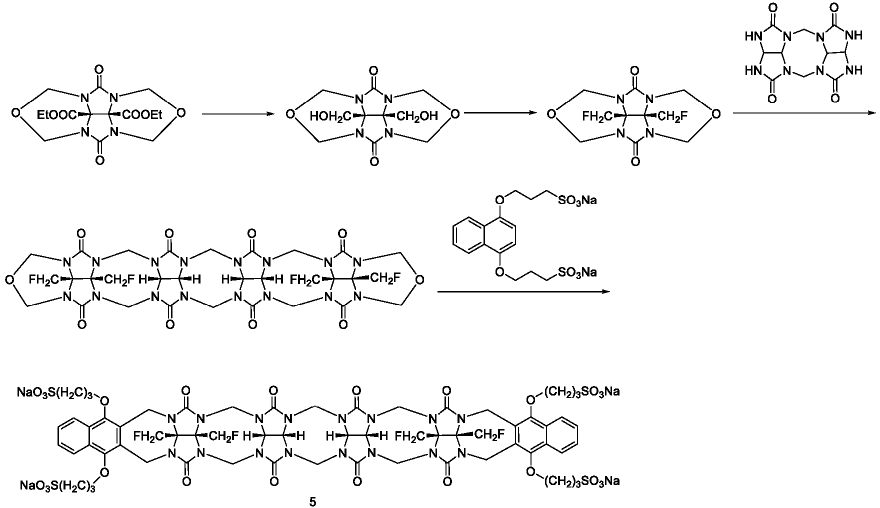

### Example 6. Preparation of Compound 6

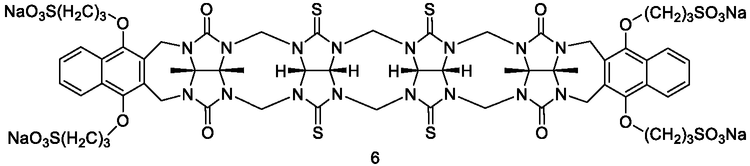

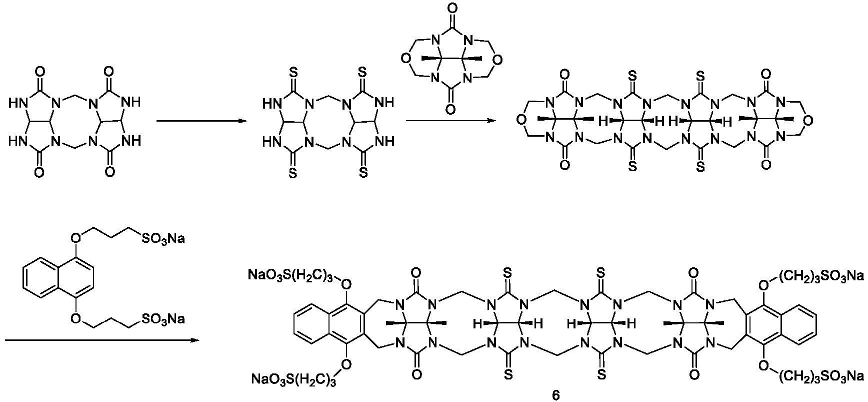

### Example 7. Preparation of Compound 7

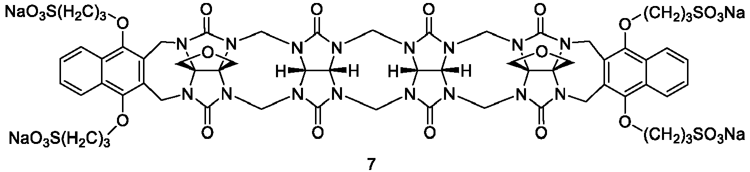

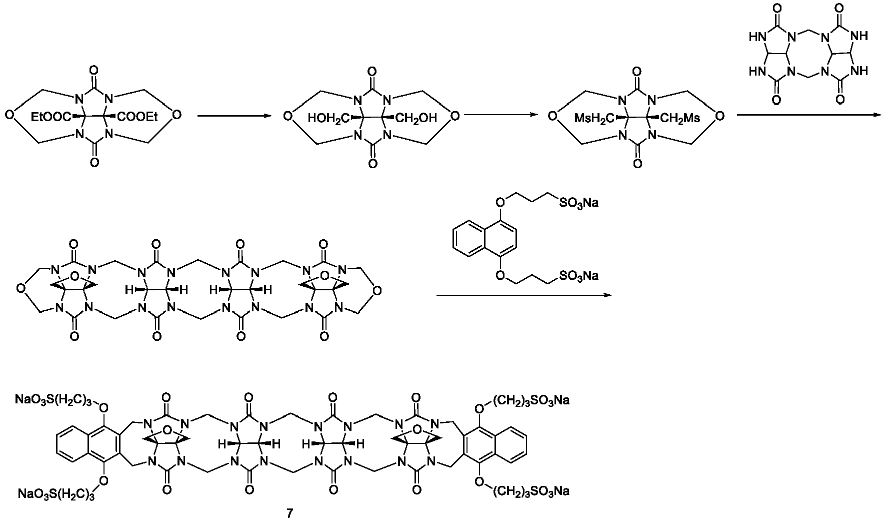

### Example 8. Preparation of Compound 8

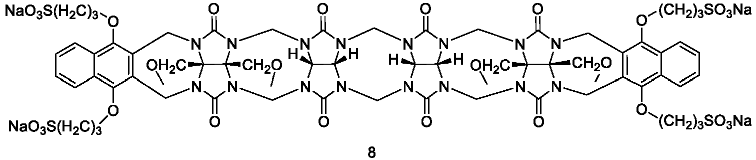

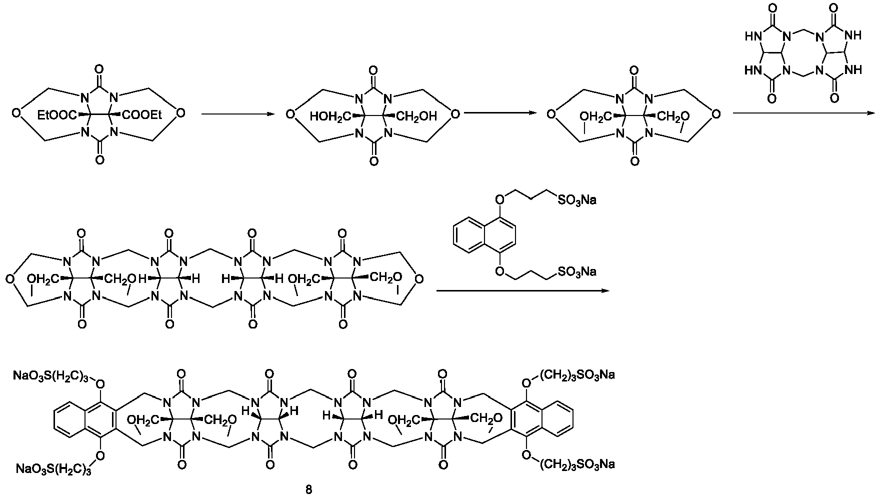

### Example 9. Preparation of Compound 9

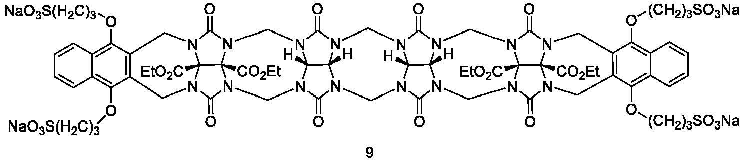

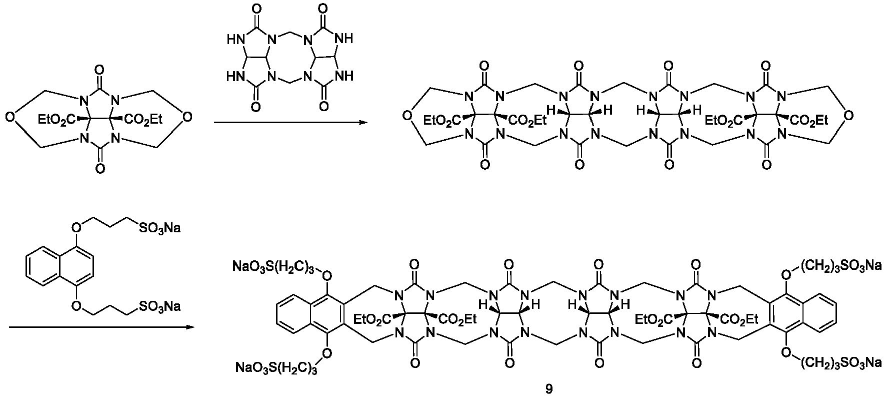

### Example 10. Preparation of Compound 10

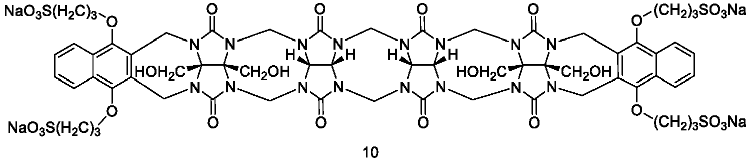

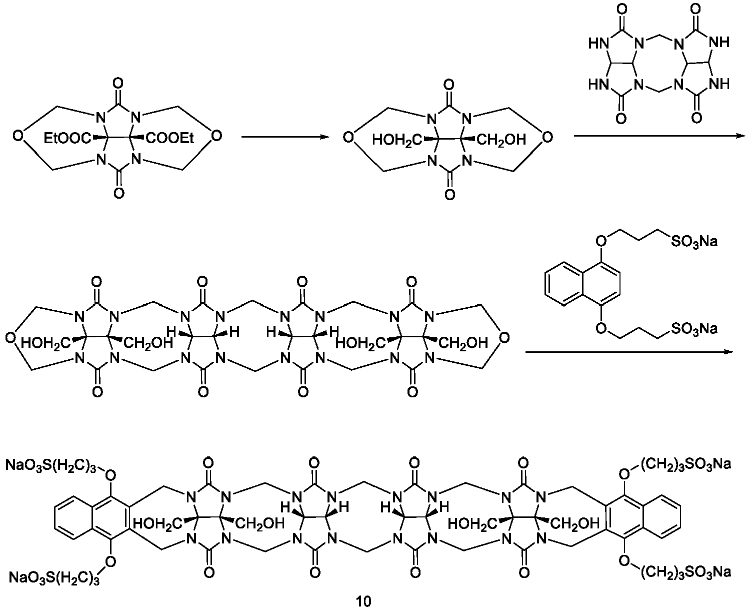

### Example 11. Preparation of Compound 11

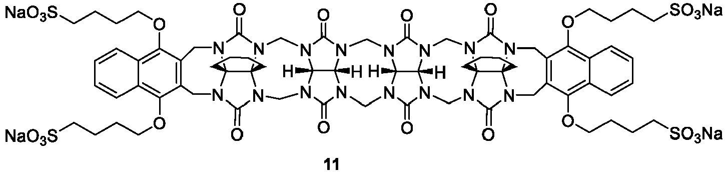

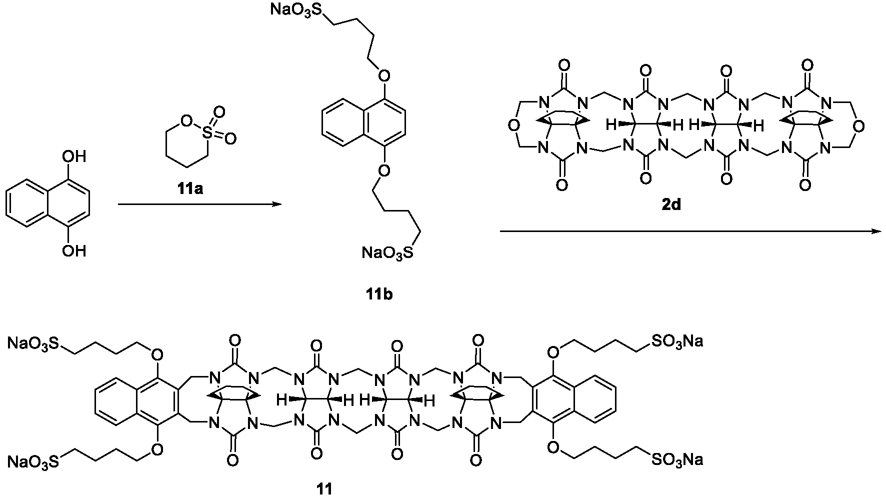

**Step 1:** 1,4-Dihydroxynaphthalene (1 g, 6.24 mmol) was dissolved in a 10% aqueous NaOH solution (8 mL), and after nitrogen purging, a solution of **compound 11a** (2.14 g, 15.6 mmol) in dioxane (12 mL) was added dropwise. The reaction mixture was stirred overnight at room temperature. The reaction mixture was dried by rotary evaporation and purified by preparative reversed-phase chromatography to give **compound 11b** as a white solid (0.8 g, yield: 26%).

MS m/z (ESI): 433.1[M-2Na +3H]⁺.

¹H NMR (400 MHz, DMSO-d6): δ 8.12-8.10 (m, 2 H), 7.53-7.51 (m, 2 H), 6.84 (s, 2 H), 4.07 (t, 4 H), 2.55-2.49 (m, 4 H), 1.91-1.79 (m, 8 H).

**Step 2: Compound 11b** (520 mg, 1.09 mmol) was dissolved in TFA (10 mL), and **compound 2d** (364 mg, 0.44 mmol) was then added. After the addition, the reaction system was heated to 70 °C and stirred for 2 h. TFA was removed by evaporation under reduced pressure, and the resulting solid was purified by column chromatography (column: SharpSil-T, 30 × 150 mm, 5 µm; mobile phase: aqueous phase and acetonitrile; gradient ratio: aqueous phase 25%-42%) to give a white solid (192 mg). The solid was dissolved in 2 mL of water, and the pH of the system was then adjusted to 5-8 with a 0.5 M aqueous sodium hydroxide solution. Ethanol (10 mL) was added, and a solid precipitated. The mixture was filtered to give **compound 11** as a white solid (120 mg, yield: 16.5%).

MS m/z (ESI): 1678.3[(M-4Na+4H+18HH)]⁺.

¹H NMR (400 MHz, D₂O): δ 7.87-7.81 (m, 4 H), 7.44-7.36 (m, 4 H), 5.53-5.46 (m, 7 H), 5.31-5.23 (m, 9 H), 4.30 (d, 3 H), 4.12-3.94 (m, 9 H), 3.78-3.76 (m, 4 H), 2.87-2.82 (m, 8 H), 2.05-1.80 (m, 24 H), 1.38-1.25 (m, 8 H).

### Example 12. Preparation of Compound 12

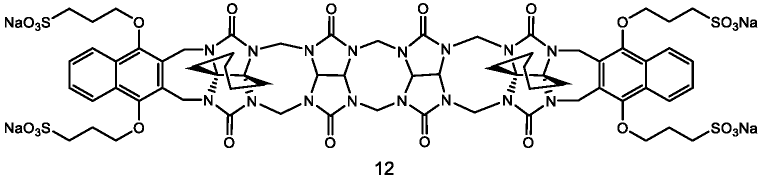

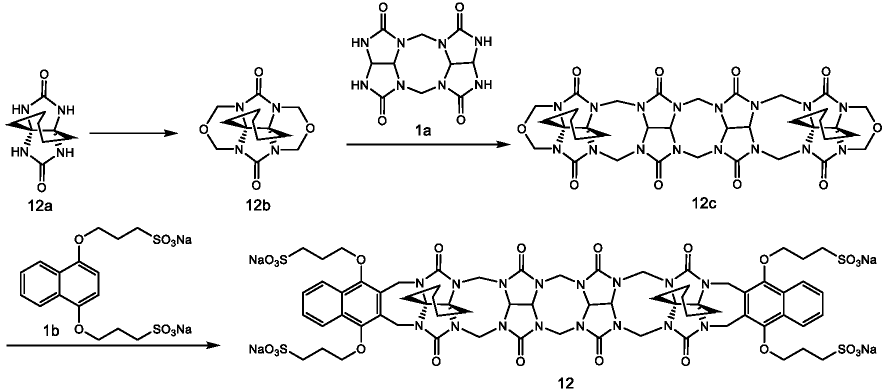

**Step 1: Compound 12a** (10.85 g, 51.6 mmol, prepared using the same preparation method as **compound 2b** in Example 1) was weighed into a dry three-necked flask, and 7.75 g of paraformaldehyde and 9 M HCl (45 mL) were added. The mixture was stirred at room temperature for 24 h. An additional 163 mL of water was added, and the reaction mixture was stirred at room temperature for another 24 h and filtered. The filter cake was washed with water (60 mL) and ethanol (60 mL) and dried to give **compound 12b** as an off-white solid (10.84 g, yield: 71.4%).

MS m/z (ESI): 295.1[M+1]⁺.

¹H NMR (400 MHz, DMSO-d6): δ 5.21 (d, 4 H, *J* = 11.3 Hz), 4.93 (d, 4 H, *J* = 11.3 Hz), 2.34 (br, 4 H), 1.48 (br, 6 H).

**Step 2: Compound 12b** (2.00 g, 6.80 mmol) was weighed into a dry three-necked flask, and the system was purged with argon. Methanesulfonic acid (7 mL) was added to dissolve the compound, and **compound 1a** (0.70 g, 2.27 mmol, prepared using the well-known method "WO2012/051407 A2") was added at room temperature (23 °C). After 18 h of stirring at room temperature, the reaction mixture was slowly added to 70 mL of water (cooled in an ice-water bath). After the addition, the mixture was warmed to room temperature and filtered, and the filter cake was washed with a small amount of water and dried to give a crude product (1.97 g). The crude product was dissolved in TFA (6 mL, 23 °C), and 24 mL of water was then added. The mixture was stirred at 23 °C for 30 min and filtered, and the filter cake was washed with a small amount of water and dried *in vacuo* to give **compound 12c** as an off-white solid (1.75 g, yield: 81.7%).

MS m/z (ESI): 861.3[M+1]⁺.

¹H NMR (400 MHz, DMSO-d6): δ 5.50-5.41 (m, 10 H), 5.16 (d, 4 H, *J =* 10.7 Hz), 4.76 (d, 4 H, *J* = 10.6 Hz), 4.22-4.15 (m, 6 H), 2.40 (br, 4 H), 2.22 (br, 4 H), 1.45-1.26 (m, 12 H).

**Step 3: Compound 12c** (1.00 g, 1.16 mmol) was weighed into a dry three-necked flask, and the system was purged with argon. TFA (10 mL) was added to dissolve the compound, and **compound 1b** was then added. After the addition, the reaction mixture was heated to 60 °C and stirred for 3 h. TFA was removed by evaporation under reduced pressure, and 20 mL of ethanol was added to the resulting solid. The mixture was heated at reflux for 2 h, cooled to room temperature, and filtered. The filter cake was washed with ethanol and dried. The resulting solid was purified by high performance liquid chromatography (mobile phase: aqueous phase and acetonitrile; gradient ratio: aqueous phase 25%-42%) and finally salified with sodium hydroxide to give **compound 12** as a white solid (0.12 g).

MS m/z (ESI): 1649.1[M-4Na+4H+NH4]⁺.

¹H NMR (400 MHz, D₂O): *δ* 8.04 (br, 4 H), 7.64 (br, 4 H), 5.68-5.59 (m, 7 H), 5.42-5.39 (m, 4 H), 5.30-5.26 (m, 4 H), 4.44-4.39 (m, 3 H), 4.22-4.07 (m, 10 H), 3.93-3.91 (m, 4 H), 3.28-3.13 (m, 8 H), 2.44-2.22 (m, 16 H), 1.61-1.34 (m, 12 H).

### Example 13. Preparation of Compound 13

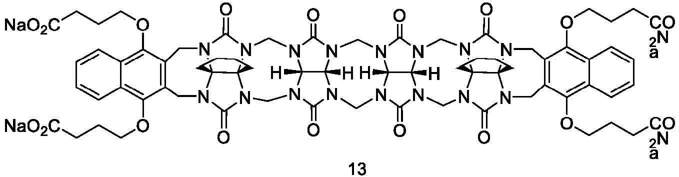

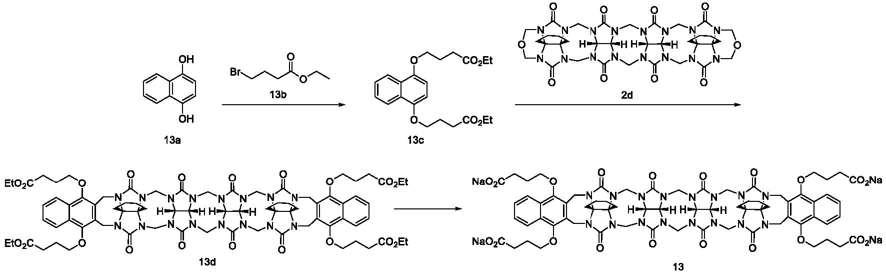

**Step 1:** DMSO (50 mL) was added to a three-necked flask, and KOH (8.75 g, 156 mmol) was added. The mixture was ultra-sonicated to disperse KOH in DMSO, then purged of air with nitrogen, and then stirred at room temperature for one hour. **Compound 13a** (5 g, 31.25 mmol) and **compound 13b** (24.3 g, 125 mmol) were separately and slowly added to the reaction mixture. The reaction mixture was heated to 60 °C and stirred for 2 h. After the reaction mixture was cooled to room temperature, 200 mL of water was added, and extraction was performed with dichloromethane (50 mL × 3). The combined organic phases were washed with saturated brine (100 mL), dried, and filtered, and the solvent was removed by rotary evaporation. The crude product was purified by column chromatography (PE:EA = 5:1) to give **compound 13c** as a red solid (4.56 g, yield: 38%).

MS m/z (ESI): 389.2[M+1]⁺.

¹H NMR (400 MHz, CDCl₃): δ 8.20 (q, 2 H), 7.49 (q, 2 H), 6.67 (s, 2 H), 4.18-4.12 (m, 8 H), 2.61 (t, 4 H), 2.27-2.20 (m, 4 H), 1.25 (t, 6 H).

**Step 2: Compound 13c** (3.50 g, 9.02 mmol) was weighed into a dry three-necked flask, and the system was purged with nitrogen. TFA (20 mL) was added to dissolve the compound, and **compound 2d** (3.00 g, 3.61 mmol) and acetic anhydride (918 mg, 9.02 mmol) were then added. After the addition, the reaction mixture was heated to 70 °C and stirred for 4 h. TFA was removed by evaporation under reduced pressure, and 50 mL of methyl tert-butyl ether was added to the resulting solid. The mixture was heated to 50 °C, stirred for half an hour, cooled to room temperature, and filtered. The filter cake was washed with methyl tert-butyl ether and dried to give **compound 13d** as a brown solid (4.2 g).

MS m/z (ESI): 1590.3[M+18]⁺.

**Step 3: Compound 13d** (2.0 g, 1.27 mmol) was added to a 50-mL single-necked flask, and methanol and water (1:1, 30 mL) and LiOH·H₂0 (534 mg, 12.7 mmol) were added to dissolve the compound. After the addition, the reaction mixture was heated to 80 °C and stirred for 3 h. The solvent was removed by evaporation under reduced pressure, and ethanol and water (10:1, 20 mL) were added. The mixture was stirred at room temperature for half an hour and filtered. The filter cake was washed with ethanol and dried. The resulting solid was dissolved in water and NaOH (1 M), then purified by high performance liquid chromatography (mobile phase: aqueous phase and acetonitrile; gradient ratio: aqueous phase 25%-42%), and finally salified with sodium hydroxide to give **compound 14** as a white solid (114 mg, yield: 6.15%).

MS m/z (ESI): 1478.2[M-4Na+4H+NH₄]⁺.

¹H NMR (400 MHz, D₂O): δ 7.87 (br, 4 H), 7.46 (br, 4 H), 5.55-5.44 (m, 6 H), 5.29-5.13 (m, 8 H), 4.20-3.92 (m, 14 H), 3.69 (br, 4 H), 2.19-1.90 (m, 24 H), 1.34 (br, 8 H).

### Example 14. Preparation of Compound 14

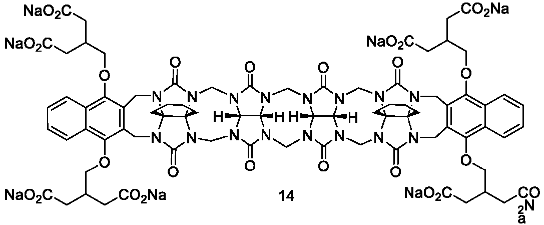

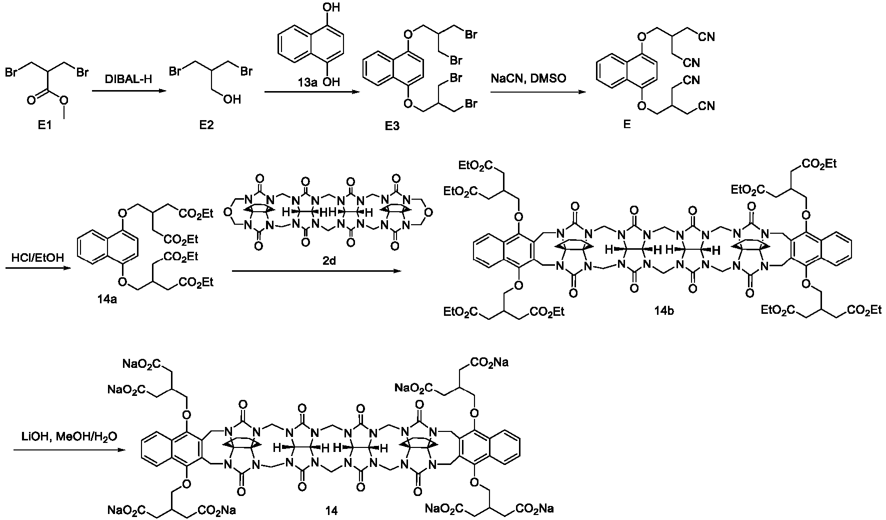

**Step 1: Compound E1** (25 g, 96 mmol) and anhydrous THF (70 mL) were added to a three-necked flask, and the system was then purged of air with nitrogen and cooled to -78 °C using a dry ice-acetone bath. Then DIBAL-H (135 mL, 202 mmol, 1.5 M in toluene) was slowly added dropwise. After the dropwise addition, the reaction mixture was warmed to 0 °C and stirred at 0 °C for 45 min. Then HCl (1 M, 500 mL) was added dropwise at 0 °C to quench the reaction. Ethyl acetate (200 mL) was added, and extraction was performed with ethyl acetate (50 mL × 3). The combined organic phases were washed with saturated brine (100 mL), dried, and filtered, and the solvent was removed by rotary evaporation. The crude product was purified by column chromatography (PE:EA = 5:1) to give **compound E2** as a pale yellow oil (15.8 g, yield: 71.5%).

¹H NMR (400 MHz, CDCl₃): δ 3.77-3.75 (m, 2 H), 3.62-3.53 (m, 4 H), 2.46 (br, 1 H), 2.29-2.22 (m, 1 H).

**Step 2: Compound E2** (7 g, 30.4 mmol), **compound 13a** (1.62 g, 10.1 mmol), and MeSO₃H (1.4 mL) were weighed into a dry three-necked flask, and the system was purged with nitrogen. The reaction mixture was heated to 100 °C and stirred for 3 h. The reaction mixture was cooled to room temperature, then poured into ice water, and extracted with ethyl acetate (50 mL × 3). The combined organic phases were washed with a NaHCO₃ solution and saturated brine in sequence, dried, and filtered, and the solvent was removed by rotary evaporation. The crude product was purified by column chromatography (PE:EA = 50:1) to give **compound E3** as a pale yellow solid (2.74 g, yield: 46%).

¹H NMR (400 MHz, CDCl₃): δ 8.16 (q, 2H), 7.54 (q, 2H), 6.74 (s, 2H), 4.23 (d, 4H), 3.81-3.71 (m, 8H), 2.73-2.70 (m, 2H).

**Step 3: Compound E3** (5.0 g, 8.56 mmol) was weighed into a dry single-necked flask, and the system was purged with nitrogen. DMSO (60 mL) was added to dissolve the compound, and NaCN (4.2 g, 85.6 mmol) was then added. After the addition, the reaction mixture was heated to 75 °C and stirred for 1 h. The reaction mixture was poured into ice water, and a large amount of solid precipitated. The mixture was filtered. Ethyl acetate was added to the resulting solid, and the mixture was stirred at room temperature for half an hour, filtered, and dried to give **compound E** as a white solid (2.33 g, yield: 73.5%).

MS m/z (ESI): 390[M+18]⁺.

**Step 4: Compound E** (2.1 g, 5.64 mmol) was weighed into a sealed tube. HCl/EtOH (75 mL, 10 M) was added, and the compound did not dissolve completely. The reaction mixture was heated to 90 °C and stirred for 24 h. The solvent was removed by rotary evaporation. The crude product was purified by column chromatography (PE:EA = 5:1) to give **compound 14a** as a colorless oil (1.08 g, yield: 34.2%).

MS m/z (ESI): 561[M+1]⁺.

¹H NMR (400 MHz, CDCl₃): δ 8.16 (q, 2H), 7.50 (q, 2H), 6.67 (s, 2H), 4.16-4.11 (m, 12H), 3.00-2.95 (m, 2H), 2.72-2.54 (m, 8H), 1.23 (t, 12H).

**Step 5: Compound 14a** (1.4 g, 2.50 mmol) was weighed into a dry three-necked flask, and the system was purged with nitrogen. TFA (5 mL) was added to dissolve the compound, and **compound 2d** (520 mg, 0.625 mmol) and acetic anhydride (5 mL) were then added. After the addition, the reaction mixture was heated to 90 °C and stirred for 4 h. TFA was removed by evaporation under reduced pressure, and 50 mL of methyl tert-butyl ether was added to the resulting solid. The mixture was heated to 50 °C, stirred for half an hour, cooled to room temperature, and filtered. The filter cake was washed with methyl tert-butyl ether and dried to give a brown solid (1.1 g). The resulting solid was dissolved in acetonitrile and then purified by high performance liquid chromatography (mobile phase: aqueous phase and acetonitrile; gradient ratio: aqueous phase 25%-42%) to give **compound 14b** as a white solid (170 mg, yield: 3.55%).

MS m/z (ESI): 1934[M+18]⁺.

**Step 6: Compound 14b** (150 mg, 0.078 mmol) was weighed into a 25-mL single-necked flask. Methanol and water (1:1, 10 mL) were added, and the compound did not dissolve. Then LiOH·H₂O (534 mg, 12.7 mmol) was added. After the addition, the reaction mixture was heated to 80 °C and stirred for 3 h. The reaction mixture was purified by high performance liquid chromatography (mobile phase: aqueous phase and acetonitrile; gradient ratio: aqueous phase 25%-42%) and finally salified with sodium hydroxide to give **compound 14** as a white solid (53 mg, yield: 40%).

MS m/z (ESI): 1710[M-8Na+8H+NH₄]⁺.

¹H NMR (400 MHz, D₂O): δ 7.80-7.97 (m, 4 H), 7.39-7.38 (m, 4 H), 5.50-5.46 (m, 6 H), 5.34 (s, 4 H), 4.96 (d, 4 H), 4.50 (d, 4 H), 4.11-3.95 (m, 10 H), 3.69-3.67 (m, 4 H), 2.69-2.47 (m, 12H), 2.37-2.32 (m, 8H), 2.18 (s, 4H), 1.94 (s, 4H), 1.44 (s, 8H).

### Example 15. Preparation of Compound 15

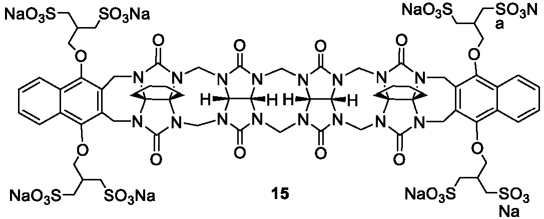

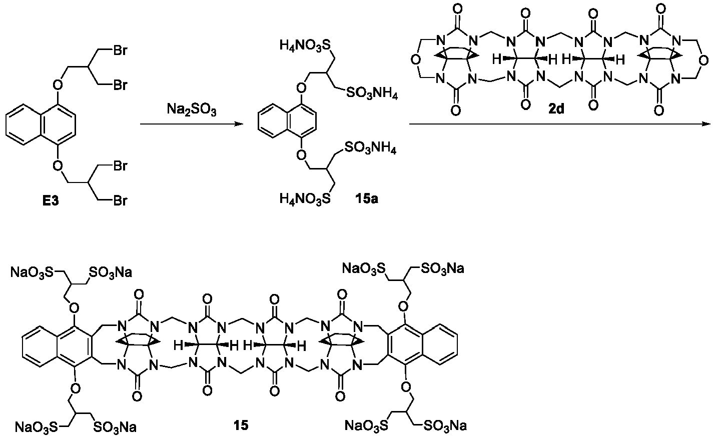

**Step 1: Compound E3** (2.25 g, 3.83 mmol) and sodium sulfite (4.91 g, 38.9 mmol) were added to a three-necked flask, and the system was purged of air with nitrogen. Then 42 mL of isopropyl alcohol and 42 mL of water were added. The reaction mixture was heated to 100 °C and stirred for 24 h. The reaction mixture was cooled to room temperature and concentrated to give a crude product. The crude product was triturated with 83 mL of methanol for 1 h, and filtration was performed. The resulting solid was collected and purified by preparative HPLC to give **compound 15a** as a white solid (1.62 g, yield: 64%).

MS m/z (ESI): 331.8[M/2+1]⁺.

¹H NMR (400 MHz, D₂O): δ 8.23-8.22 (m, 2 H), 8.57-7.55 (m, 2 H), 6.90-6.88 (m, 2 H), 4.40-4.39 (m, 4 H), 3.33-3.31 (m, 8 H), 2.94-2.92 (m, 2 H).

**Step 2: Compound 15a** (1.57 g, 2.37 mmol) was weighed into a dry three-necked flask, and the system was purged with argon. TFA (15 mL) was added to dissolve the compound, and compound 2d (0.79 g, 0.95 mmol) was then added. After the addition, the reaction mixture was heated to 70 °C and stirred for 3 h. TFA was removed by evaporation under reduced pressure, and 40 mL of ethanol was added to the resulting solid. The mixture was heated at reflux for 2 h, cooled to room temperature, and filtered. The filter cake was washed with ethanol and dried. The resulting solid was dissolved in 12 mL of water, and the pH of the system was adjusted to about 7 with a 1 M aqueous sodium hydroxide solution. Ethanol (50 mL) was added, and a viscous substance precipitated. The supernatant was removed, and the residue was dried by rotary evaporation, then purified by high performance liquid chromatography (mobile phase: aqueous phase and acetonitrile; gradient ratio: aqueous phase 25%-42%), and finally salified with sodium hydroxide to give **compound 15** as a white solid (299 mg, yield: 12%).

MS m/z (ESI): 990.6[(M-8Na+8HHH)/2]⁺.

¹H NMR (400 MHz, D₂O): δ 7.96-7.93 (m, 4 H), 7.32-7.33 (m, 4 H), 5.57-5.44 (m, 6 H), 5.35-5.31 (m, 4 H), 5.09-5.05 (m, 4 H), 4.54-4.50 (m, 4 H), 4.27-4.23 (m, 4 H), 4.10-4.01 (m, 10 H), 3.52-3.31 (m, 16 H), 2.98-2.92 (m, 4 H), 2.19-2.17 (m, 4 H), 2.01-1.98 (m, 4 H), 1.47-1.45 (m, 8 H).

### Example 16. Preparation of Compound 16

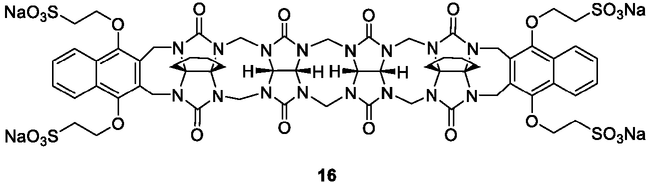

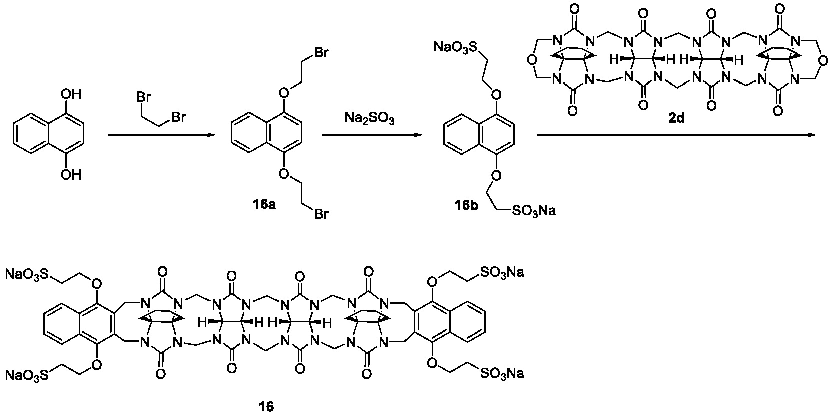

**Step 1:** 1,4-Dihydroxynaphthalene (2.00 g, 12.49 mmol), 1,2-dibromoethane (23.46 g, 124.87 mmol, 10.76 mL), and 18-crown-6 (165.02 mg, 624.34 µmol) were dissolved in acetonitrile (40 mL). The reaction mixture was purged with nitrogen 3 times, heated to 70 °C, and left to react for 2 days. The reaction mixture was cooled to room temperature and then filtered, and the filtrate was concentrated to give a crude product. The crude product was purified by column chromatography (PE:PA = 50:1) to give **compound 16a** (1.6 g, yield: 34.25%).

¹H NMR (400 MHz, CDCl₃): δ 8.27-8.25 (m, 2 H), 7.55-7.52 (m, 2 H), 6.69 (s, 2 H), 4.42 (t, 4 H), 3.76 (t, 4 H).

**Step 2: Compound 16a** (800 mg, 2.14 mmol) and sodium sulfite (593 mg, 4.70 mmol) were added to a three-necked flask, and the system was purged of air with nitrogen. Then 6 mL of DMF and 6 mL of water were added. The reaction mixture was heated to 100 °C and stirred for 24 h. The reaction mixture was cooled to room temperature and filtered. The filtrate was poured into 60 mL of acetone, and a solid precipitated. The mixture was filtered to give a pale yellow solid (880 mg). The crude product was dissolved in 2 mL of water. Ethanol (10 mL) was slowly added dropwise, and a large amount of solid precipitated. The mixture was filtered, and the solid was dried under reduced pressure to give **compound 16b** as a white solid (570 mg, yield: 63%).

MS m/z (ESI): 393.8[M-2Na+2H+18]⁺.

**Step 3: Compound 16b** (570 mg, 1.36 mmol) was weighed into a dry three-necked flask, and the system was purged with argon. TFA/Ac₂O (1:1, 10 mL) was added to dissolve the compound, and **compound 2d** (452 mg, 0.54 mmol) was then added. After the addition, the reaction mixture was heated to 70 °C and stirred for 2 h. The reaction mixture was concentrated under reduced pressure and purified by column chromatography (mobile phase: aqueous phase and acetonitrile; gradient ratio: aqueous phase 25%-42%) to give a white solid (157 mg). The solid was dissolved in 2 mL of water, and the pH of the system was then adjusted to about 7 with a 0.5 M aqueous sodium hydroxide solution. Ethanol (10 mL) was added, and the mixture was filtered to give compound 16 as a white solid (116 mg, yield: 13.8%).

MS m/z (ESI): 774.4[M/2-4Na+5HHH]⁺.

¹H NMR (400 MHz, D₂O): δ 7.76-7.74 (m, 4 H), 7.01-6.99 (m, 4 H), 5.52-5.46 (m, 6 H), 5.29-5.17 (m, 8 H), 4.48-4.36 (m, 8 H), 4.18-4.06 (m, 8 H), 3.95 (d, 2 H), 3.46 (t, 8 H), 2.15-2.07 (m, 8 H), 1.52-1.39 (m, 8 H).

### Example 17. Preparation of Compound 17

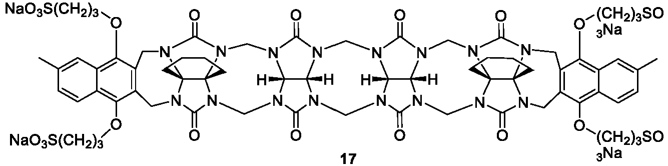

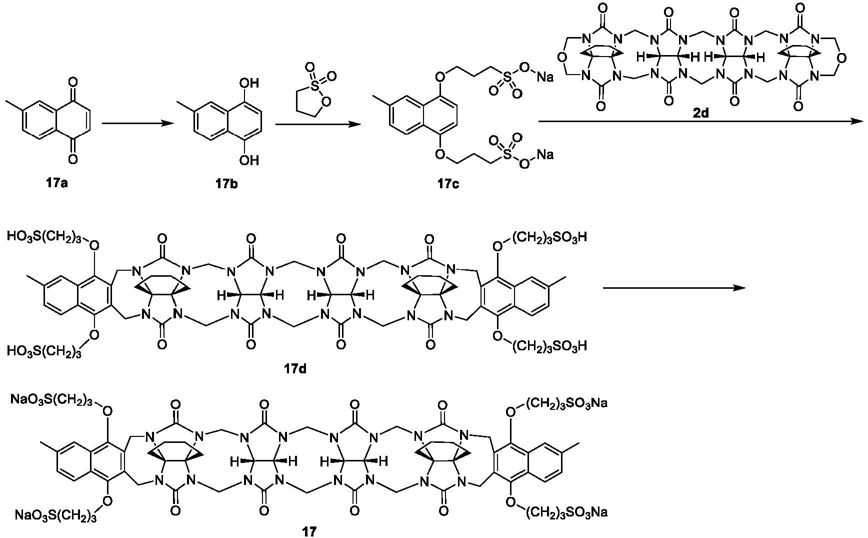

**Step 1:** Zinc powder (3.720 g, 56.9 mmol) and acetic acid (20 mL) were sequentially added to a 100-mL three-necked flask, and the system was purged with nitrogen three times. Subsequently, **compound 17a** (0.980 g, 5.69 mmol) was added in one go, and the mixture was stirred at room temperature for 5-10 min. In-process control showed that the reaction was complete. The reaction mixture was filtered, and the filter cake was rinsed with 15 mL of acetic acid. The filtrate was concentrated under reduced pressure, and the resulting solid was dissolved in 50 mL of ethyl acetate. The solution was washed with 20 mL of saturated brine, dried over anhydrous sodium sulfate, filtered, and concentrated to give **compound 17b** (0.982 g, yield: 99%, purity: 95.8%).

MS-ESI: m/z 175.1[M+H]⁺.

**Step 2:** Sodium hydroxide (0.370 g, 9.24 mmol) and water (3.3 mL) were sequentially added to a 50-mL three-necked flask, and the system was purged with nitrogen three times. After the compound was dissolved by stirring, **compound 17b** (0.7 g, 4.02 mmol) was added, and the mixture was cooled using an ice-water bath. Then a solution of 1,3-propane sultone (1.030 g, 9.24 mmol) in dioxane (8.4 mL) was added dropwise. After the dropwise addition, the mixture was warmed to room temperature and stirred for another 19 h. In-process control showed that the starting material was completely reacted. The reaction mixture was filtered, and the filter cake was rinsed with a dioxane/water (5 mL/1 mL) mixed solvent and dioxane (5 mL) in sequence. Then the filter cake was concentrated with toluene (15 mL × 3) to give **compound 17c** (0.830 g, yield: 44.6%, purity: 99.7%).

MS-ESI: m/z 418.9[M-2Na+3H]⁺.

¹H NMR (400 MHz, D₂O): δ 8.01 (d, *J =* 8.4 Hz, 1 H), 7.89 (s, 1 H), 7.37-7.35 (m, 1 H), 6.80-6.73 (m, 2 H), 4.16-4.13 (m, 4 H), 3.10-3.06 (m, 4 H), 2.42 (s, 3 H) 2.25-2.17 (m, 4 H).

**Step 3: Compound 17c** (0.416 g, 0.9 mmol), TFA (6 mL), and **compound 2d** (0.3 g, 0.36 mmol) were sequentially added to a 50-mL single-necked flask, and the system was purged with nitrogen three times. The mixture was heated to 70 °C using an oil bath and left to react for 3 h. In-process control showed that the reaction was complete, and the reaction was terminated. The reaction mixture was cooled to room temperature and concentrated to give a solid. Ethanol (15 mL) was added, and the mixture was stirred for 10 min and filtered. The filter cake was rinsed with ethanol (10 mL) to give crude **compound 17d** (822 mg, yield: 35.7%, purity: 97.7%).

MS-ESI: m/z 1650.4[M+18]⁺.

**Step 4: Compound 17d** (0.210 g, 0.128 mmol) and THF (4 mL) were sequentially added to a 100-mL single-necked flask, and the pH of the solution was adjusted to 7 with a 0.5 N aqueous sodium hydroxide solution. Subsequently, ethanol (12 mL) was added dropwise, and a solid precipitated. The mixture was filtered, and the filter cake was rinsed with ethanol (5 mL). The resulting filter cake was lyophilized to give **compound 17** (0.153 g, yield: 69.2%, purity: 98.02%).

MS-ESI: m/z 1650.3[M-4Na+18]⁺.

¹H NMR (400 MHz, DMSO-d6): δ 7.78-7.71 (m, 4 H), 7.53-7.52 (m, 2 H), 5.59-5.24 (m, 14 H), 4.33-3.72 (m, 18 H), 2.78-2.62 (m, 14 H), 2.44-1.86 (m, 17 H), 1.46-1.23 (m, 9 H).

### Example 18. Preparation of Compound 18

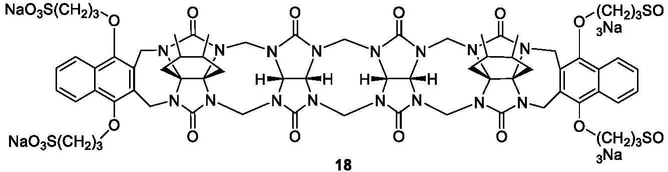

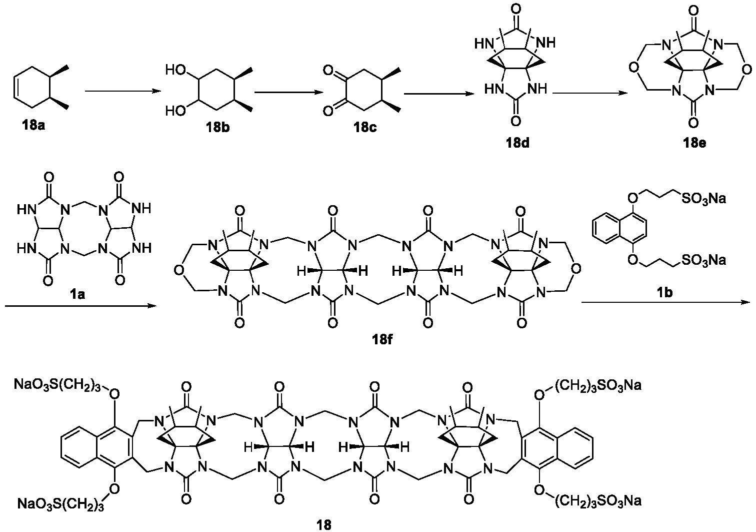

**Step 1: Compound 18a** (338 mg, 3.43 mmol, prepared using the well-known method Journal of the American Chemical Society, 1996, vol. 118, # 34, p. 7946-7968*)* was added to 2 mL of tert-butanol and dissolved by stirring, and N-methylmorpholine oxide (803.4 mg, 6.86 mmol) and potassium osmate dihydrate (25.3 mg, 68.6 µmol) were then added. After the addition, the mixture was left to react at room temperature for 16 h, and in-process control showed that the reaction was complete. The reaction mixture was quenched with 5 mL of an aqueous sodium sulfite solution and extracted with ethyl acetate (20 mL × 5). The organic phases were combined, dried over anhydrous sodium sulfate, filtered, and concentrated, and the crude product was then separated by column chromatography (PE:EA = 1:1) to give **compound 18b** (197 mg, yield: 40%).

¹H NMR (400 MHz, CDCl₃): δ 3.83 (s, 2H), 2.89 (s, 2H), 1.97-1.90 (m, 2H), 1.77-1.72 (m, 2H), 1.51-1.45 (m, 2H), 0.87-0.82 (m, 6H).

**Step 2:** DMSO (12.22 g, 156.41 mmol) and DCM (310 mL) were added to a 500-mL three-necked reaction flask, and the system was put under a nitrogen atmosphere and cooled to -60 °C. Trifluoroacetic anhydride (29.15 g, 138.77 mmol) was added dropwise over about 25 min. After 20 min of stirring at that temperature, a solution of **compound 18b** (5.1 g, 35.36 mmol) in DCM (15 mL) was added. After 1.5 h of stirring at that temperature, triethylamine (32.67 g, 322.86 mmol) was added. After 1 h of stirring at -60 °C, the mixture was warmed to room temperature and stirred. In-process control showed that the reaction was complete. 10% hydrochloric acid (324 mL) was added to the reaction system, and liquid separation was performed. The aqueous phase was extracted with DCM (100 mL × 2). The organic phases were combined, dried over anhydrous sodium sulfate, and concentrated, and the crude product was then separated by column chromatography (PE:EA = 10:1) to give **compound 18c** (3.715 g, yield: 75%).

MS m/z (ESI): 141.1[M+H]⁺.

¹H NMR (400 MHz, CDCl₃): δ 6.06 (d, 1 H), 5.90 (s, 1 H), 2.65-2.57 (m, 1H), 2.47-2.34 (m, 3H), 1.06 (d, 3 H), 1.00 (d, 3 H).

**Step 3:** Urea (5.25 g, 87.35 mmol), 0.3 M dilute hydrochloric acid (13.4 mL), and **compound 18c** (3.71 g, 26.47 mmol) were added to a 100-mL reaction flask, heated to 50 °C, and stirred for 16 h. The reaction mixture was cooled to room temperature and filtered, and the filter cake was rinsed with 10 mL of water, rinsed with 12 mL of absolute ethanol, and dried to give **compound 18d** (2.34 g, yield: 39%).

MS m/z (ESI): 225.1[M+H]⁺.

¹H NMR (400 MHz, DMSO-d6): δ 7.10 (s, 2 H), 7.02 (s, 2 H), 1.75-1.71 (m, 4H), 1.57-1.51 (m, 2H), 0.84 (s, 3 H), 0.82 (s, 3 H).

**Step 4: Compound 18d** (1.12 g, 5.0 mmol), 5 mL of 9 M hydrochloric acid, and paraformaldehyde (751 mg, 25.0 mmol) were added to a 50-mL reaction flask, and the reaction mixture was stirred at room temperature for 24 h. Water (18 mL) was added to the reaction system, and the mixture was stirred at room temperature for another 22 h. The reaction mixture was filtered, and the filter cake was rinsed with 5 mL of water, rinsed with 5 mL of ethanol, and dried to give **compound 18e** (0.54 g, yield: 35%).

MS m/z (ESI): 309.1[M+H]⁺.

¹H NMR (400 MHz, DMSO-d6): δ 5.21-5.17 (m, 4H), 4.97-4.88 (m, 4H), 2.64-2.60 (m, 2H), 1.87-1.75 (m, 2H), 1.66-1.56 (m, 2H), 0.92 (s, 3 H), 0.90 (s, 3 H).

**Step 5: Compound 18e** (0.54 g, 1.75 mmol) was weighed into a dry reaction flask, the system was purged with argon, and methanesulfonic acid (1.8 mL) was added to dissolve the compound. **Compound 1a** (0.18 g, 0.584 mmol) was added at room temperature, and the mixture was stirred for 24 h. The reaction mixture was slowly added to 18 mL of water (cooled in an ice-water bath). After the addition, the mixture was warmed to room temperature, stirred for 10 min, and filtered, and the filter cake was washed with a small amount of water. The crude product was dissolved in TFA (0.72 mL), and 2.88 mL of water was then added. The mixture was stirred at room temperature for 10 min and filtered, and the filter cake was washed with a small amount of water and dried *in vacuo* to give **compound 18f** (0.518 g, yield: 99%).

MS m/z (ESI): 889.3[M+H]⁺.

¹H NMR (400 MHz, DMSO-d6): δ 5.74-5.35 (m, 10 H), 5.21-5.08 (m, 4 H), 4.86-4.69 (m, 4 H), 4.29-4.05 (m, 4 H), 1.80-1.43 (m, 10 H), 0.98-0.74 (m, 16 H).

**Step 6: Compound 18f** (502 mg, 0.565 mmol) was weighed into a dry three-necked flask, and the system was purged with argon. Trifluoroacetic acid (5 mL) was added to dissolve the compound, and **compound 1b** (637 mg, 1.42 mmol) was then added. After the addition, the reaction mixture was heated to 70 °C and stirred for 3 h. TFA was removed by evaporation under reduced pressure, and 7.5 mL of ethanol was added to the resulting solid. The mixture was heated at reflux for 1 h, cooled to room temperature, and filtered. The filter cake was washed with ethanol and dried. The resulting solid was purified by preparative high performance liquid chromatography (mobile phase: water and acetonitrile; gradient ratio: aqueous phase 25%-42%) and then salified with sodium hydroxide to give **compound 18** (0.12 g, yield: 12%).

MS m/z (ESI): 1678.5[M-4Na+4H+18]⁺.

¹H NMR (400 MHz, DMSO-d6): δ 8.00-7.89 (m, 4 H), 7.75-7.63 (m, 4 H), 7.35-6.99 ( (m, 7 H), 5.63-5.19 (m, 14 H), 4.42-4.00 (m, 12 H), 3.92-3.78 (m, 3H), 2.88-2.77 (m, 7 H), 2.66-2.56 (m, 1 H), 2.19-1.94 (m, 8 H), 1.83-1.62 (m, 8 H), 1.01-0.82 (m, 12 H).

### Example 19. Preparation of Compound 19

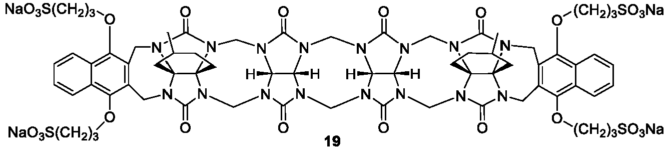

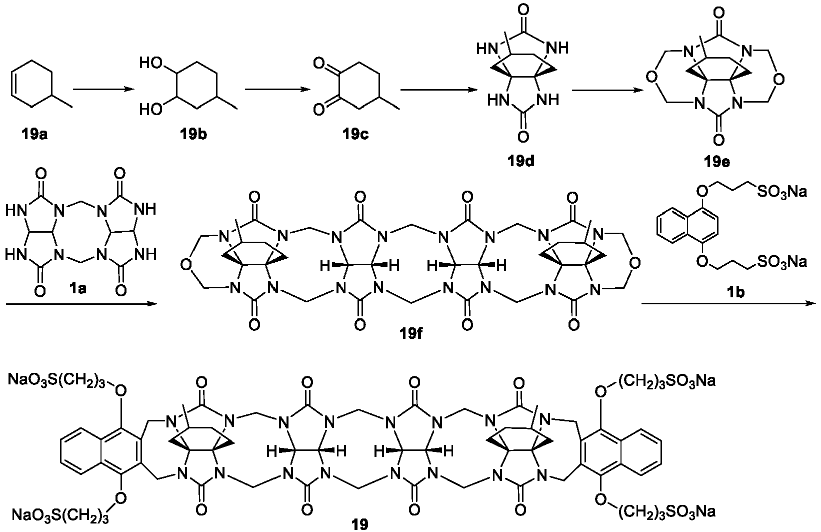

**Step 1: Compound 19a** (4 g, 41.59 mmol) was added to tert-butanol (1.4 mL) and dissolved by stirring, and N-methylmorpholine oxide (5.43 g, 46.35 mmol) and potassium osmate dihydrate (172 mg, 467 µmol) were then added. After the addition, the mixture was left to react at room temperature for 16 h, and in-process control showed that the reaction was complete. The reaction mixture was quenched with 30 mL of an aqueous sodium sulfite solution and extracted with ethyl acetate (3 × 30 mL). The organic phases were combined, dried over sodium sulfate, filtered, and concentrated to give a crude product, and the crude product was separated by column chromatography (PE:EA= 1:1) to give **compound 19b** (3 g, yield: 55%).

¹H NMR (400 MHz, CDCl₃): δ 3.99-3.91 (m, 1H), 3.66-3.54 (m, 1H), 2.43-2.11 (m, 2H), 1.97-1.61 (m, 4H), 1.50-1.09 (m, 3H), 0.97-0.85 (m, 3H).

**Step 2:** DMSO (9.82 g, 125.69 mmol) and DCM (200 mL) were added to a 500-mL three-necked reaction flask, and the system was put under a nitrogen atmosphere and cooled to -60 °C. Trifluoroacetic anhydride (24.24 g, 115.40 mmol) was added dropwise over about 20 min. After 20 min of stirring at that temperature, a solution of **compound 19b** (3 g, 23.04 mmol) in DCM (8 mL) was added. After 1.5 h of stirring at that temperature, triethylamine (26.50 g, 261.87 mmol) was added. After 1 h of stirring at -60 °C, the mixture was warmed to room temperature and stirred. In-process control showed that the reaction was complete. 10% hydrochloric acid (about 216 mL) was added to the reaction system, and extraction was performed with DCM (60 mL × 3). The extract was dried over anhydrous sodium sulfate and concentrated, and the crude product was then separated by column chromatography (PE:EA = 10:1) to give **compound 19c** (2.01 g, yield: 69%).

MS m/z (ESI): 127.1[M+H]⁺.

¹H NMR (400 MHz, CDCl₃): δ 6.13-6.09 (m, 1 H), 2.64-2.37 (m, 3H), 2.29-2.06 (m, 3H), 1.09 (d, 3 H).

**Step 3:** Urea (3.17 g, 52.78 mmol), 0.3 M dilute hydrochloric acid (7.24 mL), and **compound 19c** (2.01 g, 15.93 mmol) were added to a 100-mL reaction flask, heated to 50 °C, and stirred for 16 h. The reaction mixture was cooled to room temperature and filtered, and the filter cake was rinsed with 10 mL of water, rinsed with 10 mL of absolute ethanol, and dried to give **compound 19d** (1.52 g, yield: 45%).

MS m/z (ESI): 211.1[M+H]⁺.

¹H NMR (400 MHz, DMSO-d6): δ 7.11-6.98 (m, 4 H), 2.00-1.86 (m, 2H), 1.59-1.44 (m, 3H), 1.26-1.10 (m, 1H), 1.00-0.86 (m, 4H).

**Step 4: Compound 19d** (1.02 g, 4.85 mmol), 4.85 mL of 9 M hydrochloric acid, and paraformaldehyde (730 mg, 24.31 mmol) were added to a 50-mL reaction flask, and the reaction mixture was stirred at room temperature for 24 h. The reaction mixture was concentrated, and the residue was separated by column chromatography (dichloromethane:acetonitrile = 10:1) to give **compound 19e** (191 mg, yield: 13%).

MS m/z (ESI): 295.1[M+H]⁺.

¹H NMR (400 MHz, DMSO-d6): δ 5.21-5.17 (m, 4H), 4.96-4.88 (m, 4H), 2.62-2.57 (m, 1H), 1.96-1.61 (m, 3H), 1.25-1.17 (m, 3H), 1.00 (d, 3 H).

**Step 5: Compound 19e** (66 mg, 0.21 mmol) was weighed into a dry three-necked flask, and the system was purged with argon. Methanesulfonic acid (0.5 mL) was added to dissolve the compound, and **compound 1a** (189 mg, 0.64 mmol) was added at room temperature. After 18 h of stirring at room temperature, the reaction mixture was slowly added to 5 mL of water (cooled in an ice-water bath). After the addition, the mixture was warmed to room temperature and filtered, and the filter cake was washed with a small amount of water. The dried crude product was dissolved in TFA (0.26 mL), and 1 mL of water was then added. The mixture was stirred at room temperature for 10 min and filtered, and the filter cake was washed with a small amount of water and dried *in vacuo* to give **compound 19f** (185 mg, yield: 100%).

MS m/z (ESI): 878.1[M+18]⁺.

¹H NMR (400 MHz, DMSO-d6): δ 5.68-5.42 (m, 10 H), 5.16-5.14 (m, 4 H), 4.80-4.74 (m, 4 H), 4.20-4.16 (m, 6 H), 2.08-1.99 (m, 2 H), 1.55-1.47 (m, 6 H), 1.24-0.97 (m, 12 H).

**Step 6: Compound 19f** (204 mg, 0.24 mmol) was weighed into a dry three-necked flask, and the system was purged with argon. TFA (2 mL) was added to dissolve the compound, and **compound 1b** (266 mg, 0.59 mmol) was then added. After the addition, the reaction mixture was heated to 70 °C and stirred for 3 h. TFA was removed by evaporation under reduced pressure, and 3 mL of ethanol was added to the resulting solid. The mixture was heated at reflux (80 °C) for 40 min, cooled to room temperature, and filtered. The filter cake was washed with ethanol and dried to give a crude product, and the crude product was purified by preparative high performance liquid chromatography (mobile phase: water and acetonitrile; gradient ratio: aqueous phase 25%-42%) and then salified with sodium hydroxide to give **compound 19** (0.15 g, yield: 36%).

MS m/z (ESI): 1650.4[M-4Na+4H+18]⁺.

¹H NMR (400 MHz, D₂O): δ 7.99-7.55 (m, 4 H), 7.47-7.04 (m, 4 H), 5.43-4.81 (m, 14 H), 4.20-3.52 (m, 18H), 3.07-2.66 (m, 8 H), 2.20-0.90 (m, 28 H).

### Test Example 1. Antagonistic Effects of Disclosed Compounds on Muscle Relaxants

### I. Objective

To evaluate the reversal effects of the compounds provided in the present disclosure on the efficacy of muscle relaxants in the gastrocnemius muscle of a rat neuromuscular model based on indices such as onset time and TOF and compare the compounds with CB2 and neostigmine.

### II. Materials

SPF SD male rats, BL-420A biological function experiment system (host, stimulator, and tension transducer), ventilator, electronic balance, surgical instruments, syringes, hair clipper, electronic balance, iron stand, foam board, ethyl carbamate, sodium chloride, CB2, compound 2, compound 3, cisatracurium, succinylcholine, neostigmine, sterile water, and 95% alcohol.

### III. Method

Quarantined and certified SPF male SD rats were housed under controlled conditions (room temperature 22 ± 0.5 °C; 20-50 air changes/h; air velocity: 0.05-0.18 m/s; 12/12-hour light/dark cycle). The rats were acclimatized in the animal facility for 3 or more days and housed 6 per cage. The experiment was started when their weight ranged from 220 g to 250 g.

The test compounds were reconstituted using 0.9% sodium chloride. After content calculation, the required amounts of the test compounds were weighed out and dissolved in a 0.9% sodium chloride solution for injection (within 30 min), and then the solutions were each well mixed and shaken using a vortex mixer. CB2 was reconstituted using purified water. Actual drug weight (mg) = administered formulation concentration A (mg/mL) × vehicle volume (mL) / content (%)

The rats were randomized into groups of 4 (the actual number of rats per group prevails): CB2, compound 2, compound 3, and neostigmine. The administered volume was 2 mL/kg.

The structure of CB2 (prepared using the known method "WO2012051407A2") is shown below:

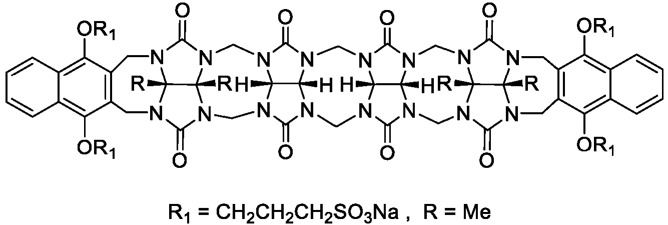

After the rats were anesthetized, the sciatic nerve and gastrocnemius muscle were isolated. The sciatic nerve was stimulated, and muscle tension signals were recorded using a tension transducer. Tracheal intubation was performed, and mechanical ventilation was provided using a small animal ventilator. After muscle tension signals were stably recorded for a period of time, drugs were administered: First, a muscle relaxant (cisatracurium) was administered at 2 times the ED₉₀ dose (0.8 mg/kg), and there should be a decrease in the muscle tension curve. Thirty to sixty seconds after administration, antagonists (the test compounds and neostigmine) were injected. After the muscle tension curve recovered to 95% or more, a muscle relaxant (succinylcholine) was administered at the ED₉₀ dose (0.9 mg/kg), and a decrease in muscle tension should be observed. Once the muscle tension naturally recovered to 95% or more, the experiment was terminated. Muscle tension signals were continuously recorded during the experiment. Indices such as onset time and duration time were statistically analyzed. By comparing muscle tension signals after administration, the reversal effects of the antagonists on muscle relaxation were determined and compared.

### IV. Procedures

### 4.1. Rat weighing and anesthesia

The rats were weighed, and after their emotional state stabilized, a 25% urethane (ethyl carbamate) solution was prepared and intraperitoneally injected at 1 mL/100 g to anesthetize the rats. After the pain reflex disappeared, the rats were secured in a prone position on a foam board. Hair removal was performed on the gluteal region and the lateral side of the right thigh.

### 4.2. Sciatic nerve isolation

An incision was made in the skin along the lateral edge of the femur at the middle of the thigh, posterior to the hip joint. The skin and superficial fascia layer were lifted, and the muscles were bluntly dissected to expose the sciatic nerve. It should be noted that a glass dissecting needle was used in the dissection to prevent metal instruments from damaging the nerve.

### 4.3. Gastrocnemius muscle isolation

An incision was made in the skin of the lower leg from the ankle joint, and the anterior ligaments of the ankle joint were severed. The gastrocnemius muscle was isolated. A ligature was tied around the gastrocnemius tendon at the ankle, and the tendon was cut off distal to the ligature.

### 4.4. Signal collection

The ligature on the gastrocnemius muscle was connected to a tension transducer, and a stimulator was connected to the sciatic nerve. The input signal was set to tension, and the parameters were set as follows: square wave; fine voltage; train stimulation; latency: 0.05 ms; wave width: 0.2 ms; frequency: 2 Hz; intensity: 0.225 ± 0.025 V; intensity increment: 0; train length: 4; main period: 12 s; stop count: 30,000. The muscle contraction curve was recorded. During the measurement, the muscle and nerve were always kept moist with normal saline, which was applied once every 3-5 min.

### 4.5. Connecting to the ventilator and administering muscle relaxants and antagonists

The ventilator tube mouth was wiped with alcohol. An incision was made in the skin of the neck to locate the jugular vein and trachea. The trachea was cut open and connected to the ventilator, which was configured with the following parameters: tidal volume: 6 mL; inspiration-to-expiration ratio: 5:4; breathing frequency: 80 breaths/min. After about 5 min of stabilization, drugs were administered via the jugular vein based on settings described in references: First, a muscle relaxant (cisatracurium) was administered at 2 times the ED₉₀ dose, and there should be a decrease in the muscle tension curve. Thirty to sixty seconds after administration, an antagonist (a test compound or neostigmine) was injected. After the muscle tension curve recovered to 95% or more, a muscle relaxant (succinylcholine) was administered at the ED₉₀ dose. Once the muscle tension curve recovered to 95% or more again, the experiment was terminated. The muscle tension curve was continuously recorded during the experiment.

**Table 1.**

| Drug | Dose (mg/kg) | N | Route of administration | Process | Indices |
|---|---|---|---|---|---|
| Neostigmine | 0.08 | 4 | The drugs were dissolved in normal saline except that CB2 was dissolved in purified water, and the solutions were intravenously injected. | Cisatracurium was intravenously injected at 0.8 mg/kg for muscle relaxation, and after 30-60 s, an antagonist was administered. After the muscle tension recovered and substantially stabilized, succinylcholine was intravenously injected at 0.9 mg/kg for secondary muscle relaxation. | TOF0.9; Onset time of succinylcholine for secondary muscle relaxation |
| CB2 | 20 | 4 | | | |
| | 40 | 4 | | | |
| | | | | | |
| Compound 2 | | | | | |
| Compound 3 | 60 | 4 | | | |

### 4.6. Statistics for duration time indices

Indices such as onset time and onset time for secondary muscle relaxation were statistically analyzed using a biological function experiment system. The statistical criteria are shown below:
1) Time for TOF to recover to 90% (TOF0.9): the time required for the T₄/T₁ value of the TOF stimulation to recover to about 90% - the time when the antagonist was administered; and
2) Duration of action of succinylcholine for muscle relaxation: the time when the muscle tension decreased to its lowest value - the time when succinylcholine was administered.

### V. Conclusion

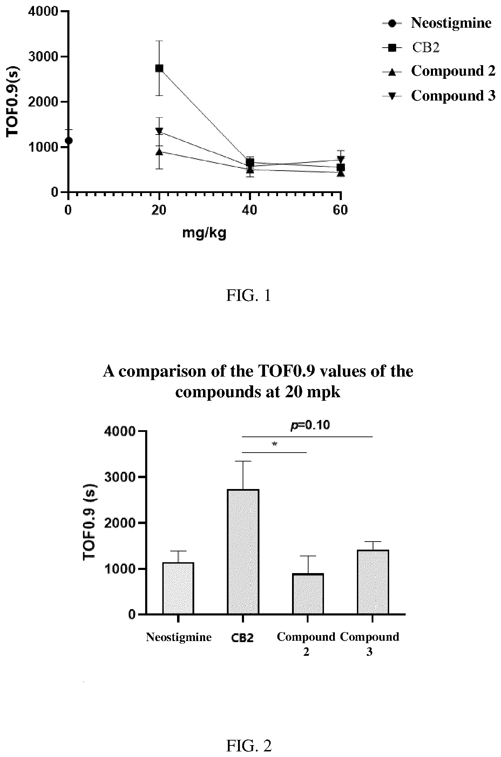

As can be seen from FIGs. 1 and 2, compounds 2 and 3 at 20 mg/kg achieved the efficacy level of neostigmine and outperformed CB2.

### Test Example 2. In Vitro Binding Activity of Disclosed Compounds to Muscle Relaxant

### I. Objective:

To evaluate the *in vitro* binding activity of the compounds of the present disclosure to a muscle relaxant based on K_{d} values using isothermal titration calorimetry (ITC).

### II. Materials:

Isothermal titration calorimeter (including a computer host and matched software), CB2, compound 2, compound 3, compound 7, compound 12, compound 13, compound 15, cisatracurium, and deionized water.

### III. Method and procedures:

The tubing, sample cell, and titration syringe of the isothermal titration calorimetry system were washed with deionized water using the preset washing program. An aqueous solution of cisatracurium and aqueous solutions of the test compounds were prepared, with cisatracurium:test compound = 10:1-20:1. The sample cell was filled with CB2 and a test compound using the sample-loading syringe. Cisatracurium was drawn into the titration syringe using the sample-loading program. The titration syringe was placed into the sample cell, stirring was started, and the system was equilibrated for 5-10 min. The titration parameters were set as follows: A total of 20 sample drops, 2.5 µL each; titration interval: 150 s. Titration was started, and the thermal curve was recorded.

After the titration was completed, the K_{d} value for the binding of the test compound to cisatracurium was calculated based on the thermal curve. The tubing, sample cell, and titration syringe were washed using the washing program, and the next compound was tested.

### IV. Results:

The ability of CB2, compound 2, compound 3, compound 7, compound 12, compound 13, and compound 15 to bind to cisatracurium *in vitro* was measured using the ITC method, and their dissociation constant K_{d} values are shown in Table 2. The results show that the ability of compound 2, compound 13, and compound 15 to bind to cisatracurium *in vitro* is greater than that of CB2.

**Table 2: The ability of the test compounds to bind to cisatracurium in vitro**

| **Test compound** | **K_{d} (M)** |
|---|---|
| CB2 | 1.736E-5 |
| Compound 2 | 7.068E-6 |
| Compound 3 | 1.66E-5 |
| Compound 7 | 6.171E-5 |
| Compound 12 | 5.925E-5 |
| Compound 13 | 2.431E-6 |
| Compound 15 | 1.044E-5 |

### Test Example 3. Antagonistic Effects of Disclosed Compounds on Muscle Relaxants

### I. Objective

To evaluate the reversal effects of the compounds provided in the present disclosure on the efficacy of muscle relaxants in the gastrocnemius muscle of a rat neuromuscular model based on indices such as onset time and TOF and compare the compounds with CB2 and neostigmine.

### II. Materials

SPF SD male rats, BL-420A biological function experiment system (host, stimulator, and tension transducer), ventilator, electronic balance, surgical instruments, syringes, hair clipper, electronic balance, iron stand, foam board, ethyl carbamate, sodium chloride, CB2, compound 2, compound 3, cisatracurium, succinylcholine, neostigmine, sterile water, and 95% alcohol.

### III. Method

Quarantined and certified SPF male SD rats were housed under controlled conditions (room temperature 22 ± 0.5 °C; 20-50 air changes/h; air velocity: 0.05-0.18 m/s; 12/12-hour light/dark cycle). The rats were acclimatized in the animal facility for 3 or more days and housed 6 per cage. The experiment was started when their weight ranged from 220 g to 250 g.

The test compounds were reconstituted using 0.9% sodium chloride. After content calculation, the required amounts of the test compounds were weighed out and dissolved in a 0.9% sodium chloride solution for injection (within 30 min), and then the solutions were each well mixed and shaken using a vortex mixer. CB2 was reconstituted using purified water. Actual drug weight (mg) = administered formulation concentration A (mg/mL) × vehicle volume (mL) / content (%)

The rats were randomized into groups of 5 (the actual number of rats per group prevails): CB2, compound 2, compound 15, and neostigmine. The administered volume was 2 mL/kg.

The structure of CB2 (prepared using the known method "WO2012051407A2") is shown below: 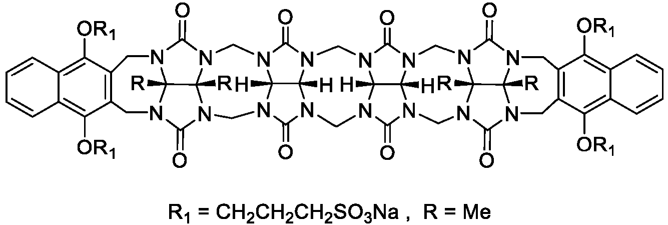 After the rats were anesthetized, the sciatic nerve and gastrocnemius muscle were isolated. The sciatic nerve was stimulated, and muscle tension signals were recorded using a tension transducer. Tracheal intubation was performed, and mechanical ventilation was provided using a small animal ventilator. After muscle tension signals were stably recorded for a period of time, drugs were administered: First, a muscle relaxant (cisatracurium) was administered at 2 times the ED₉₀ dose (0.8 mg/kg), and there should be a decrease in the muscle tension curve. Thirty to sixty seconds after administration, antagonists (the test compounds and neostigmine) were injected. After the muscle tension curve recovered to 95% or more, a muscle relaxant (succinylcholine) was administered at the ED₉₀ dose (0.9 mg/kg), and a decrease in muscle tension should be observed. Once the muscle tension naturally recovered to 95% or more, the experiment was terminated. Muscle tension signals were continuously recorded during the experiment. Indices such as onset time and duration time were statistically analyzed. By comparing muscle tension signals after administration, the reversal effects of the antagonists on muscle relaxation were determined and compared.

### IV. Procedures

### 4.1. Rat weighing and anesthesia

The rats were weighed, and after their emotional state stabilized, a 25% urethane (ethyl carbamate) solution was prepared and intraperitoneally injected at 1 mL/100 g to anesthetize the rats. After the pain reflex disappeared, the rats were secured in a prone position on a foam board. Hair removal was performed on the gluteal region and the lateral side of the right thigh.

### 4.2. Sciatic nerve isolation

An incision was made in the skin along the lateral edge of the femur at the middle of the thigh, posterior to the hip joint. The skin and superficial fascia layer were lifted, and the muscles were bluntly dissected to expose the sciatic nerve. It should be noted that a glass dissecting needle was used in the dissection to prevent metal instruments from damaging the nerve.

### 4.3. Gastrocnemius muscle isolation

An incision was made in the skin of the lower leg from the ankle joint, and the anterior ligaments of the ankle joint were severed. The gastrocnemius muscle was isolated. A ligature was tied around the gastrocnemius tendon at the ankle, and the tendon was cut off distal to the ligature.

### 4.4. Signal collection

The ligature on the gastrocnemius muscle was connected to a tension transducer, and a stimulator was connected to the sciatic nerve. The input signal was set to tension, and the parameters were set as follows: square wave; fine voltage; train stimulation; latency: 0.05 ms; wave width: 0.2 ms; frequency: 2 Hz; intensity: 0.225 ± 0.025 V; intensity increment: 0; train length: 4; main period: 12 s; stop count: 30,000. The muscle contraction curve was recorded. During the measurement, the muscle and nerve were always kept moist with normal saline, which was applied once every 3-5 min.

### 4.5. Connecting to the ventilator and administering muscle relaxants and antagonists

The ventilator tube mouth was wiped with alcohol. An incision was made in the skin of the neck to locate the jugular vein and trachea. The trachea was cut open and connected to the ventilator, which was configured with the following parameters: tidal volume: 6 mL; inspiration-to-expiration ratio: 5:4; breathing frequency: 80 breaths/min. After about 5 min of stabilization, drugs were administered via the jugular vein based on settings described in references: First, a muscle relaxant (cisatracurium) was administered at 2 times the ED₉₀ dose, and there should be a decrease in the muscle tension curve. Thirty to sixty seconds after administration, an antagonist (a test compound or neostigmine) was injected. After the muscle tension curve recovered to 95% or more, a muscle relaxant (succinylcholine) was administered at the ED₉₀ dose. Once the muscle tension curve recovered to 95% or more again, the experiment was terminated. The muscle tension curve was continuously recorded during the experiment.

**Table 3.**

| Drug | Dose (mg/kg) | N | Route of administration | Process | Indices |
|---|---|---|---|---|---|
| Neostigmine | 0.08 | 5 | The drugs were dissolved in normal saline except that CB2 was dissolved in purified water, and the solutions were intravenously injected. | Cisatracurium was intravenously injected at 0.8 mg/kg for muscle relaxation, and after 30-60 s, an antagonist was administered. After the muscle tension recovered and substantially stabilized, succinylcholine was intravenously injected at 0.9 mg/kg for secondary muscle relaxation. | TOF0.9; Onset time of succinylcholine for secondary muscle relaxation |
| CB2 | 20 | 5 | | | |
| Compound 2 | 20 | 5 | | | |
| Compound 15 | 20 | 5 | | | |

### 4.6. Statistics for duration time indices

Indices such as onset time and onset time for secondary muscle relaxation were statistically analyzed using a biological function experiment system. The statistical criteria are shown below:
1) Time for TOF to recover to 90% (TOF0.9): the time required for the T₄/T₁ value of the TOF stimulation to recover to about 90% - the time when the antagonist was administered; and
2) Duration of action of succinylcholine for muscle relaxation: the time when the muscle tension decreased to its lowest value - the time when succinylcholine was administered.

### V. Conclusion

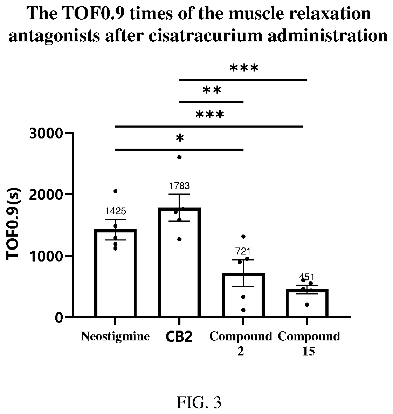

As can be seen from FIG. 3, compounds 2 and 15 at 20 mg/kg achieved the efficacy level of neostigmine and outperformed CB2.

### Test Example 4. In Vitro Binding Activity of Disclosed Compounds to Muscle Relaxant

### I. Objective:

To evaluate the *in vitro* binding activity of the compounds of the present disclosure to a muscle relaxant based on K_{d} values using isothermal titration calorimetry (ITC).

### II. Materials:

Isothermal titration calorimeter (including a computer host and matched software), CB2, compound 2, compound 11, compound 15, compound 16, cisatracurium, and deionized water.

### III. Method and procedures:

The tubing, sample cell, and titration syringe of the isothermal titration calorimetry system were washed with deionized water using the preset washing program. An aqueous solution of cisatracurium and aqueous solutions of the test compounds were prepared, with cisatracurium:test compound = 10:1-20:1. The sample cell was filled with CB2 and a test compound using the sample-loading syringe. Cisatracurium was drawn into the titration syringe using the sample-loading program. The titration syringe was placed into the sample cell, stirring was started, and the system was equilibrated for 5-10 min. The titration parameters were set as follows: A total of 20 sample drops, 2.5 µL each; titration interval: 150 s. Titration was started, and the thermal curve was recorded.

After the titration was completed, the K_{d} value for the binding of the test compound to cisatracurium was calculated based on the thermal curve. The tubing, sample cell, and titration syringe were washed using the washing program, and the next compound was tested.

### IV. Results:

The ability of CB2, compound 2, compound 11, compound 15, and compound 16 to bind to cisatracurium *in vitro* was measured using the ITC method, and their dissociation constant K_{d} values are shown in Table 4. The results show that the ability of compound 2, compound 11, compound 15, and compound 16 to bind to cisatracurium *in vitro* is greater than that of CB2.

**Table 4: The ability of the test compounds to bind to cisatracurium in vitro**

| **Test compound** | **K_{d} (M)** |
|---|---|
| CB2 | 2.506E-5 |
| Compound 2 | 7.475E-6 |
| Compound 11 | 7.190E-6 |
| Compound 15 | 1.593E-5 |
| Compound 16 | 3.057E-6 |

## Claims

1. A compound represented by formula (I) or a pharmaceutically acceptable salt thereof: 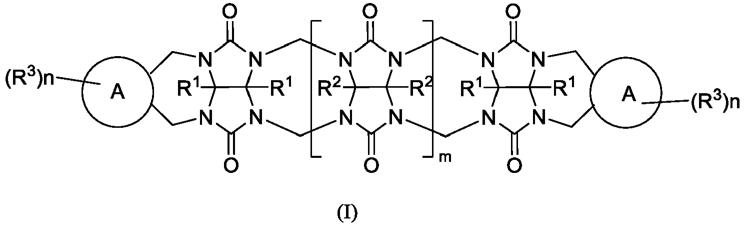 wherein:
each R¹ is independently selected from the group consisting of C₂₋₆ alkyl, C₁₋₆ haloalkyl, C₁₋₆ hydroxyalkyl, -C(O)₂R', R'-(O)-alkylene-, hydroxy, NR'(R"), 3- to 7-membered cycloalkyl, and 3- to 7-membered heterocyclyl; or two R¹ attached to adjacent carbon atoms form together 3- to 10-membered cycloalkyl or 3- to 10-membered heterocyclyl;
R¹ is optionally substituted with one or more R^{1A};
each R² is independently selected from the group consisting of hydrogen, C₁₋₆ alkyl, C₁₋₆ haloalkyl, C₁₋₆ hydroxyalkyl, -C(O)₂R', R'-(O)-alkylene-, hydroxy, NR'(R"), 3- to 7-membered cycloalkyl, and 3- to 7-membered heterocyclyl; or two R² attached to adjacent carbon atoms form together 3- to 10-membered cycloalkyl or 3- to 10-membered heterocyclyl; R² is optionally substituted with one or more R^{2A};
each ring A is independently selected from the group consisting of 5- to 12-membered aryl and 5- to 12-membered heteroaryl;
each R³ is independently selected from the group consisting of halogen, C₁₋₆ alkyl, hydroxy, nitro, cyano, -C(O)₂R', NR'(R"), R'-(O)-alkylene-, 3- to 7-membered cycloalkyl, 3- to 7-membered heterocyclyl, and 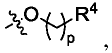 and at least one R³ is 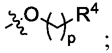 R³ is optionally substituted with one or more R^{3A};
R⁴ is 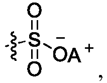 and A⁺ is a monovalent or divalent cation;
R^{1A}, R^{2A}, and R^{3A} are each independently selected from the group consisting of halogen, cyano, nitro, amino, C₁₋₆ alkyl, and C₁₋₆ alkoxy;
R' and R" are each independently selected from the group consisting of hydrogen, C₁₋₆ alkyl, C₁₋₆ hydroxyalkyl, C₁₋₆ haloalkyl, 3- to 7-membered cycloalkyl, and 3- to 7-membered heterocyclyl;
m and n are each independently selected from the group consisting of 1, 2, 3, 4, and 5;
each p is independently selected from the group consisting of 1, 2, 3, 4, 5, and 6.

2. The compound represented by formula (I) or the pharmaceutically acceptable salt thereof according to claim 1, wherein ring A is phenyl or naphthyl, preferably naphthyl.

3. The compound represented by formula (I) or the pharmaceutically acceptable salt thereof according to claim 1 or 2, being a compound represented by formula (I-1) or a pharmaceutically acceptable salt thereof: 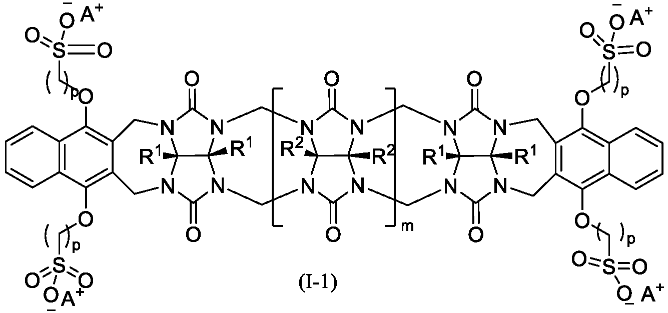 wherein R¹, R², p, m, and A⁺ are as defined in claim 1.

4. The compound represented by formula (I) or the pharmaceutically acceptable salt thereof according to any one of claims 1 to 3, wherein two R¹ attached to adjacent carbon atoms form together 3- to 10-membered cycloalkyl or 3- to 10-membered heterocyclyl; preferably, two R¹ attached to adjacent carbon atoms form together 6-membered cycloalkyl; more preferably, two R¹ attached to adjacent carbon atoms form together 5- to 6-membered heterocyclyl, wherein a heteroatom is selected from the group consisting of nitrogen and oxygen and is preferably oxygen.

5. The compound represented by formula (I) or the pharmaceutically acceptable salt thereof according to any one of claims 1 to 3, wherein each R¹ is independently C₂₋₆ alkyl, preferably ethyl; or each R¹ is independently C₁₋₆ haloalkyl, preferably C₁₋₃ haloalkyl, and more preferably methyl substituted with one or two or three fluorine atoms; or each R¹ is independently -C(O)₂R', wherein each R' is independently selected from the group consisting of hydrogen, C₁₋₆ alkyl, and C₁₋₆ haloalkyl; preferably, R' is H or C₁₋₆ alkyl; more preferably, R' is methyl or ethyl; or each R¹ is independently C₁₋₆ hydroxyalkyl or C₁₋₆ alkyl-O-C₁₋₆ alkylene-, preferably C₁₋₃ hydroxyalkyl or C₁₋₃ alkyl-O-C₁₋₃ alkylene-, and more preferably hydroxymethyl or methyl-O-methylene-; or each R¹ is independently R'-(O)-alkylene-, wherein R' is selected from the group consisting of C₁₋₆ alkyl, C₁₋₆ hydroxyalkyl, C₁₋₆ haloalkyl, 3- to 7-membered cycloalkyl, and 3- to 7-membered heterocyclyl; preferably, R' is C₁₋₆ alkyl or C₁₋₆ haloalkyl; more preferably, R' is C₁₋₃ alkyl.

6. The compound represented by formula (I) or the pharmaceutically acceptable salt thereof according to any one of claims 1 to 5, wherein each R² is independently hydrogen.

7. The compound represented by formula (I) or the pharmaceutically acceptable salt thereof according to any one of claims 1 to 6, wherein A⁺ is a monovalent cation selected from the group consisting of H⁺, Na⁺, K⁺, H₄N⁺, Et₃NH⁺, (HOCH₂CH₂)₃NH⁺, and cationic forms of ethylenediamine, piperazine, and triphenylmethylaminomethane, preferably from the group consisting of H⁺, Na⁺, and K⁺, and more preferably from the group consisting of Na⁺; or A⁺ is a divalent cation selected from the group consisting of Ca²⁺, Mg²⁺, and Zn²⁺.

8. The compound represented by formula (I) or the pharmaceutically acceptable salt thereof according to any one of claims 1 to 7, wherein each p is independently selected from the group consisting of 2, 3, and 4, preferably from the group consisting of 3.

9. The compound represented by formula (I) or the pharmaceutically acceptable salt thereof according to any one of claims 1 to 8, wherein each m is independently selected from the group consisting of 2, 3, and 4, preferably from the group consisting of 2.

10. The compound represented by formula (I) or the pharmaceutically acceptable salt thereof according to any one of claims 1 to 9, being selected from the group consisting of: 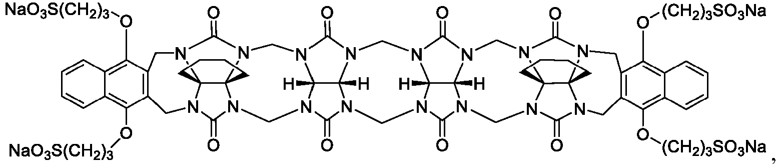 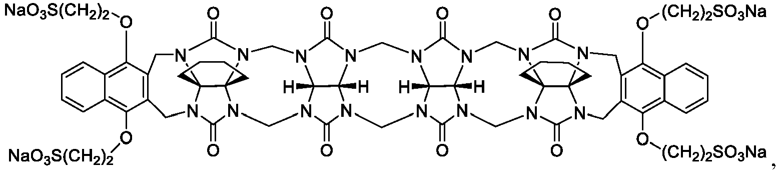 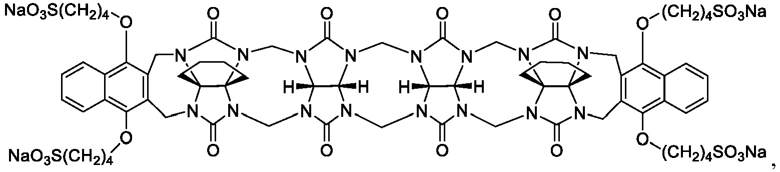 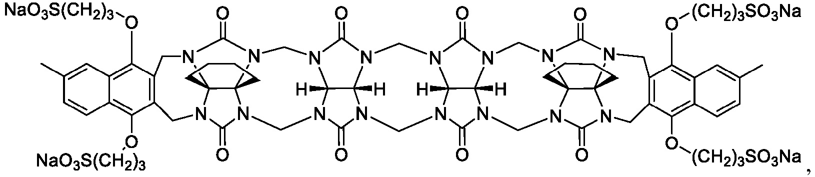 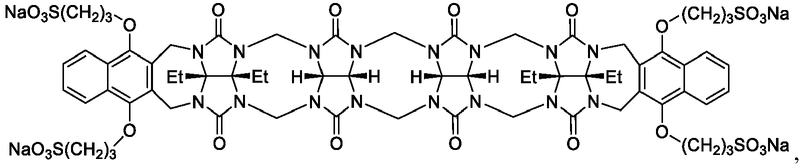 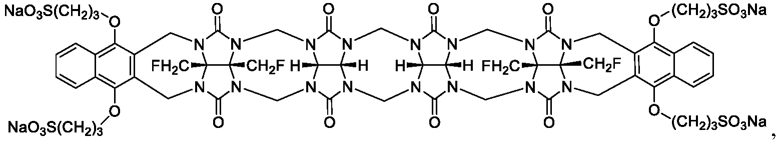 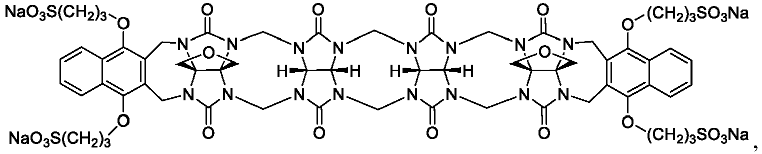 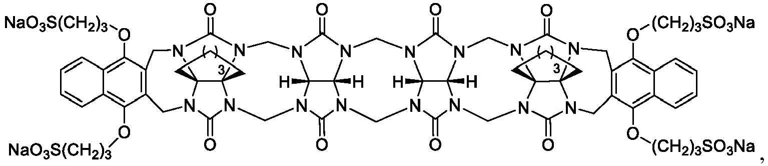 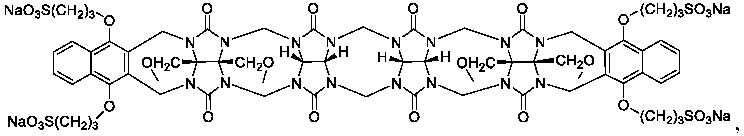 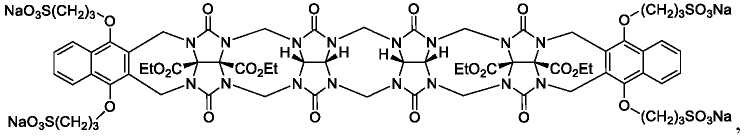 and

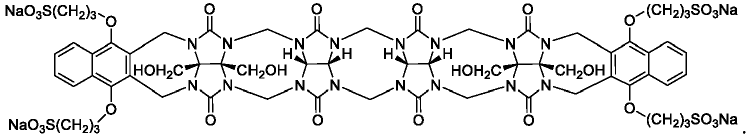

11. A compound represented by formula (III) or a pharmaceutically acceptable salt thereof: 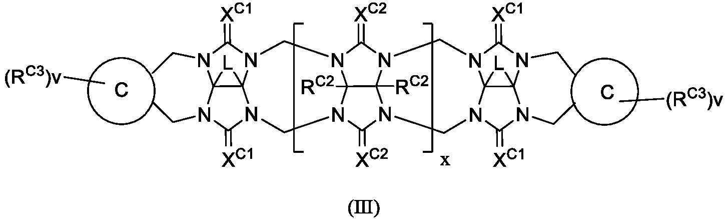 wherein:
X^{C1} and X^{C2} are each independently selected from the group consisting of O, S, and -NH-;
each R^{C2} is independently selected from the group consisting of hydrogen, C₁₋₆ alkyl, C₁₋₆ haloalkyl, C₁₋₆ hydroxyalkyl, -C(O)₂R', R'-(O)-alkylene-, hydroxy, NR'(R"), 3- to 7-membered cycloalkyl, and 3- to 7-membered heterocyclyl; or two R^{C2} attached to adjacent carbon atoms form together 3- to 10-membered cycloalkyl or 3- to 10-membered heterocyclyl; R^{C2} is optionally substituted with one or more halogen, cyano, nitro, amino, C₁₋₆ alkyl, or C₁₋₆ alkoxy groups;
ring C is selected from the group consisting of 6- to 18-membered aryl and 5- to 18-membered heteroaryl;
each R^{C3} is independently 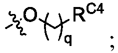
each R^{C4} is independently 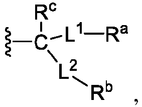 a carboxylic acid group, a -carboxylate-cation, a phosphoric acid group, a -phosphate-cation, a sulfonic acid group, or a -sulfonate-cation;
L, L¹, and L² are identical or different and are each independently alkylene or heteroalkylene, and the alkylene or heteroalkylene is optionally substituted with one or more halogen, cyano, nitro, amino, C₁₋₆ alkyl, or C₁₋₆ alkoxy groups;
each R^{c} is independently selected from the group consisting of hydrogen, halogen, cyano, nitro, amino, C₁₋₆ alkyl, and C₁₋₆ alkoxy;
R^{a} and R^{b} are identical or different and are each independently selected from the group consisting of a carboxylic acid group, a -carboxylate-cation, a phosphoric acid group, a -phosphate-cation, a sulfonic acid group, and a -sulfonate-cation;
R' and R" are each independently selected from the group consisting of hydrogen, C₁₋₆ alkyl, C₁₋₆ hydroxyalkyl, C₁₋₆ haloalkyl, 3- to 7-membered cycloalkyl, and 3- to 7-membered heterocyclyl;
x is selected from the group consisting of 1, 2, 3, 4, and 5;
each v is independently selected from the group consisting of 1, 2, 3, 4, and 5;
each q is independently selected from the group consisting of 1, 2, 3, 4, 5, and 6.

12. The compound represented by formula (III) or the pharmaceutically acceptable salt thereof according to claim 11, wherein X^{C1} and X^{C2} are identical and are selected from the group consisting of O, S, and -NH-, preferably from the group consisting of O.

13. The compound represented by formula (III) or the pharmaceutically acceptable salt thereof according to claim 11 or 12, wherein each L is independently C₁₋₆ alkylene.

14. The compound represented by formula (III) or the pharmaceutically acceptable salt thereof according to any one of claims 11 to 13, wherein ring C is selected from the group consisting of a benzene ring, a naphthalene ring, and an anthracene ring, preferably from the group consisting of a naphthalene ring and an anthracene ring.

15. The compound represented by formula (III) or the pharmaceutically acceptable salt thereof according to any one of claims 11 to 14, being selected from the group consisting of compounds represented by formula (III-A), formula (III-B), formula (III-C), and formula (III-D) or pharmaceutically acceptable salts thereof: 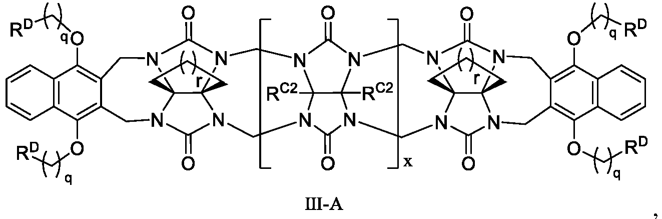 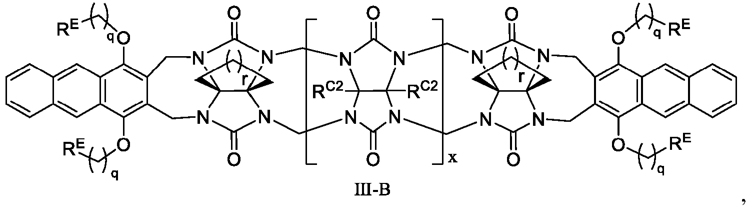 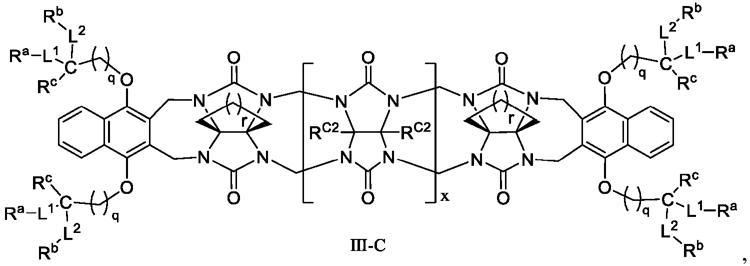 and 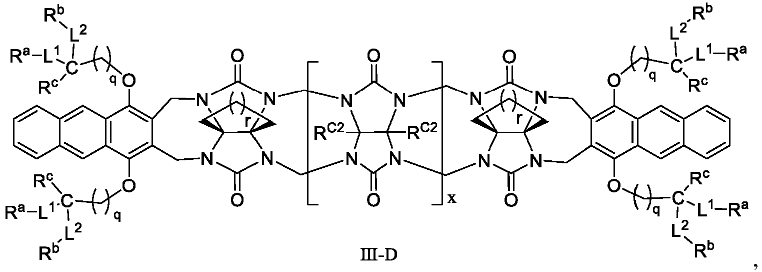 wherein:
each R^{D} is independently selected from the group consisting of a carboxylic acid group, a -carboxylate-cation, a phosphoric acid group, and a -phosphate-cation;
each R^{E} is independently selected from the group consisting of a carboxylic acid group, a -carboxylate-cation, a phosphoric acid group, a -phosphate-cation, a sulfonic acid group, and a -sulfonate-cation;
r is selected from the group consisting of 1, 2, and 3;
R^{C2}, q, x, L¹, L², R^{a}, R^{b}, and R^{c} are as defined in claim 11.

16. The compound represented by formula (III) or the pharmaceutically acceptable salt thereof according to any one of claims 11 to 15, wherein L₁ and L₂ are identical or different and are each independently C₁₋₆ alkylene.

17. The compound represented by formula (III) or the pharmaceutically acceptable salt thereof according to any one of claims 11 to 16, being selected from the group consisting of compounds represented by formula (III-A-1), formula (III-B-1), formula (III-B-2), formula (III-C-1), formula (III-C-2), formula (III-C-3), formula (III-D-1), formula (III-D-2), and formula (III-D-3) or pharmaceutically acceptable salts thereof: 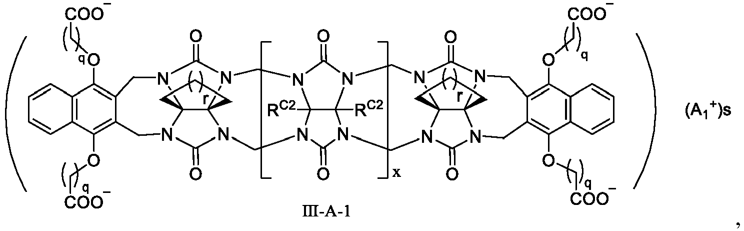 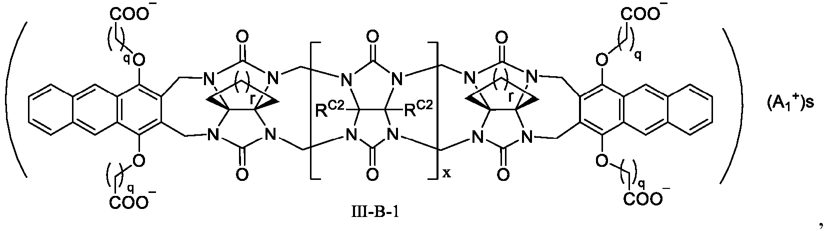 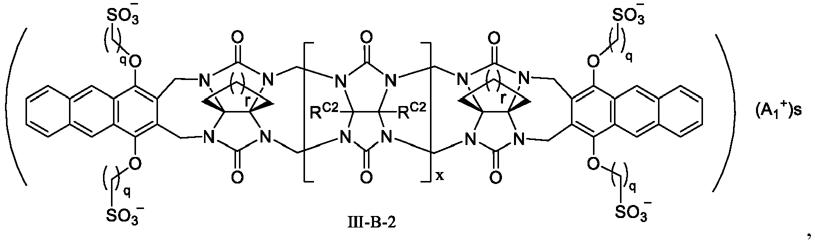 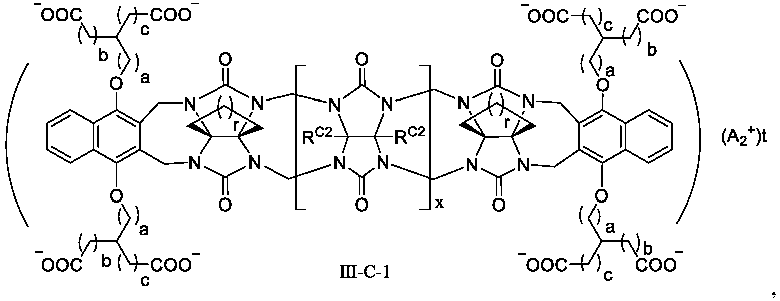 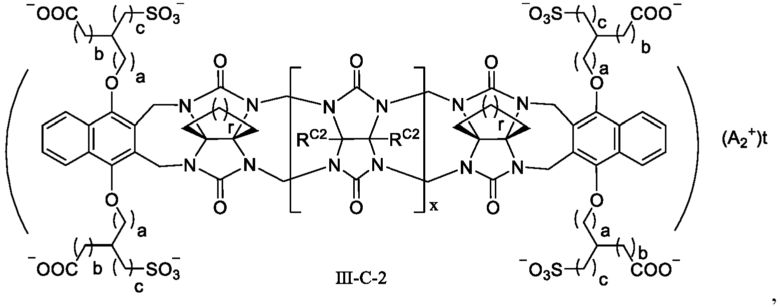 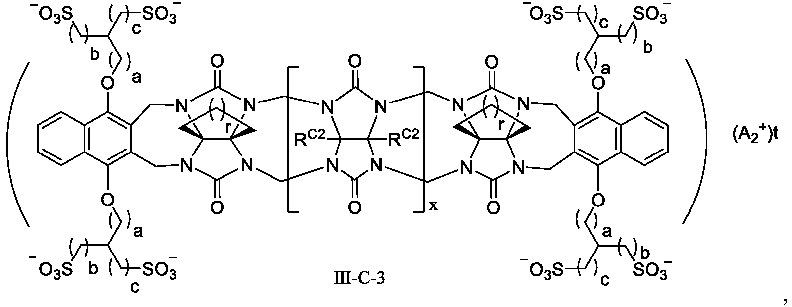 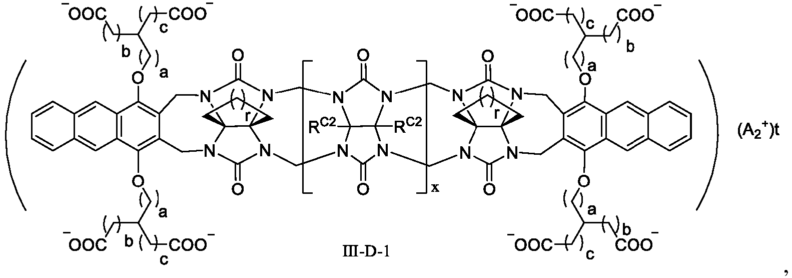 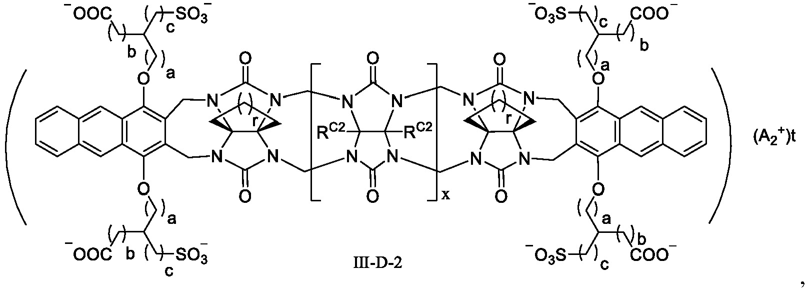 and 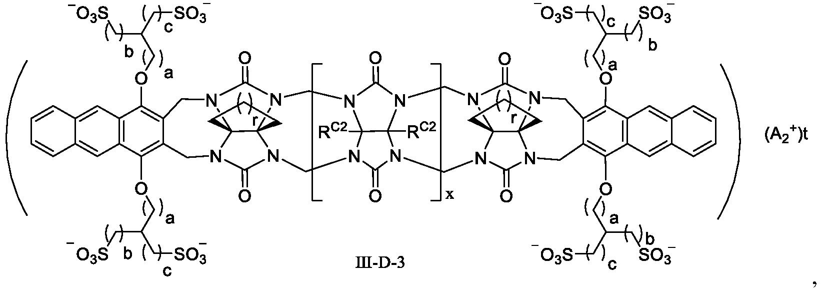 wherein:
each a is independently selected from the group consisting of 1, 2, 3, and 4;
each b is independently selected from the group consisting of 1, 2, 3, and 4;
each c is independently selected from the group consisting of 1, 2, 3, and 4;
A₁⁺ or A₂⁺ is identical or different and is each independently a monovalent cation or a divalent cation;
when A₁⁺ is a monovalent cation, s is 4;
when A₁⁺ is a divalent cation, s is 2;
when A₂⁺ is a monovalent cation, t is 8;
when A₂⁺ is a divalent cation, t is 4;
R^{C2}, q, and x are as defined in claim 11, and r is as defined in claim 15.

18. The compound represented by formula (III) or the pharmaceutically acceptable salt thereof according to claim 17, wherein each R^{C2} is independently hydrogen.

19. The compound represented by formula (III) or the pharmaceutically acceptable salt thereof according to claim 17 or 18, wherein r is 2 or 3, preferably 2.

20. The compound represented by formula (III) or the pharmaceutically acceptable salt thereof according to any one of claims 17 to 19, wherein a, b, and c are identical or different and are each independently selected from the group consisting of 1, 2, and 3; preferably, a, b, and c are all 1.

21. The compound represented by formula (III) or the pharmaceutically acceptable salt thereof according to any one of claims 17 to 20, wherein each q is independently selected from the group consisting of 2, 3, and 4, preferably from the group consisting of 3 and 4, and more preferably from the group consisting of 3.

22. The compound represented by formula (III) or the pharmaceutically acceptable salt thereof according to any one of claims 17 to 21, wherein x is selected from the group consisting of 2, 3, and 4, preferably from the group consisting of 2 and 3, and more preferably from the group consisting of 2.

23. The compound represented by formula (III) or the pharmaceutically acceptable salt thereof according to any one of claims 17 to 22, wherein A₁⁺ and A₂⁺ are each independently a monovalent cation selected from the group consisting of H⁺, Na⁺, K⁺, H₄N⁺, Et₃NH⁺, (HOCH₂CH₂)₃NH⁺, and cationic forms of ethylenediamine, piperazine, and triphenylmethylaminomethane, preferably from the group consisting of H⁺, Na⁺, and K⁺, and more preferably from the group consisting of Na⁺.

24. The compound represented by formula (III) or the pharmaceutically acceptable salt thereof according to any one of claims 17 to 23, wherein A₁⁺ and A₂⁺ are each independently a divalent cation selected from the group consisting of Ca²⁺, Mg²⁺, and Zₙ₂₊.

25. The compound represented by formula (III) or the pharmaceutically acceptable salt thereof according to any one of claims 17 to 24, being selected from the group consisting of: 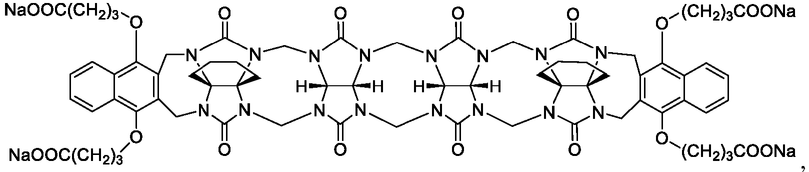 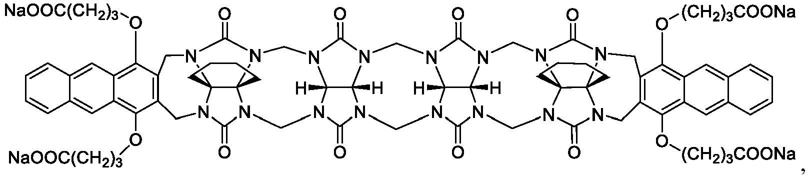 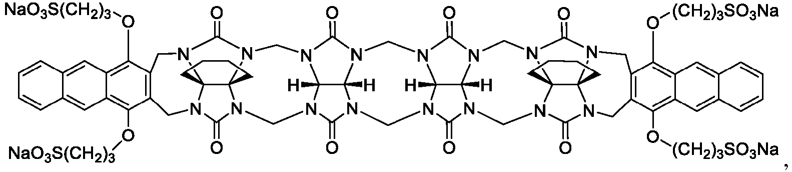 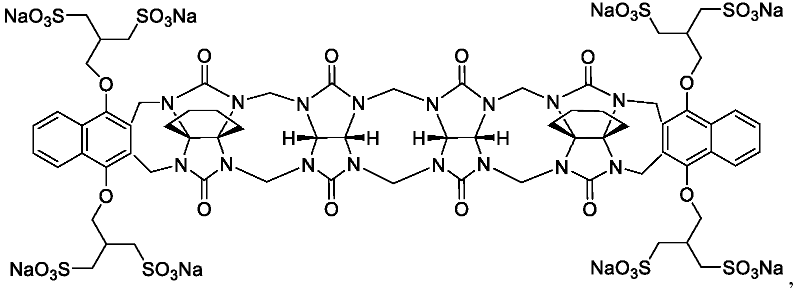 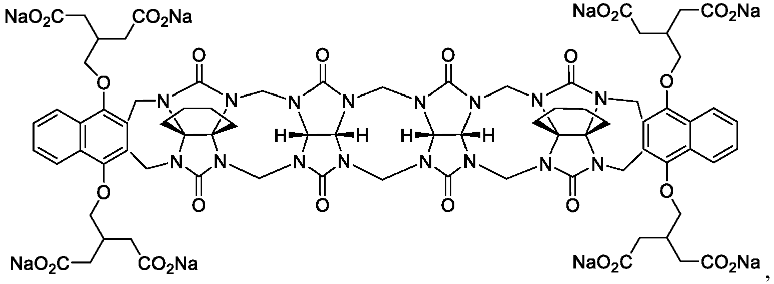 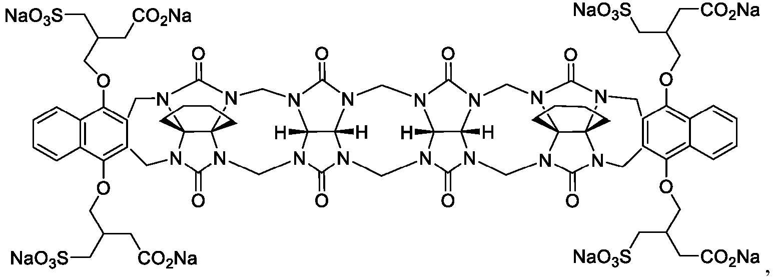 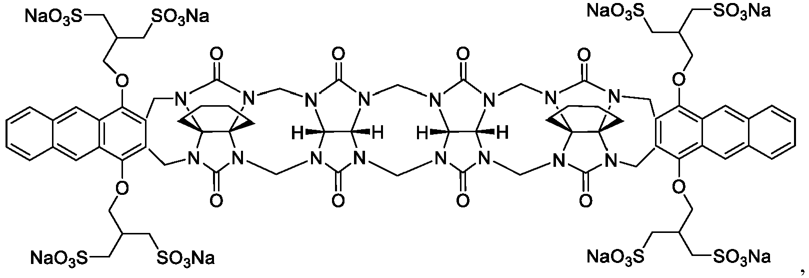 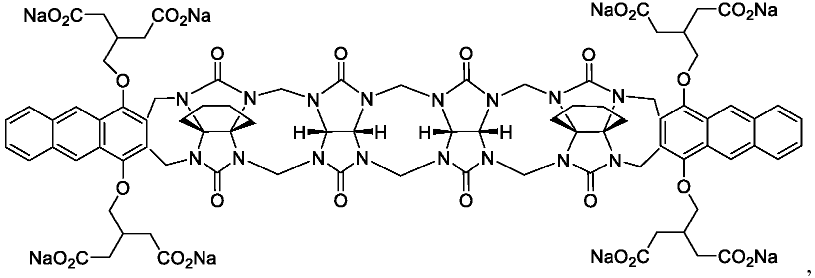 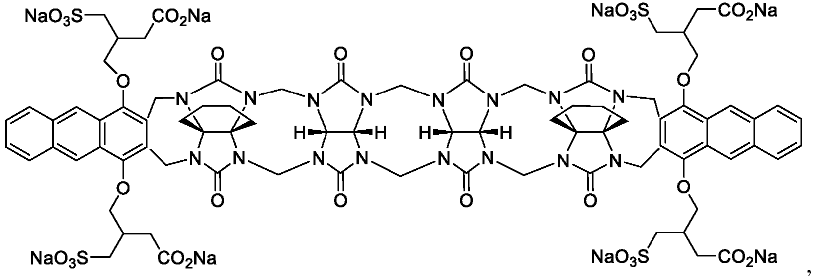 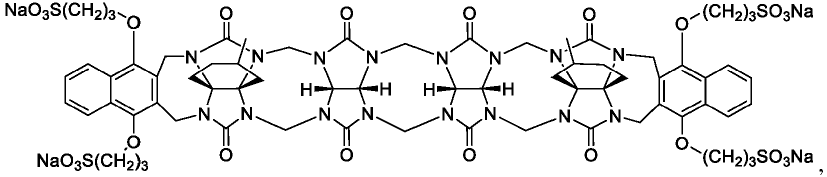 and

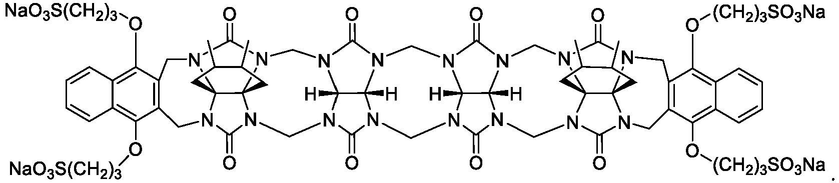

26. An isotopically substituted form of the compound or the pharmaceutically acceptable salt thereof according to any one of claims 1-25, wherein preferably, the isotopically substituted form is a deuterium-substituted form.

27. A pharmaceutical composition, comprising a therapeutically effective amount of at least one of the compound or the pharmaceutically acceptable salt thereof according to any one of claims 1-25 and the isotopically substituted form according to claim 26, and a pharmaceutically acceptable excipient.

28. Use of the compound or the pharmaceutically acceptable salt thereof according to any one of claims 1-25, the isotopically substituted form according to claim 26, or the pharmaceutical composition according to claim 27 in the preparation of a medicament for treating or preventing a disease or disorder selected from the group consisting of a proliferative disease, a cardiovascular related disease, and a hematological cancer.

29. Use of the compound or the pharmaceutically acceptable salt thereof according to any one of claims 1-25, the isotopically substituted form according to claim 26, or the pharmaceutical composition according to claim 27 in the preparation of a medicament for reversing drug-induced neuromuscular blockade and/or anesthesia.
